(19) Europäisches Patentamt
European Patent Office
Office européen des brevets

(11) Publication number: **0 085 476**
**B1**

(12) # EUROPEAN PATENT SPECIFICATION

(45) Date of publication of patent specification: **22.04.87**

(21) Application number: **83300078.9**

(22) Date of filing: **07.01.83**

(51) Int. Cl.[4]: **C 07 D 405/00,**
C 07 D 409/00,
C 07 D 401/00,
C 07 D 403/00,
C 07 D 239/00, A 01 N 47/36

(54) **Herbicidal sulfonamides.**

(30) Priority: **28.10.82 US 437325**
**07.01.82 US 337933**
**01.11.82 US 437367**

(43) Date of publication of application:
**10.08.83 Bulletin 83/32**

(45) Publication of the grant of the patent:
**22.04.87 Bulletin 87/17**

(84) Designated Contracting States:
**AT BE CH DE FR IT LI LU NL SE**

(56) References cited:
**EP-A-0 001 515**
**EP-A-0 015 683**
**EP-A-0 030 139**

(73) Proprietor: **E.I. DU PONT DE NEMOURS AND COMPANY**
**1007 Market Street**
**Wilmington Delaware 19898 (US)**

(72) Inventor: **Sauers, Richard Frank**
**504 Revere Court**
**Hockessin Delaware 19707 (US)**
Inventor: **Shapiro, Rafael**
**2221-F Prior Road**
**Wilmington Delaware 19809 (US)**

(74) Representative: **Hildyard, Edward Martin et al**
**Frank B. Dehn & Co. European Patent Attorneys**
**Imperial House 15-19 Kingsway**
**London WC2B 6UZ (GB)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 085 476 B1

**Description**

Background of the Invention

The present invention relates to *ortho*-heterocyclic benzenesulfonylureas and, more particularly, to *ortho*-(mono, di or triazinyl)benzenesulfonylureas and to their use as pre- or post-emergent herbicides or as plant growth regulants.

U.S. Patent 4,169,719 discloses and claims N-(heterocyclicaminocarbonyl)arylsulfonamides, such as N-[(4,6-dimethylpyrimidin-2-yl)aminocarbonyl]benzenesulfonamide, which are useful as herbicides and as plant growth regulants.

U.S. Patent 4,127,405 teaches that N-(1,3,5-triazin-2-ylaminocarbonyl)arylsulfonamides, such as N-[(4,6-dimethyl-1,3,5-triazin-2-yl)aminocarbonyl]benzenesulfonamide, are useful as herbicides and as plant growth regulants.

Our EP—A—44,209, published January 20, 1982, relates to α-substituted -*o*-methylbenzenesulfonamides of the general formula:

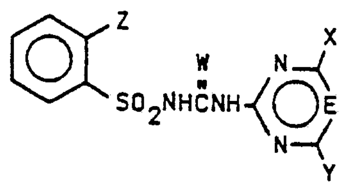

wherein, among other values,

E can be CH or N;

X and Y can be $CH_3$ or $OCH_3$;

Z can be $CH_2SO_2N(CH_3)_2$, $CH_2WQ$, $CH_2OH$, $CH_2Cl$, $CHCl_2$, $CH(CH_3)Cl$ or

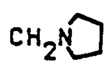 ;

W can be O, S, SO or $SO_2$; and

Q can be $C_1$—$C_4$ alkyl.

These compounds are useful as herbicides and as plant growth regulants.

Our EP—A—44,211, published January 20, 1982 relates to N-(heterocyclicaminocarbonyl)-*o*-(hydrocarbyl)benzenesulfonamides which are useful as herbicides and plant growth regulants. Compounds of the general formula:

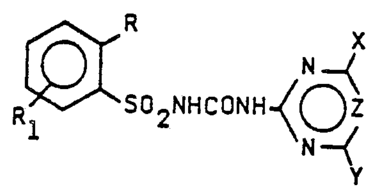

are disclosed wherein

$R_1$ is H, F, Cl, Br, $NO_2$, $CF_3$, $C_1$—$C_4$ alkyl, $OCF_3$ or $C_1$—$C_4$ alkoxy;

R is

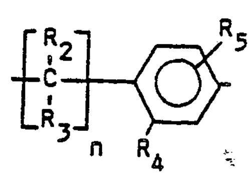 ;

n is 0 or 1;

$R_2$ and $R_3$ are independently H or $CH_3$;

$R_4$ and $R_5$ can be, among other values, H or $CH_3$;

X and Y can be $CH_3$, $OCH_3$ or $OCH_2CH_3$; and

Z is CH or N.

South African Patent Application No. 82/0465 to Nihon Tokushu Noyaku Seizo K.K. discloses and claims substituted phenylsulfonylurea derivatives of the formula:

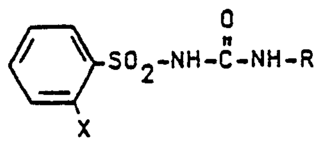

wherein X is a phenyl or phenoxy group and R represents a group:

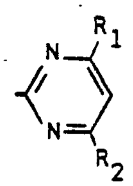

or

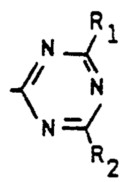

wherein $R_1$ and $R_2$ are methyl or methoxy. The compounds are said to show a superior selective control effect, especially when employed as pre-germination soil treating agents or plant stems and leaves and soil treating agents for weeds in paddy rice fields.

Our EP—A—30139 also discloses some herbicidal sulfonamides having stuctural similarities to the compounds of the present application.

Summary of the Invention

This invention relates to compounds of Formulae I or Ia, suitable agricultural compositions containing them, and their method-of-use as general and/or selective pre-emergence and/or post-emergence herbicides and as plant growth regulants.

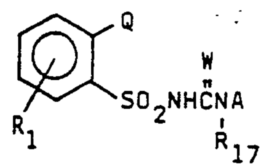

I

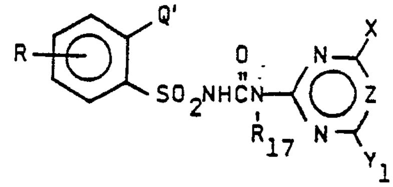

Ia

wherein

Q is

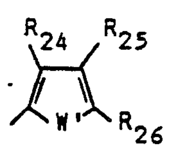

,

Q-1

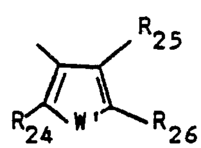

Q-2

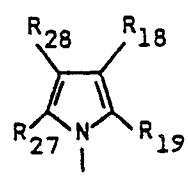

,

Q-3

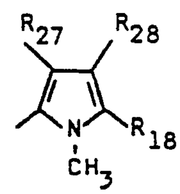

,

Q-4

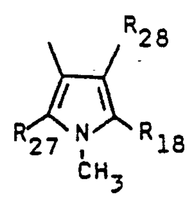

,

Q-5

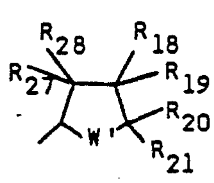

,

Q-6

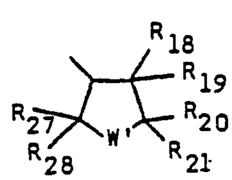

,

Q-7

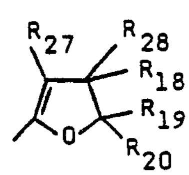

,

Q-8

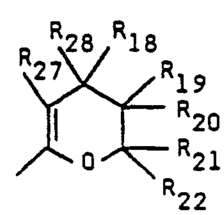

,

Q-9

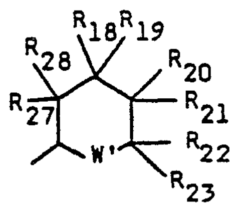 or 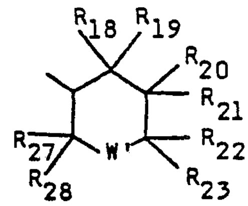 ;

Q-10 Q-11

where Q' is

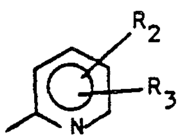 , 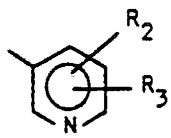 , 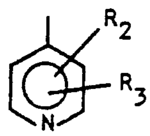 , 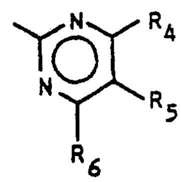 , 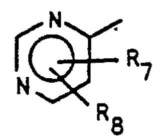 ,

Q'-1 Q'-2 Q'-3 Q'-4 Q'-5

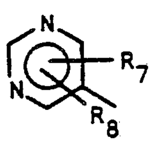 , 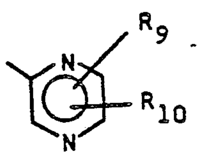 , 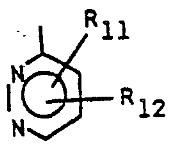 , 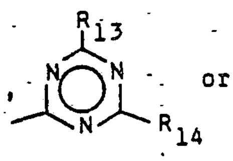 or 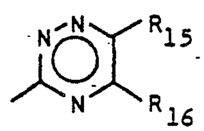 ;

Q'-6 Q'-7 Q'-8 Q'-9 Q'-10

W is O or S;
W' is O or S;
R is H, F, Cl, $CH_3$ or $OCH_3$;
$R_1$ is H, F, Cl, Br, $CH_3$, $CF_3$ or $OCH_3$;
$R_2$, $R_3$, $R_5$, $R_9$, $R_{10}$, $R_{11}$, $R_{12}$ and $R_{17}$ are independently H or $CH_3$;
$R_4$, $R_6$, $R_7$ and $R_8$ are independently H, $CH_3$ or $OCH_3$;
$R_{13}$, $R_{14}$, $R_{15}$ and $R_{16}$ are independently $CH_3$ or $OCH_3$;
$R_{18}$ is H or $CH_3$;
$R_{19}$ is H or $CH_3$;
$R_{20}$ is H or $CH_3$;
$R_{21}$ is H or $CH_3$;
$R_{22}$ is H or $CH_3$;
$R_{23}$ is H or $CH_3$;
$R_{24}$ is H, $CH_3$, $C_2H_5$, Cl, Br, $OCH_3$ or $OC_2H_5$;
$R_{25}$ is H, $CH_3$, $C_2H_5$, Cl, Br, $OCH_3$ or $OC_2H_5$;
$R_{26}$ is H, $CH_3$, $C_2H_5$, Cl, Br, $OCH_3$, $OC_2H_5$ or $CO_2CH_3$;
$R_{27}$ is H or $CH_3$;
$R_{28}$ is H or $CH_3$;
A is

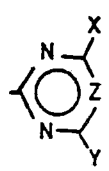 , 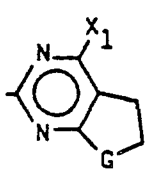 , 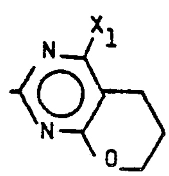 , 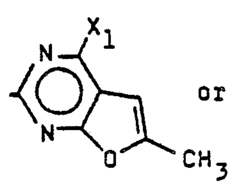 or 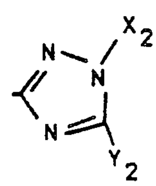 ;

X is $CH_3$, $OCH_3$ or Cl;
Y is $CH_3$, $C_2H_5$, $CH_2OCH_3$, $OCH_3$, $OC_2H_5$, $CH(OCH_3)_2$, $SCH_3$, $CF_3$, $NH_2$, $NHCH_3$, $N(CH_3)_2$, $OCH_2CH_2OCH_3$, $OCH_2CF_3$ or

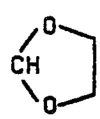 ;

$Y_1$ is $CH_3$, $C_2H_5$, $CH_2OCH_3$, $OCH_3$, $OC_2H_5$, $CH(OCH_3)_2$, $NH_2$, $NHCH_3$ or $N(CH_3)_2$;
Z is CH or N;
$X_1$ is $CH_3$, $OCH_3$ or Cl;
G is O or $CH_2$;
$X_2$ is $C_1$—$C_3$ alkyl or $CH_2CF_3$;
$Y_2$ is $CH_3O$, $C_2H_5O$, $CH_3S$ or $C_2H_5S$; and their agriculturally suitable salts; provided that
1) when W is S, then $R_{17}$ is H,
A is

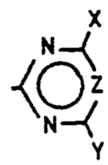,

Y is $CH_3$, $OCH_3$, $C_2H_5$, $CH_2OCH_3$, $CH(OCH_3)_2$ or

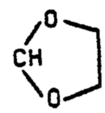;

2) when Q is

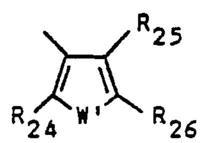,

then $R_{24}$ is H, $CH_3$ or $C_2H_5$; $R_{25}$ is H, $CH_3$ or $C_2H_5$; and $R_{26}$ is H, $CH_3$, $C_2H_5$ or —$CO_2CH_3$;

3) when one of $R_{24}$, $R_{25}$, $R_{26}$ is other than H, then the other two substituents are H or $CH_3$;

4) the total number of carbon atoms in $R_{18}$, $R_{19}$, $R_{20}$, $R_{21}$, $R_{22}$, $R_{23}$, $R_{27}$ and $R_{28}$ is less than or equal to 4;

5) when X is Cl, then Z is CH and Y or $Y_1$ is $OCH_3$, $NH_2$, $OC_2H_5$, $NHCH_3$ or $N(CH_3)_2$; and

6) when R is $CH_3$, then R is in the 3-, 4- or 5-position of the benzene ring, relative to the sulfonamido group in the 1-position.

Preferred for their higher herbicidal activity and/or their more favorable ease of synthesis are:

1) Compounds of Formula I where W is o;

2) Compounds of Preferred (1) where $R_1$ and $R_{17}$ are H;

3) Compounds of Preferred (2) where $R_{24}$ is H, $CH_3$ or $C_2H_5$; $R_{25}$ is H, $CH_3$ or $C_2H_5$; and $R_{26}$ is H, $CH_3$, $C_2H_5$ or —$CO_2CH_3$;

4) Compounds of Preferred (3) where Y is $CH_3$, $CH_2OCH_3$, $OCH_3$, $OC_2H_5$, $CH(OCH_3)_2$ or

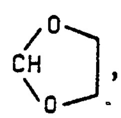,

and A is

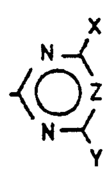;

5) Compounds of Formula Ia where R and $R_{17}$ are H;

6) Compounds of Preferred (5) where $Y_1$ is $CH_3$, $OCH_3$, $CH_2OCH_3$ or $N(CH_3)_2$;

7) Compounds of Preferred (4) where Q is

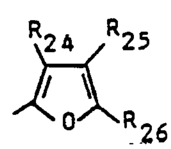;

8) Compounds of Preferred (4) where Q is

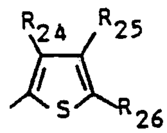 ;

9) Compounds of Preferred (4) where Q is

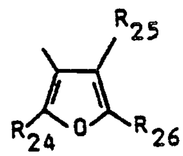 ;

10) Compounds of Preferred (4) where Q is

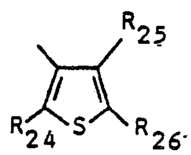 ;

11) Compounds of Preferred (4) where Q is

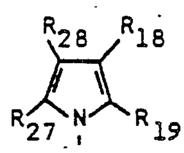 ;

12) Compounds of Preferred (4) where Q is

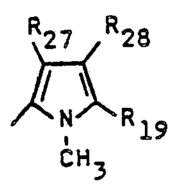 ;

13) Compounds of Preferred (4) where Q is

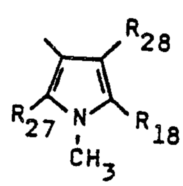 ;

14) Compounds of Preferred (4) where Q is

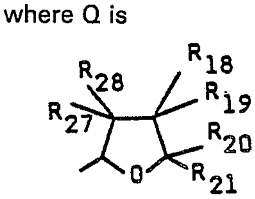 ;

15) Compounds of Preferred (4) where Q is

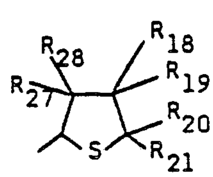 ;

16) Compounds of Preferred (4) where Q is

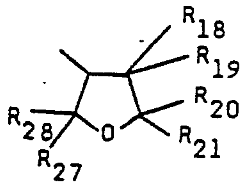

;

17) Compounds of Preferred (4) where Q is

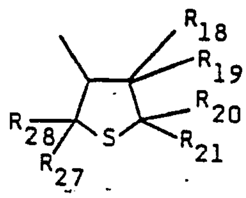

;

18) Compounds of Preferred (4) where Q is

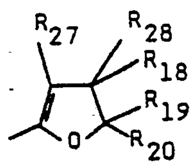

;

19) Compounds of Preferred (4) where Q is

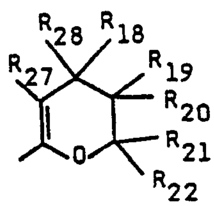

;

20) Compounds of Preferred (4) where Q is

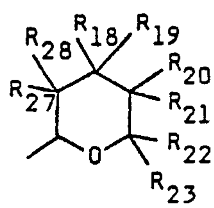

;

21) Compounds of Preferred (4) where Q is

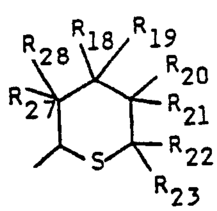

;

22) Compounds of Preferred (4) where Q is

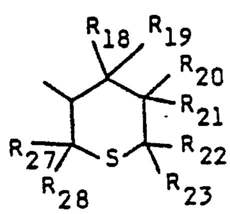

;

23) Compounds of Preferred (4) where Q is

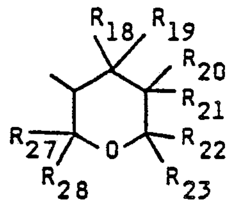 ;

24) Compounds of Preferred (6) where Q' is

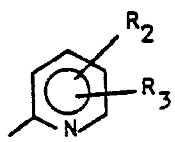 , 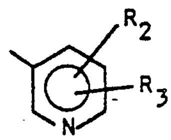 or 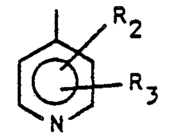 ;

25) Compounds of Preferred (6) where Q' is

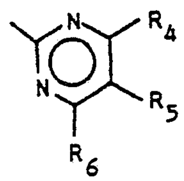 , 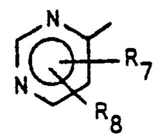 or 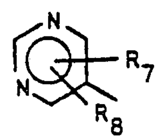 ;

26) Compounds of Preferred (6) where Q' is

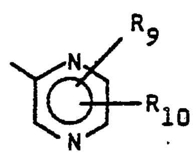 ;

27) Compounds of Preferred (6) where Q' is

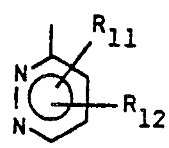 ;

28) Compounds of Preferred (6) where Q' is

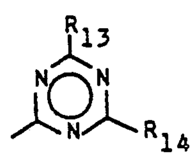 ; and

29) Compounds of Preferred (6) where Q' is

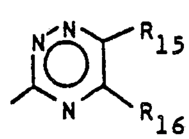 .

Specifically preferred for reasons of their highest herbicidal activity or most favorable ease of synthesis are:

N-[(4,6-dimethylpyrimidin-2-yl)aminocarbonyl]-2-(tetrahydrofuran-2-yl)benzenesulfonamide;

N-[(4-methoxy-6-methylpyrimidin-2-yl)aminocarbonyl]-2-(tetrahydrofuran-1-yl)benzenesulfonamide;

N-[(4,6-dimethoxypyrimidin-2-yl)aminocarbonyl]-2-(tetrahydrofuran-2-yl)benzenesulfonamide;
N-[(4-chloro-6-methoxypyrimidin-2-yl)aminocarbonyl]-2-(tetrahydrofuran-2-yl)benzenesulfonamide;
N-[(4,6-dimethoxy-1,3,5-triazin-2-yl)aminocarbonyl]-2-(tetrahydrofuran-2-yl)benzenesulfonamide;
N-[(4-methoxy-6-methyl-1,3,5-triazin-2-yl)aminocarbonyl]-2-(tetrahydrofuran-2-yl)benzenesulfonamide;
N-[(4-methoxy-6-methylpyrimidin-2-yl)aminocarbonyl]-2-(2-pyridinyl)benzenesulfonamide;
N-[(4,6-dimethylpyrimidin-2-yl)aminocarbonyl]-2-(2-pyridinyl)benzenesulfonamide;
N-[(4,6-dimethoxypyrimidin-2-yl)aminocarbonyl]-2-(2-pyridinyl)benzenesulfonamide; and
N-[(4-methoxy-6-methylpyrimidin-2-yl)aminocarbonyl]-2-(2-pyrimidinyl)benzenesulfonamide.

Detailed Description of the Invention Synthesis

The *ortho*-heterocyclicbenzenesulfonylureas of Formula I can be prepared by reacting the appropriately substituted benzenesulfamide II with an appropriate methyl heteroaryl carbamate III in the presence of an equimolar amount of trimethylaluminum according to the procedure of Equation 1. Unless indicated otherwise, all temperatures are in °C.

Equation 1

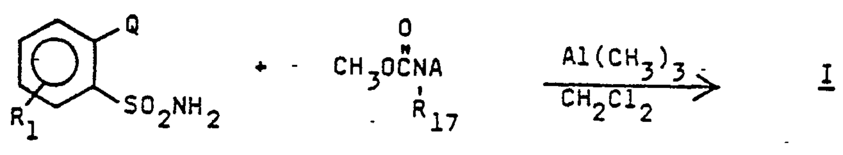


II III

wherein

$R_1$, $R_{17}$, Q and A are as previously defined, and W is O.

The reaction of Equation 1 is best carried out in methylene chloride at 25° to 40°C for 24 to 96 hours under a nitrogen atmosphere. The product can be isolated by the addition of an aqueous acetic acid solution followed by extraction of the product into methylene chloride or direct filtration of a product of low solubility. The product can ordinarily be purified by trituration with solvents such as *n*-butyl chloride or ether or by column chromatography.

Further details of this reaction and the preparation of the carbamates of Formula III can be found in U.S. Serial No. 337,934, laid open as a priority document in support of our EP—A—83975.

Equation 1a below illustrates the preparation of the required methylpyrimidinyl carbamates and methyltriazinyl carbamates of Formula (III) in Equation I above.

Equation 1a

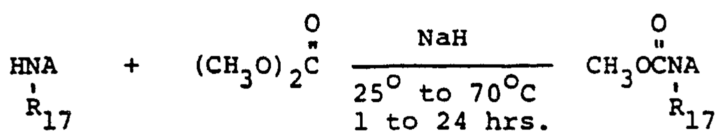


(V) (III)

wherein

A and $R_{17}$ are as originally defined.

According to Equation 1a, a heterocyclic amine of Formula (V) is reacted with two equivalents of sodum hydride and excess dimethylcarbonate to form III. The reaction is run in an inert solvent such as tetrahydrofuran at 25°C to reflux for 1 to 24 hours. The product is isolated by (a) adding about two equivalents of concentrated HCl and water saturated with NaCl, and (b) separating out the organic phase and concentrating to dryness *in vacuo*.

Literature references relating to the synthesis of heterocyclic amines such as V are given hereinafter.

Compounds of Formula I, in which Q does not include Q—3, Q—4 or Q—5, may also be prepared by reacting the appropriately substituted benzenesulfonyl isocyanate or isothiocyanate with an appropriate aminoheterocycle, V, as shown in Equation 2.

Equation 2

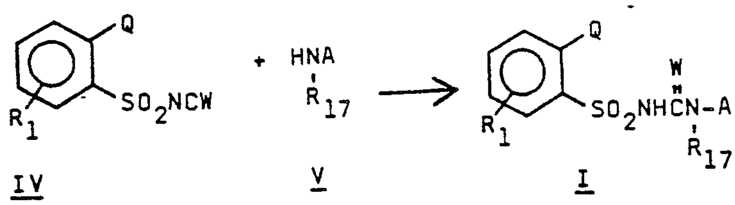

IV V I

wherein

$R_1$, $R_{17}$, W and A are as previously defined, and Q is Q—1, Q—2, and Q—6 or Q—11.

The reaction of Equation 2 can best be carried out in an inert aprotic solvent such as methylene chloride, tetrahydrofuran or acetonitrile at a temperature between 20 and 80°. A catalytic amount of 1,4-diazabicyclo[2,2,2]octane (DABCO) may be used to accelerate the reaction. In cases in which the products are insoluble in the reaction solvent, they may be isolated by simple filtration. When the products are soluble, they may be isolated by evaporation of the solvent and trituration of the residue with solvents such as 1-chlorobutane, ethyl ether or methanol and filtration.

The benzenesulfonyl isocyanates of Formula IVa may be prepared as shown below in Equation 3, by phosgenation of the sulfonamide II in the presence of butyl isocyanate. The sulfonyl isocyanates of Formula IV may also be prepared, as shown in Equation 4, by phosgenation of the butyl ureas of Formula VI.

Equation 3

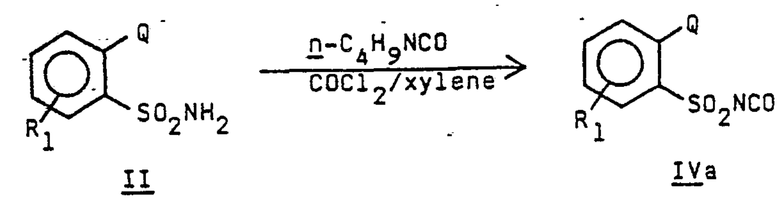


II IVa

wherein

$R_1$ and Q are as defined in Equation 2.

The above reaction can be carried out by heating a mixture of the appropriate sulfonamide (III), an alkyl isocyanate such as butyl isocyanate and a catalytic amount of a tertiary amine such as 1,4-diaza[2,2,2]-bicyclooctane (DABCO) in xylene, or other inert solvent of boiling point ≥135° to approximately 135°C. Phosgene can then be added to the mixture over a 1—6 hour period until an excess of phosgene is present as indicated by a drop in the boiling point to less than 130°C. The mixture is then cooled and filtered to remove a small amount of insoluble by-products. The solvent and the alkyl isocyanate can be distilled off *in vacuo* leaving a residue of the crude sulfonyl isocyanate, IVa, which can be used without further purification.

Equation 4

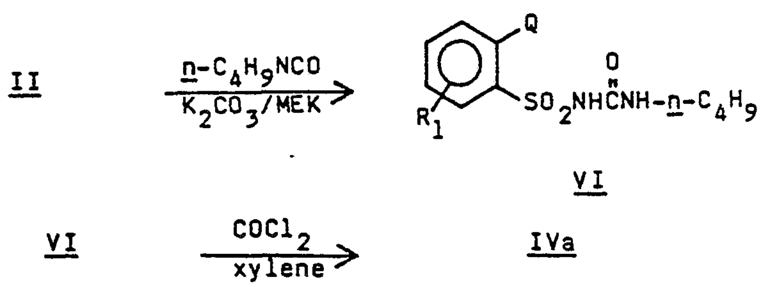


II VI

VI IVa

wherein

$R_1$ and Q are as defined in Equation 2.

The compounds of Formula VI can be prepared by stirring a mixture of the sulfonamides, II, anhydrous potassium carbonate, and *n*-butyl isocyanate in acetone or methyl ethyl ketone at 25—80° until all of the sulfonamide has reacted. The products can be isolated by quenching in dilute mineral acid and re-cystallizing the solid product. The compounds VI are then treated with phosgene and a catalytic amount of DABCO in refluxing xylene or chlorobenzene in a manner analogous to that described in Equation 3.

Sulfonylisothiocyanates of Formula IV where W=S can be prepared by treatment of sulfonamides with carbon disulfide and potassium hydroxide followed by the reaction of the dipotassium salt with phosgene

according to the teaching of K. Hartke, *Arch. Pharm., 229,* 174 (1966).

Alternatively, compounds of Formula IB in which W is S may be prepared by the reaction of the appropriate sulfonamide of Formula II with a heterocyclic isothiocyanate of Formula Va as depicted in Equation 5.

Equation 5

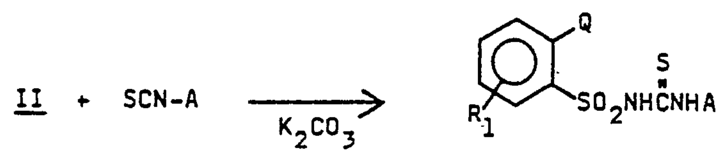


Va Ib

wherein

A, Q and $R_1$ are as previously defined.

The reaction of Equation 5 is best carried out by dissolving or suspending the sulfonamide and isothiocyanate in a polar solvent such as acetone, acetonitrile, ethyl acetate or methyl ethyl ketone, adding an equivalent of a base such as potassium carbonate and stirring the mixture at ambient temperature up to the reflux temperature for one to twenty-four hours. In some cases, the product precipitates from the reaction mixture and can be removed by filtration. The product is stirred in dilute mineral acid, filtered and washed with cold water. If the product does not precipitate from the reaction mixture it can be isolated by evaporation of the solvent, trituration of the residue with dilute mineral acid and filtering off the insoluble product.

The heterocyclic isothiocyanates which are used in the procedure of Equation 5 are prepared, for example, according to the method of Japan patent Application Pub: Kokai 51—143686, June 5, 1976, or that of W. Abraham and G. Barnikow, *Tetrahedron, 29,* 691—7 (1973).

The synthesis of heterocyclic amine derivatives such as those depicted by Formula V has been reviewed in "The Chemistry of Heterocyclic Compounds", a series published by Interscience Publ., New York and London, to which the reader is now referred for further information. Aminopyrimidines are described by D. J. Brown in "The Pyrimidines", Vol. XVI of the above series.

The 5,6-dihydofuro[2,3-d]pyrimidin-2-amines, the cyclopenta[d] pyrimidin-2-amines, and the 6,7-dihydro-5H-pyrano[2,3-d]pyrimidin-2-amines, are prepared as described in our EP—A—15,683. Synthesis of the furo[2,3-d]pyrimidin-2-amines are described in our EP—A—46,677. The heterocyclic amines in which A is a triazole ring are disclosed in our EP—A—73562. Pyrimidine and triazine amines in which Y is dimethoxymethyl or 1,3-dioxolan-2-yl are prepared as described in copending U.S. Application Serial No. 328,098 EP—A—84224.

Sulfonamides of Formula II, in which Q is Q—1 or Q—2, can be prepared by the sequence of reactions shown in Equation 6.

Equation 6

(6a)

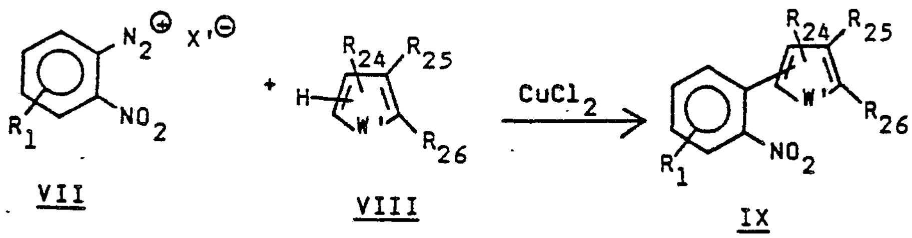


VII VIII IX

(6b)

IX

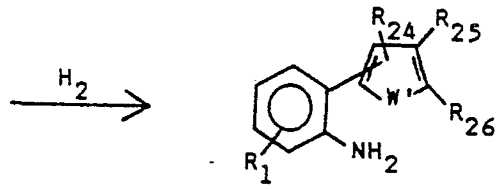


X

(6c)

X

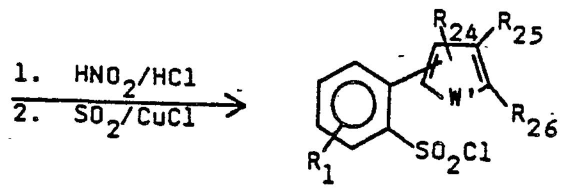


XI

(6d)

XI

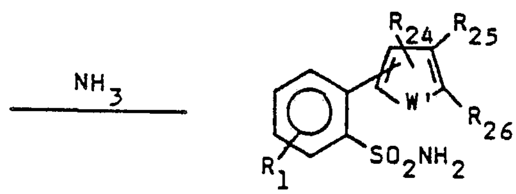


IIa

wherein

$R_1$ and W' are so previously defined;
X' is Cl, Br or $HSO_4$;
$R_{24}$ is $R_{25}$ are H, $CH_3$ or $C_2H_5$; and
$R_{26}$ is H, $CH_3$; $C_2H_5$ or $CO_2CH_3$.

Reaction (6a):

In this reaction an *o*-nitrosubstituted phenyl diazonium halide or sulfate is coupled with an appropriately substituted thiophene or furan in the presence of a catalyst such as cupric chloride. The conditions necessary for this transformation are described in Gomberg and Bachmann, *J. Am. Chem. Soc., 46,* 2339 (1924); J. Johnson, *J. Chem. Soc.,* 895 (1946); and in *J. Pharm. Soc. (Japan), 90,* 1150—1155 (1970). In cases where both the α- and the β-position of the thiophene or furan are available for ocupling, both isomers are usually obtained with the α-coupled product being the predominent isomer. These isomers can be separated by fractional distillation or chromatography and carried on through the remaining three reactions of Equation 6.

Reaction (6b):

The reduction described in step (6b) is accomplished by treating a solution of the compounds of Formula IX, in a solvent such as ethanol, ethyl acetate, or DMF, in a pressure vessel, with 100—1000 pounds per square inch (689—6890 kPa) of hydrogen at 80—150° in the presence of a hydrogenation catalyst such as 5—10% palladium absorbed on carbon. When the theoretical amount of hydrogen has been absorbed, the solution is then cooled and the catalyst is removed by filtration. The product is then isolated by evaporation of the solvent.

Reaction (6c):

The diazotization and coupling with sulfur dioxide, described in step *6c,* is accomplished in the following manner. A solution of the aniline of Formula X in a mixture of concentrated hydrochloric acid and glacial acetic acid is treated with a solution of sodium nitrite in water at −5 to 0°C. After stirring for 10—15 minutes at 0°C to insure complete diazotization, this solution is added to a mixture of an excess of sulfur dioxide, and a catalytic amount of cuprous chloride in glacial acid at 0—5°. The temperature is kept at 0—5° for ¼ to 1 hour then raised to 20—25° and held at that temperature for 2—4 hours. This solution is then poured into a large excess of ice water, and the sulfonyl chloride products, XI, is isolated by filtration or by extraction into a solvent such as ethyl ether or methylene chloride followed by evaporation of the solvent.

Reaction (6d):

The amination described in step (6d) is carried out by treating a solution of the sulfonyl chloride of Formula XI with an excess of anhydrous ammonia in a solvent such as ethyl ether or methylene chloride at 0—25°C. If the product sulfonamide, II, is insoluble, it may be isolated by filtration followed by washing out the salts with water. If the product sulfonamide is soluble in the reaction solution, it may be isolated by filtering off the precipitated ammonium chloride and evaporation of the solvent.

Sulfonamides of Formula IIa can be chlorinated or brominated on the thiophene or furan ring by procedures well known in the art. These halogenated sulfonamides may then be reacted with sodium methoxide in methanol or sodium ethoxide in ethanol to replace the halogen atom with a methoxy or ethoxy group.

Alternatively, the substituted nitrobenzenes of Formula IX can be prepared by the procedure shown in Equation 7.

Equation 7

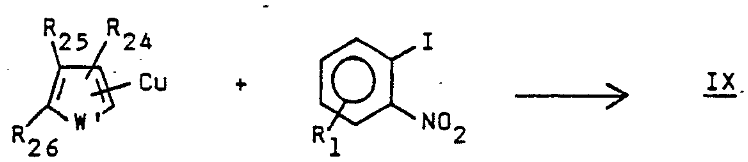

XII

wherein

$R_1$, $R_{24}$, $R_{25}$, $R_{26}$, and W' are as previously defined.

In the reaction shown in Equation 7, a furyl- or thienyl copper compound of Formula XII is reacted with an iodonitrobenzene in a solvent such as pyridine or quinoline. The copper compounds of Formula XII are prepared by reacting the corresponding lithium compounds with cuprous iodide or cuprous bromide in a solvent such as diethyl ether. The detailed procedures for these types of reactions are described in the following references: M. Nilsson and C. Ullenius, *Acta. Chem. Scand., 24,* 2379—2388 (1970); C. Ullenius, *Acta. Chem. Scand., 26,* 3383—3386 (1972).

Sulfonamides containing a tetrahydrofuran or tetrahydrothiophene group (Formula II, Q = Q—6 and Q—7) can be prepared by catalytic reduction of the corresponding furan or thiophene compounds as shown in Equation 8.

Equation 8

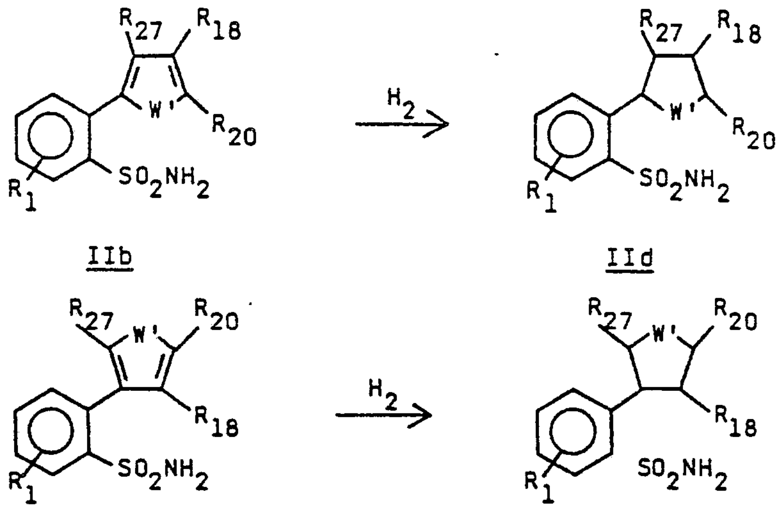


IIb

IId

IIc

IIe

wherein

$R_1$, $R_{27}$, $R_{18}$, $R_{20}$, and W' are as previously defined.

Selective reductions of the type shown in Equation 8 are well known in the literature. The choice of catalyst, solvent, pressure and temperature for reduction of furans has been reviewed by Samuel Sevadesh in *The Furans* by A. P. Dunlop and F. N. Peters, Reinhold Publishing Corporation, New York, N.Y. 1953, pp. 674—713; and by P. N. Rylander in *Catalytic Hydrogenation in Organic Synthesis*, Academic Press, 1979, pp. 227—234. The reduction of thiophenes is reviewed by H. S. Hartough in *Thiophene and Its Derivatives*, The Chemistry of Heterocyclic Compound Series, Interscience Publishers, Inc., New York, N.Y. 1952, pp. 167—169.

Sulfonamides containing a tetrahydrothiophene or tetrahydrothiopyran group can also be prepared by the sequence of reactions shown in Equation 9.

Equation 9

(9a)

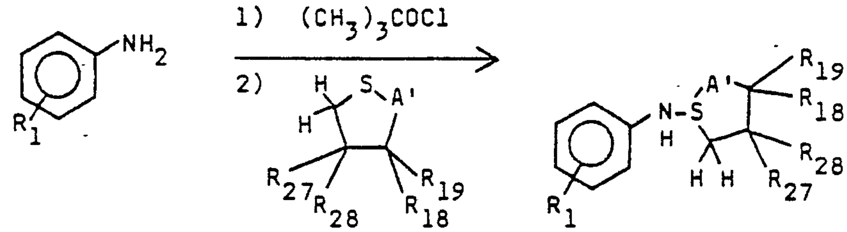


XIII XIV

(9b)

XIV

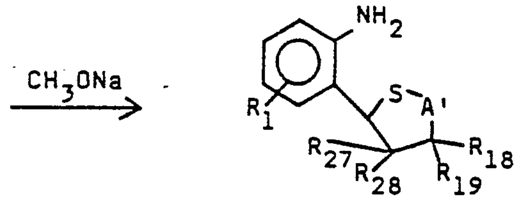


XV

(9c)

XV

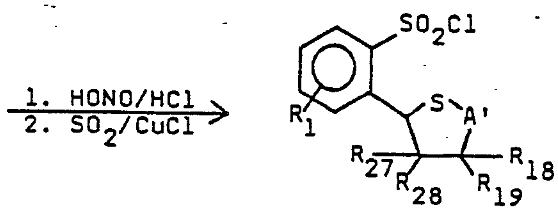


XVI

(9d)

XVI

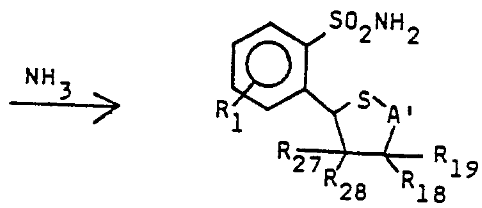


IIf

wherein

A' is 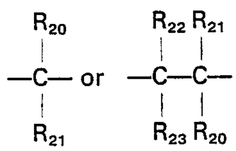 or

$R_1$, $R_{27}$, $R_{28}$, $R_{18}$, $R_{19}$, $R_{20}$, $R_{21}$, $R_{22}$ and $R_{23}$ are as previously defined.

The general conditions used to carry out the reactions described in Reactions (9a) and (9b) are described by P. G. Gassman and G. D. Gruetzmacher, *J. Am. Chem. Soc., 96,* 5487—5495 (1974). The procedures shown in Reactions (9c) and (9d) are directly analogous to those described in Reactions (6c) and (6d).

Other sulfonamides containing tetrahydrofuran or tetrahydropyran groups can be prepared by the sequence of reactions shown in Equation 10.

Equation 10

(10a)

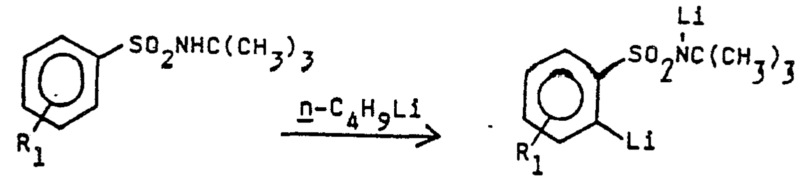


XVII XVIII

(10b)

XVIII +

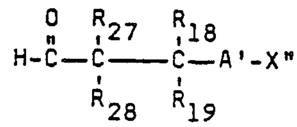

XXI

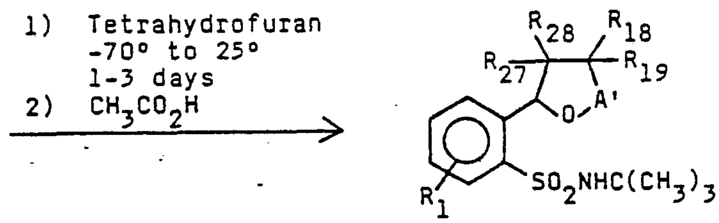


XIX

(10c)

XIX

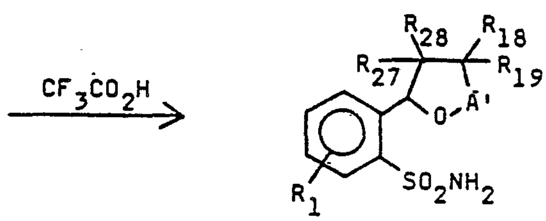


IIg

wherein

A′, $R_1$, $R_{27}$, $R_{28}$, $R_{18}$, $R_{19}$, $R_{20}$, $R_{21}$, $R_{22}$ and $R_{23}$ are as previously defined; and

X″ is Cl or Br.

Reaction (10a):

In this reaction a tert-butyl sulfonamide of Formula XVII is treated with two equivalents of *n*-butyl lithium at −50° to −80° in tetrahydrofuran solution to give the dilithio salt XVIII.

Reaction (10b):

The salt XVIII is then immediately treated with one equivalent of a chloro- or bromoaldehyde of Formula XXI at −70° to −80°. The temperature is raised to 25° for 1 to 3 days, and the reaction is then quenched by the addition of an acid such as acetic acid. The pure product, XIX, is isolated by stripping off the solvent and chromatographing or recrystallizing the crude product.

Reaction (10c):

In this step the tert-butyl group is removed from XIX by heating these compounds in trifluoroacetic acid at 72° for 1—6 hours. The sulfonamides of Formula IIg are isolated in pure form by stripping off the trifluoroacetic acid solvent.

Sulfonamides of Formula IIj, which contain a dihydrofuran or a dihydropyran group, can be prepared by the method shown in Equation 11.

Equation 11

(11a)

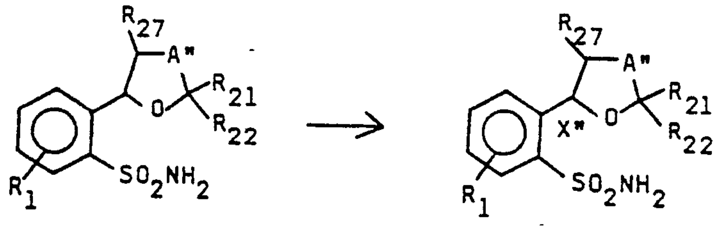

IIh IIi

(11b)

IIi

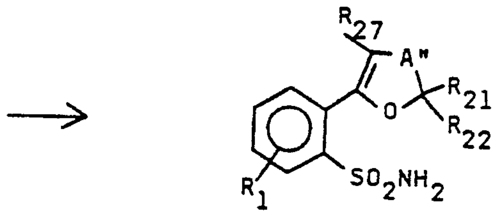

IIj

wherein

A'' is 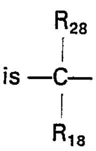 or 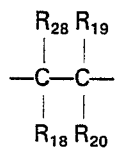

X'' is Cl or Br; and

$R_1$, $R_{27}$, $R_{28}$, $R_{18}$, $R_{19}$, $R_{20}$, $R_{21}$, and $R_{22}$ are as previously defined.

Reaction (11a);

In this reaction a sulfonamide of Formula IIh is treated with one equivalent of N-chlorosuccinimide or N-bromosuccinimide at 0°—60° in a solvent such as carbon tetrachloride or chloroform for 2—24 hours. the by-product succinimide is removed by filtration and the solution carried directly on to the next step.

Reaction (11b):

The sulfonamides of Formula IIi are dehydrohalogenated in carbon tetrachloride or chloroform solution by treatment with at least one equivalent of an appropriate base such as triethylamine, DABCO, pyridine, sodium methoxide or anhydrous potassium carbonate at 0—60° for 1—6 hours. The products are isolated by washing the organic solution with water, drying, and stripping off the solvent. The sulfonamides of Formula IIj can be obtained in pure form by recrystallization from a solvent such as ethanol, 1-chlorobutane, or acetonitrile, or by column chromatography.

Sulfonamides containing a pyrrole group can be prepared by the sequence of reactions shown in Equation 12.

Equation 12

(12a) 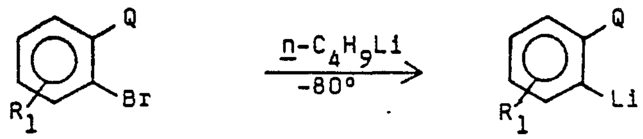


XX XXI

(12b) XXII 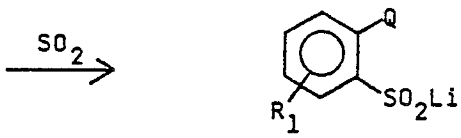


XXIII

(12c) XXIII 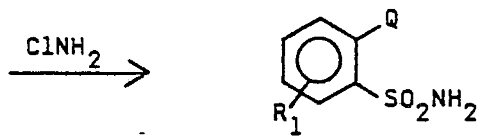


wherein

Q is

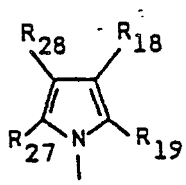 , 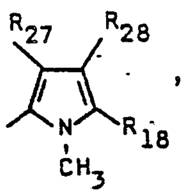 , 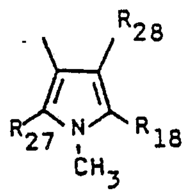 ; and

$R_1$, $R_{27}$, $R_{28}$, $R_{18}$, and $R_{19}$ are as previously defined.

Reaction (12a):

The lithio compounds of Formula XXI are prepared from the bromo compounds of Formula XX by treatment with *n*-butyl lithium at −80° in either hexane/ether or hexane/tetrahydrofuran solvent systems. The details of this type of transmetallation are further described by M. E. K. Cartoon and G. W. H. Cheeseman in *J. Organometallic Chemistry, 212,* 1—9 (1981).

Reaction (12b):

The lithio compounds, XXII, are immediately treated with at least one equivalent of sulfur dioxide at 0° to −50°. After warming to 25°, the lithium sulfinates of Formula XXIII are filtered off, and can be carried directly on to reaction (12c).

Reaction (12c):

In this step chloramine is prepared at 0° by the addition of aqueous sodium hypochlorite to a stirred mixture of 30% ammonium hydroxide solution and diethyl ether. The lithium sulfinates of Formula XXIII are added to this mixture at 0° and the resulting mixture stirred at room temperature for 2—24 hours. The

product sulfonamides are isolated by extraction into a solvent such as ethyl acetate and evaporation of the solvent. The sulfonamides of Formula IIk may be purified by recrystallization from solvents such as ethanol, 1-chlorobutane, or acetonitrile, or by column chromatography.

The bromo compounds of Formula XX can be prepared by methods well known in the art, such as those reviewed by A. R. Jones and G. P. Bean in *The Chemistry of Pyrroles,* Academic Press, New York, N.Y., 1977.

Sulfonamides of Formula II in which Q is Q—11 can be prepared by the sequence of reactions shown in Equation 13.

Reaction (13a):

The dilithio salt of Formula XVIII is prepared as described above in reaction (10a) then treated with a ketone of Formula XXIV at −70° to −80°. The temperature is raised to 25° for 1 to 3 days, and the reaction is then quenched by the addition of an acid such as acetic acid. The product, XXV, is isolated by evaporation of the solvent followed by chromatography or recrystallization.

Equation 13

(13a)

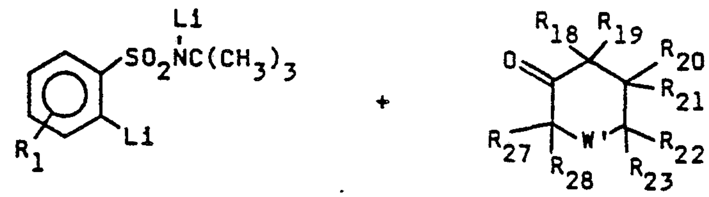

XVIII

XXIV

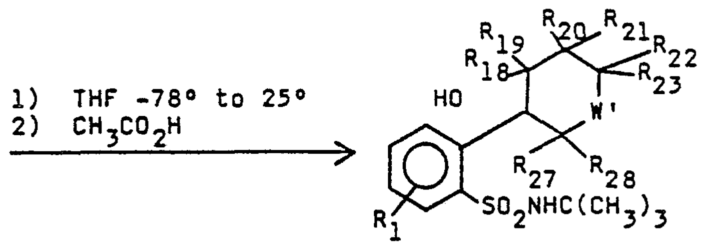


XXV

(13b)

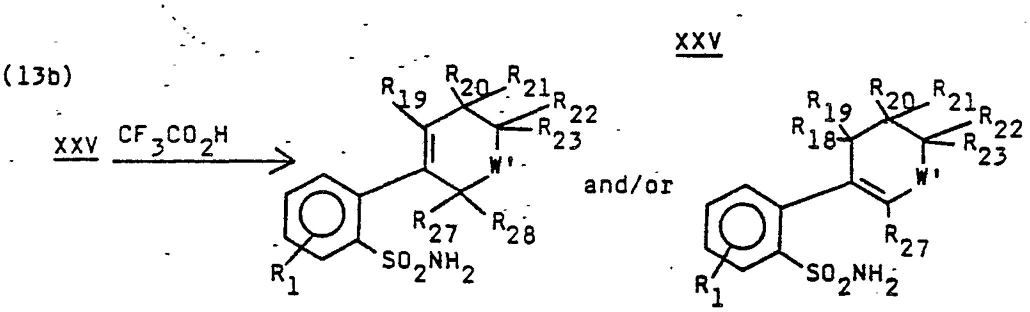


XXVI

(13c)

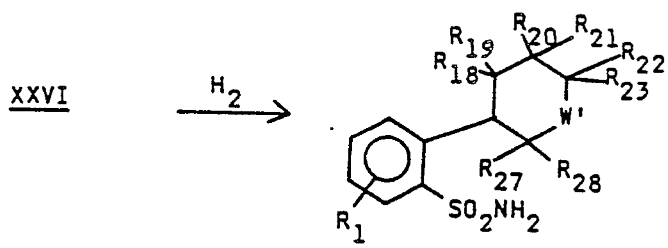


XXVII

wherein

$R_1$, $R_{27}$, $R_{28}$, $R_{18}$, $R_{19}$, $R_{20}$, $R_{21}$, $R_{22}$, $R_{23}$ and W' are as previously defined and at least one of $R_{27}$, $R_{28}$, $R_{18}$ or $R_{19}$ is hydrogen.

Reaction (13b):

Removal of the *tert*-butyl group is accomplished by heating the above products, XXV, in trifluoroacetic acid at 72° for 1—6 hours. Dehydration of the tertiary hydroxyl group occurs simultaneously and the isomeric mixture of olefinic sulfonamides, XXVI, are isolated upon evaporation of the trifluoroacetic acid solvent.

In reaction (13c), sulfonamides of Formula XXVII are produced upon hydrogenation of the olefins, XXVI, under standard conditions.

The *ortho*-heterocyclicbenzenesulfonylureas of Formula Ia can be prepared by reacting the appropriately substituted benzenesulfonamide with an appropriate methyl pyrimidinyl carbamate or methyl triazinyl carbamate in the presence of an equimolar amount of trimethylaluminum according to the procedure of Equation 14. Unless indicated otherwise, all temperatures are in °C.

Equation 14

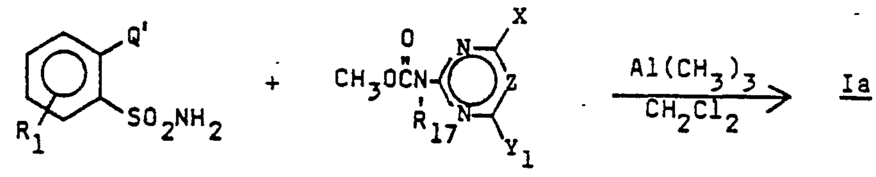


II III

wherein

A, $R_1$, $R_{17}$, Q', X, $Y_1$ and Z are as previously defined.

The reaction of Equation 14 is best carried out in methylene chloride at 25° to 40°C for 24 to 96 hours under a nitrogen atmosphere. The product can be isolated by the addition of an aqueous acetic acid solution followed by extraction of the product into methylene chloride or direct filtration of a product of low solubility. The product can ordinarily be purified by trituration with solvents such as *n*-butyl chloride or ether or by column chromatography.

Further details of this reaction and the preparation of the carbamates of Formula III can be found in U.S. Serial No. 337,934 (EP—A—83975).

Sulfonamides of Formula II may be prepared by the sequence of reactions outlined in Equation 15.

Equation 15

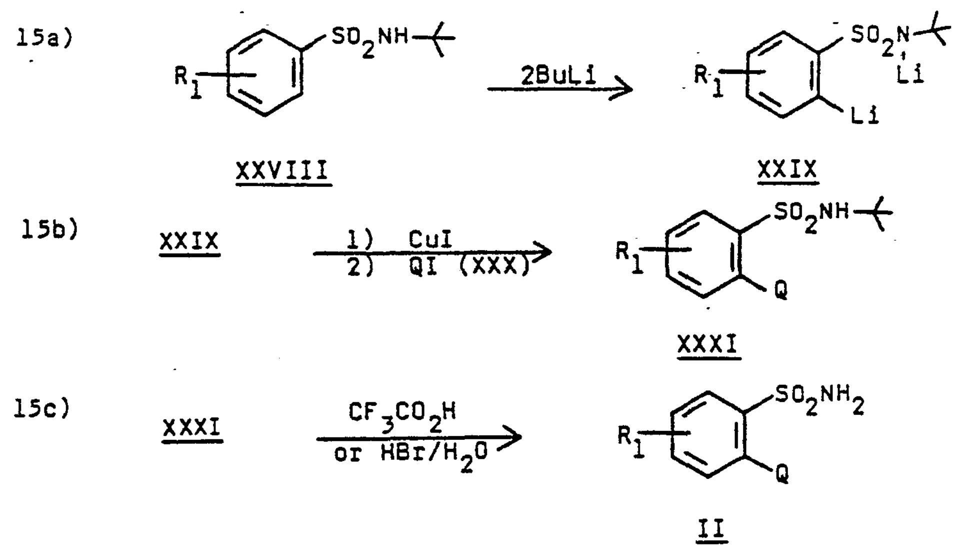


wherein $R_1$ and Q are as previously defined.

The compounds of Formula XXIX are prepared by analogy with the teaching of J. G. Lombardino in *J. Org. Chem., 36,* 1843.

An N-*t*-butyl sulfonamide of Formula XXVIII is dissolved in an ethereal solvent, such as tetrahydrofuran, and two equivalents of *n*-butyllithium in hexane are added at 0—25°. After 1—5 hours at 0—25°, the compound of FOrmula XXIX is formed. This is not isolated, but one equivalent of a copper (I) salt is added at −20 to 0°, followed by 1—1.5 equivalents of an appropriately substituted heteroaromatic iodide (XXX). The reaction mixture is then heated at 40—70° for 1—3 days, concentrated, poured onto aqueous ammonia, and filtered to provide the compound of Formula XXXI. The reaction of Formula 15c is conducted by heating a compound of Formula XXXi with 2—10 equivalents of trifluoroacetic acid or aqueous HBr with or without an inert solvent at 30—70° for 1—3 days. The product II may be isolated as a trifluoroacetate or hydrobromide salt by evaporation of solvent and excess acid and trituration with ether. The free base may be obtained by neutralization of the salt with aqueous base, extraction into an organic solvent, and concentration of the organic extracts.

The compounds of Formula XXX may be prepared according to methods known in the art, such as those reviewed in "The Chemistry of Heterocyclic Compounds," a series published by Interscience Publ., New York and London, the teachings of which are incorporated herein by reference. The iodopyridines are described in Vol. 14 of the above series, pp. 407—488. Iodopyrimidines are described by D. J. Brown and S. F. Mason in Vol. 16 of the above series. The preparation of iodopyrazines is taught by A. Hirschberg and P. E. Spoerri, *J. Org. Chem., 26,* 1907 (1981) and iodopyridazines are described in D. L. Aldons and R. N. Castle in Vol. 28 of the Interscience series, pp. 240—241. The iodo-1,3,5-triazines are described by E. M. Smolin

and L. Rapoport, in Vol. 13 of the above series, and a method for preparing iodo-1,2,4-triazines is taught by A. Rykowski and H. C. van der Plas, in *J. Org. Chem., 45,* 881 (1980).

Agriculturally suitable salts of compounds of Formula I are also useful herbicides and can be prepared in a number of ways known to the art. For example, metal salts can be made by treating compounds of Formula I with a solution of an alkali or alkaline earth metal salt having a sufficiently basic anion (e.g., hydroxide, alkoxide, carbonate or hydride). Quaternary amine salts cna be made by similar techniques. Detailed examples of such techniques are given in United States Patent 4,127,405, the disclosure of which is herein incorporated by reference.

The compounds of this invention and their preparation are further illustrated by the following examples wherein temperatures are given in degrees centigrade and all parts are by weight unless otherwise indicated. Examples 1—3, 5 and 6 illustrate the preparation of intermediates.

Example 1

Methyl 5-[(2-aminosulfonyl)phenyl]furan-2-carboxylate

To a solution of 65.1 g of methyl 5-(2-aminophenyl)furan-2-carboxylate in a mixture of 270 ml of concentrated hydrochloric acid and 75 ml of glacial acetic acid was added a solution of 25.8 g of sodium nitrite in 75 ml of water at −5° to 0° dropwise over a 30-minute period. The resulting solution was stirred an additional 10 minutes at 0° before being slowly added to a mixture of 6.0 g of cuprous chloride and 50 ml of sulfur dioxide in 375 ml of glacial acetic acid at ~5°. This reaction mass was stirred at 0° for 1 hour and at 25° for 2 hours before being poured into 2.2 l of ice water. The intermediate sulfonyl chloride precipitated as an oil and was extracted into 1-chlorobutane, washed with water, 5% sodium bicarbonate solution, and dried over anhydrous magnesium sulfate.

The solution of the sulfonyl chloride in 1-chlorobutane was cooled to 5° and treated with 13.5 ml of anhydrous ammonia at 5—10°. After stirring for 30 minutes at 25°, the reaction mass was filtered and the solid washed well with water and then 1-chlorobutane. The solid was dried *in vacuo* at 60° to give 20.9 g of the title compound, mp = 186.5—188.5°.

NMR (DMSO-$d_6$) δ: 3.8 (s, 2.7 H, $CO_2CH_3$); 7.1—8.3 (m, 8.3 H, 6 aromatics + $SO_2NH_2$).

Example 2

2-(2-Carbomethoxyfur-5-yl)-N-(butylaminocarbonyl)benzenesulfonamide

A mixture of 16.9 g of the product of Example 1, 7.5 g of *n*-butyl isocyanate, and 10.4 g of anhydrous potassium carbonate in 150 ml of 2-butanone was stirred at 80° for 3 hours. The reaction mixture was cooled to 25°, poured into 500 ml of ice water and acidified to pH = 1 with concentrated hydrochloric acid. The precipitated solid was filtered, washed with water, and dried at 60° *in vacuo* to give 22.5 g of the title compound, mp = 145—148°.

NMR (DMSO-$d_6$) δ: 0.7—1.5 (m, 7.5 H, 7 butyl's); 2.8—3.1 (m, 3.0 H, 2 butyl's); 3.9 (s, 2.8 H, $CO_2CH_3$); 6.2—6.5 (t, 0.9 H, NH); 7.0—7.5 (m, 1.9 H, 2 furan's); 7.6—8.5 (m, 4.2 H, 4 aromatics); ~10.3 (broad singlet, 0.7 H, NH).

Example 3

2-(2-Carbomethoxyfur-5-yl)benzenesulfonyl isocyanate

A solution of 19.0 g of the product of Example 2 and 0.1 g of 1,4-diazabicyclo[2.2.2]octane (DABCO) in 90 ml of dry xylenes was heated to reflux temperature (139°). To this solution was added 3.6 ml of liquid phosgene over a period of about 2 hours, keeping the temperature between 125° and 136° by adjusting the rate of phosgene addition. The reaction mixture was cooled to 25°, and filtered under nitrogen to remove DABCO hydrochloride. The filtrate was concentrated *in vacuo* to give the title compound as a viscous, moisture-sensitive oil. This material was carried out to the desired products without further purification.

IR (neat) = 2280 $cm^{-1}$ ($SO_2NCO$); 1760 $cm^{-1}$ (—$CO_2CH_3$).

Example 4

2-(2-Carbomethoxyfur-5-yl)-N-[(4,6-dimethoxypyrimidin-2-yl)aminocarbonyl]benzenesulfonamide

Under a nitrogen atmosphere, a mixture of 2.5 g of the product of Example 3, 0.9 g of 2-amino-4,6-dimethoxypyrimidine, and a few crystals of DABCO in 15 ml of dry acetonitrile was heated at 50—55° for 1 hour, then stirred at 25° overnight. The solid was filtered off, washed with acetonitrile and 1-chlorobutane, and dried at 60° *in vacuo* to give 2.5 g of the title compound, mp = 215—217°(d).

NMR (DMSO-$d_6$) δ: 3.6, 3.8 (two singlets, 8.4 H, 3 $CH_3O$'s); 5.9 (s, 1.2 H, Het-H); 6.9—8.4 (m, 6.2 H, 6 aromatics); 10.7 (s, 1.0 H, NH); 12—13 (broad, 1.1 H, NH).

Anal. Calcd. for $C_{19}H_{18}N_4O_8S$:

C, 49.4; H, 3.9; N, 12.1.

Found:

C, 49.6; H, 4.0; N, 12.4;
49.8; 4.1; 12.7.

Example 5

N-(1,1-dimethylethyl)-2-(2-pyridinyl)benzenesulfonamide

To a solution of 30 g of N-(1,1-dimethylethyl)-1-(2-pyridinyl)benzenesulfonamide in 600 ml of tetrahydrofuran was added 190 ml of 1.6M butyllithium in hexane at −10 to 10°. The reaction mixture was allowed to stir at 25° for 1 hour, then cooled to −10° and 28 g of cuprous iodide was added. After 15 minutes stirring at 0°, 35 g of 2-iodopyidine was added and the mixture was heated to reflux for 16 hours. After cooling to 25°, 25 ml of acetic acid was added and solvent was removed *in vacuo*. A cold solution of aqueous ammonia was added and the precipitate was filtered. The solid was digested with methylene chloride and filtered. The filtrate was concentrated triturated with *n*-chlorobutane, and the solid was filtered to afford 36 g (78%) of product, m.p. 147—150°.

NMR ($CDCl_3$) δ: 1.3 (s, 9), 7.1—8.7 (m, 9).

Example 6

2-(2-pyridinyl)benzenesulfonamide

A solution of 14.8 g of the sulfonamide from Example 5 and 10 ml of 48% hydrobromic acid in 100 ml of methanol was heated at 40—45° for 16 hours, concentrated, and neutralized with aqueous sodium bicarbonate. The solid was filtered, washed with ice-water, and air-dried to afford 6.0 g of product, m.p. 186—190°.

NMR ($CDCl_3$/DMSO-$d_6$) δ: 7.5 (m, 7); 8.1 (m, 2), 8.6 (d, 1).

IR (Nujol) 3300, 3260 $cm^{-1}$.

m/e $M^+$ 234.

Example 7

N-[(4,6-dimethoxypyrimidin-2-yl)aminocarbonyl]-2-(2-pyridinyl)benzenesulfonamide

A solution of 1.2 g of the compound from Example 6 and 1.3 g of methyl N-(4,6-dimethoxypyrimidin-2-yl)carbamate in 50 ml of methylene chloride was stirred under nitrogen and 3.4 ml of a 2N solution of trimethylaluminum in toluene was added. After heating at reflux for 4 days, the solution was cooled to 0°, 3 ml of 2N HCl (aq.) was added, the mixture was partitioned between methylene chloride and water. The organic layer was concentrated and the residue was chromatographed on silica gel to afford 0.9 g of the product, m.p. 175—176°.

NMR (DMSO-$d_6$) δ: 3.8 (s, 6). 6.0 (s, 1), 7.2—8.3 (m).

m/e ($M^+$ + 1) 416.

IR 1700 $cm^{-1}$.

Using the procedures described in the equations and Examples 1—7 above, the compounds described in the following Tables I—XXV can be prepared.

TABLE I

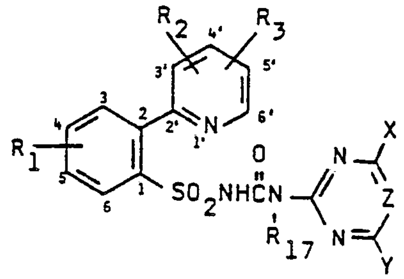

| $R_1$ | $R_2$ | $R_3$ | $R_{17}$ | X | Y | Z | m.p. (°C) |
|---|---|---|---|---|---|---|---|
| H | H | H | H | $CH_3$ | $CH_3$ | CH | 171—174° |
| H | H | H | H | $CH_3$ | $OCH_3$ | CH | 180—184° |
| H | H | H | H | $OCH_3$ | $OCH_3$ | CH | 175—176° |
| H | H | H | H | $CH_3$ | $CH_3$ | N | |
| H | H | H | H | $CH_3$ | $OCH_3$ | N | 160—173° |
| H | H | H | H | $OCH_3$ | $OCH_3$ | N | 172—205° |
| H | H | H | H | $CH_3$ | $CH_2OCH_3$ | CH | |
| H | H | H | H | $CH_3$ | $CH(OCH_3)_2$ | CH | |
| H | H | H | H | $OCH_3$ | $N(CH_3)_2$ | N | |
| H | H | H | H | $OCH_3$ | $NH_2$ | CH | |
| H | H | H | H | Cl | $OCH_3$ | CH | |
| H | H | H | H | $OCH_3$ | $NHCH_3$ | CH | |
| H | H | H | H | $CH_3$ | $CH_2OCH_3$ | N | |
| H | H | H | H | Cl | $CH_3$ | CH | |
| H | H | H | H | $OCH_3$ | $CH(OCH_3)_2$ | N | |
| H | 4′-$CH_3$ | H | H | $CH_3$ | $CH_3$ | CH | |
| H | 4′-$CH_3$ | 6′-$CH_3$ | H | $CH_3$ | $OCH_3$ | CH | |
| H | 5′-$CH_3$ | H | H | $OCH_3$ | $OCH_3$ | N | |
| H | 3′-$CH_3$ | H | H | $OCH_3$ | $OCH_3$ | CH | |
| H | 3′-$CH_3$ | 5′-$CH_3$ | H | $CH_3$ | $OCH_3$ | CH | |
| H | 6′-$CH_3$ | H | H | $OCH_3$ | $CH_3$ | N | |
| H | H | H | $CH_3$ | $CH_3$ | $CH_3$ | CH | |
| H | H | H | $CH_3$ | $OCH_3$ | $OCH_3$ | CH | |
| H | 6′-$CH_3$ | H | H | $CH_3$ | $CH_3$ | N | |
| H | 4′-$CH_3$ | H | H | $OCH_3$ | $CH_2OCH_3$ | N | |

TABLE I (continued)

| $R_1$ | $R_2$ | $R_3$ | $R_{17}$ | X | Y | Z | m.p. (°C) |
|---|---|---|---|---|---|---|---|
| H | 5'-$CH_3$ | H | H | $CH_3$ | $CH_3$ | CH | |
| H | H | H | H | $OCH_3$ | $CH(OCH_3)_2$ | CH | |
| 3-F | H | H | H | $CH_3$ | $CH_3$ | N | |
| 3-F | H | H | H | $OCH_3$ | $CH_3$ | N | |
| 3-F | H | H | H | $CH_3$ | $N(CH_3)_2$ | CH | |
| 3-F | H | H | H | Cl | $OCH_3$ | CH | |
| 3-F | H | H | $CH_3$ | $OCH_3$ | $NH_2$ | N | |
| 3-F | H | H | H | $CH_3$ | $OCH_3$ | N | |
| 4-Cl | H | H | H | $OCH_3$ | $CH_3$ | CH | |
| 4-Cl | H | H | H | $CH_3$ | $OC_2H_5$ | CH | |
| 4-Cl | H | H | H | $OCH_3$ | $C_2H_5$ | N | |
| 4-Cl | 6'-$CH_3$ | H | H | Cl | $CH_2OCH_3$ | N | |
| 4-Cl | H | H | H | $OCH_3$ | $OCH_3$ | CH | |
| 4-Cl | H | H | $CH_3$ | $CH_3$ | $OCH_3$ | CH | |
| 5-$CH_3$ | H | H | H | $OCH_3$ | $NHCH_3$ | N | |
| 5-$CH_3$ | H | H | H | $CH_3$ | $C_2H_5$ | N | |
| 5-$CH_3$ | H | H | H | $OCH_3$ | $CH_3$ | CH | |
| 5-$CH_3$ | H | H | H | $CH_3$ | $CH_3$ | CH | |
| 5-$CH_3$ | H | H | H | $OCH_3$ | $CH_3$ | N | |
| 4-$OCH_3$ | H | H | H | $CH_3$ | $OCH_3$ | N | |
| 4-$OCH_3$ | H | H | H | $OCH_3$ | $OCH_3$ | CH | |
| 4-$OCH_3$ | H | H | H | $CH_3$ | $CH_3$ | CH | |
| 4-$OCH_3$ | H | H | H | $OCH_3$ | $OCH_3$ | N | |

TABLE II

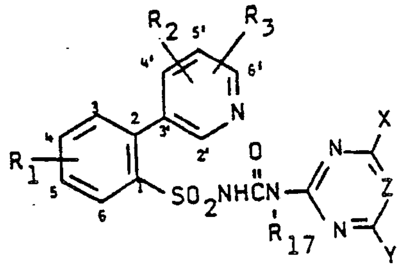

| $R_1$ | $R_2$ | $R_3$ | $R_{17}$ | X | Y | Z | m.p. (°C) |
|---|---|---|---|---|---|---|---|
| H | H | H | H | $CH_3$ | $CH_3$ | CH | |
| H | H | H | H | $CH_3$ | $OCH_3$ | CH | |
| H | H | H | H | $OCH_3$ | $OCH_3$ | CH | |
| H | H | H | H | $CH_3$ | $CH_3$ | N | |
| H | H | H | H | $CH_3$ | $OCH_3$ | N | |
| H | H | H | H | $OCH_3$ | $OCH_3$ | N | |
| H | H | H | H | $CH_3$ | $CH_2OCH_3$ | CH | |
| H | H | H | H | $CH_3$ | $CH(OCH_3)_2$ | CH | |
| H | H | H | H | $OCH_3$ | $N(CH_3)_2$ | N | |
| H | H | H | H | $OCH_3$ | $NH_2$ | CH | |
| H | H | H | H | Cl | $OCH_3$ | CH | |
| H | H | H | H | $OCH_3$ | $NHCH_3$ | CH | |
| H | H | H | H | $CH_3$ | $CH_2OCH_3$ | N | |
| H | H | H | H | Cl | $CH_3$ | CH | |
| H | H | H | H | $OCH_3$ | $CH(OCH_3)_2$ | N | |
| H | 2'-$CH_3$ | H | H | $OCH_3$ | $CH_3$ | CH | |
| H | 5'-$CH_3$ | 6'-$CH_3$ | H | $OCH_3$ | $CH_3$ | N | |
| H | 6'-$CH_3$ | H | H | $CH_3$ | $CH_3$ | CH | |
| H | 5'-$CH_3$ | H | H | $CH_3$ | $OCH_3$ | CH | |
| H | 4'-$CH_3$ | 6'-$CH_3$ | H | $OCH_3$ | $OCH_3$ | CH | |
| H | 2'-$CH_3$ | H | H | $OCH_3$ | $OCH_3$ | N | |
| H | H | H | $CH_3$ | $CH_3$ | $CH_3$ | CH | |
| H | H | H | $CH_3$ | $OCH_3$ | $OCH_3$ | CH | |
| H | 6'-$CH_3$ | H | H | $CH_3$ | $CH_3$ | N | |
| H | H | H | H | $OCH_3$ | $CH_2OCH_3$ | N | |
| H | 4'-$CH_3$ | H | H | $CH_3$ | $CH_3$ | CH | |

TABLE II (continued)

| $R_1$ | $R_2$ | $R_3$ | $R_{17}$ | X | Y | Z | m.p. (°C) |
|---|---|---|---|---|---|---|---|
| H | H | H | H | $OCH_3$ | $CH(OCH_3)_2$ | CH | |
| 6-F | H | H | H | $CH_3$ | $CH_3$ | N | |
| 6-F | H | H | H | $OCH_3$ | $CH_3$ | N | |
| 6-F | H | H | H | $CH_3$ | $N(CH_3)_2$ | CH | |
| 6-F | H | H | H | Cl | $OCH_3$ | CH | |
| 6-F | H | H | $CH_3$ | $OCH_3$ | $NH_2$ | N | |
| 6-F | H | H | H | $CH_3$ | $OCH_3$ | N | |
| 3-Cl | H | H | H | $OCH_3$ | $CH_3$ | CH | |
| 3-Cl | H | H | H | $CH_3$ | $OC_2H_5$ | CH | |
| 3-Cl | H | H | H | $OCH_3$ | $C_2H_5$ | N | |
| 3-Cl | H | H | H | Cl | $CH_2OCH_3$ | N | |
| 3-Cl | H | H | H | $OCH_3$ | $OCH_3$ | CH | |
| 3-Cl | H | H | $CH_3$ | $CH_3$ | $OCH_3$ | CH | |
| 4-$CH_3$ | H | H | H | $OCH_3$ | $NHCH_3$ | N | |
| 4-$CH_3$ | 5'-$CH_3$ | H | H | $CH_3$ | $C_2H_5$ | N | |
| 4-$CH_3$ | H | H | H | $OCH_3$ | $CH_3$ | CH | |
| 4-$CH_3$ | H | H | H | $CH_3$ | $CH_3$ | CH | |
| 4-$CH_3$ | H | H | H | $OCH_3$ | $CH_3$ | N | |
| 5-$OCH_3$ | H | H | H | $CH_3$ | $OCH_3$ | N | |
| 5-$OCH_3$ | H | H | H | $OCH_3$ | $OCH_3$ | CH | |
| 5-$OCH_3$ | H | H | H | $CH_3$ | $CH_3$ | CH | |
| 5-$OCH_3$ | H | H | H | $OCH_3$ | $OCH_3$ | N | |

TABLE III

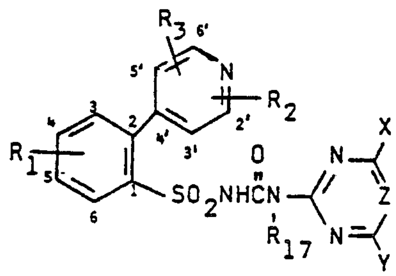

| R₁ | R₂ | R₃ | R₁₇ | X | Y | Z | m.p. (°C) |
|---|---|---|---|---|---|---|---|
| H | H | H | H | $CH_3$ | $CH_3$ | CH | |
| H | H | H | H | $CH_3$ | $OCH_3$ | CH | |
| H | H | H | H | $OCH_3$ | $OCH_3$ | CH | |
| H | H | H | H | $CH_3$ | $CH_3$ | N | |
| H | H | H | H | $CH_3$ | $OCH_3$ | N | |
| H | H | H | H | $OCH_3$ | $OCH_3$ | N | |
| H | H | H | H | $CH_3$ | $CH_2OCH_3$ | CH | |
| H | H | H | H | $CH_3$ | $CH(OCH_3)_2$ | CH | |
| H | H | H | H | $CH_3$ | $N(CH_3)_2$ | N | |
| H | H | H | H | $OCH_3$ | $NH_2$ | CH | |
| H | H | H | H | Cl | $OCH_3$ | CH | |
| H | H | H | H | $OCH_3$ | $NHCH_3$ | CH | |
| H | H | H | H | $CH_3$ | $CH_2OCH_3$ | N | |
| H | H | H | H | Cl | $CH_3$ | CH | |
| H | H | H | H | $OCH_3$ | $CH(OCH_3)_2$ | N | |
| H | 2′-$CH_3$ | H | H | $CH_3$ | $OCH_3$ | N | |
| H | 2′-$CH_3$ | 6′-$CH_3$ | H | $CH_3$ | $OCH_3$ | CH | |
| H | 3′-$CH_3$ | H | H | $CH_3$ | $CH_3$ | CH | |
| H | 2′-$CH_3$ | H | H | $OCH_3$ | $OCH_3$ | N | |
| H | 3′-$CH_3$ | 6′-$CH_3$ | H | $OCH_3$ | $OCH_3$ | CH | |
| H | 2′-$CH_3$ | H | H | $CH_3$ | $OCH_3$ | CH | |
| H | H | H | $CH_3$ | $CH_3$ | $CH_3$ | CH | |
| H | H | H | $CH_3$ | $OCH_3$ | $OCH_3$ | CH | |
| H | 2′-$CH_3$ | H | H | $CH_3$ | $CH_3$ | N | |
| H | H | H | H | $OCH_3$ | $CH_2OCH_3$ | N | |

TABLE III (continued)

| $R_1$ | $R_2$ | $R_3$ | $R_{17}$ | X | Y | Z | m.p. (°C) |
|---|---|---|---|---|---|---|---|
| H | 3'-$CH_3$ | H | H | $CH_3$ | $CH_3$ | CH | |
| H | H | H | H | $OCH_3$ | $CH(OCH_3)_2$ | CH | |
| 5-F | H | H | H | $CH_3$ | $CH_3$ | N | |
| 5-F | H | H | H | $OCH_3$ | $CH_3$ | N | |
| 5-F | H | H | H | $CH_3$ | $N(CH_3)_2$ | CH | |
| 5-F | H | H | H | Cl | $OCH_3$ | CH | |
| 5-F | H | H | $CH_3$ | $OCH_3$ | $NH_2$ | N | |
| 5-F | H | H | H | $CH_3$ | $OCH_3$ | N | |
| 4-Cl | H | H | H | $OCH_3$ | $CH_3$ | CH | |
| 4-Cl | H | H | H | $CH_3$ | $OC_2H_5$ | CH | |
| 4-Cl | H | H | H | $OCH_3$ | $C_2H_5$ | N | |
| 4-Cl | H | H | H | Cl | $CH_2OCH_3$ | N | |
| 4-Cl | H | H | H | $OCH_3$ | $OCH_3$ | CH | |
| 4-Cl | 2'-$CH_3$ | 6'-$CH_3$ | $CH_3$ | $CH_3$ | $OCH_3$ | CH | |
| 3-$CH_3$ | H | H | H | $OCH_3$ | $NHCH_3$ | N | |
| 3-$CH_3$ | H | H | H | $CH_3$ | $C_2H_5$ | N | |
| 3-$CH_3$ | H | H | H | $OCH_3$ | $CH_3$ | CH | |
| 3-$CH_3$ | H | H | H | $CH_3$ | $CH_3$ | CH | |
| 3-$CH_3$ | H | H | H | $OCH_3$ | $CH_3$ | N | |
| 3-$OCH_3$ | H | H | H | $CH_3$ | $OCH_3$ | N | |
| 3-$OCH_3$ | H | H | H | $OCH_3$ | $OCH_3$ | CH | |
| 3-$OCH_3$ | H | H | H | $CH_3$ | $CH_3$ | CH | |
| 3-$OCH_3$ | H | H | H | $OCH_3$ | $OCH_3$ | N | |

TABLE IV

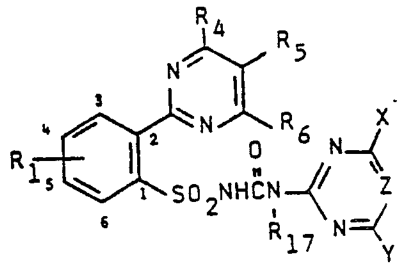

| $R_1$ | $R_4$ | $R_5$ | $R_6$ | $R_{17}$ | X | Y | Z | m.p. (°C) |
|---|---|---|---|---|---|---|---|---|
| H | H | H | H | H | $CH_3$ | $CH_3$ | CH | |
| H | H | H | H | H | $CH_3$ | $OCH_3$ | CH | 178—185° |
| H | H | H | H | H | $OCH_3$ | $OCH_3$ | CH | 197—210° |
| H | H | H | H | H | $CH_3$ | $CH_3$ | N | |
| H | H | H | H | H | $CH_3$ | $OCH_3$ | N | |
| H | H | H | H | H | $OCH_3$ | $OCH_3$ | N | |
| H | H | H | H | H | $CH_3$ | $CH_2OCH_3$ | CH | |
| H | H | H | H | H | $CH_3$ | $CH(OCH_3)_2$ | CH | |
| H | H | H | H | H | $CH_3$ | $N(CH_3)_2$ | CH | |
| H | H | H | H | H | $OCH_3$ | $NH_2$ | CH | |
| H | H | H | H | H | $OCH_3$ | $NHCH_3$ | CH | |
| H | H | H | H | H | $OCH_3$ | $CH(OCH_3)_2$ | CH | |
| H | H | H | H | H | Cl | $OCH_3$ | CH | |
| H | H | H | H | H | $CH_3$ | $CH_2OCH_3$ | N | |
| H | H | H | H | H | $OCH_3$ | $N(CH_3)_2$ | N | |
| H | H | H | H | H | $CH_3$ | $CH(OCH_3)_2$ | N | |
| H | H | H | H | H | $OCH_3$ | $CH_2OCH_3$ | N | |
| H | $CH_3$ | H | H | H | $CH_3$ | $OCH_3$ | N | |
| H | $CH_3$ | H | $CH_3$ | H | $OCH_3$ | $OCH_3$ | N | |
| H | $OCH_3$ | H | $CH_3$ | H | $CH_3$ | $CH(OCH_3)_2$ | N | |
| H | $OCH_3$ | H | H | H | $CH_3$ | $CH(OCH_3)_2$ | N | |
| 3-Cl | H | H | H | H | $CH_3$ | $OCH_3$ | CH | |
| 4-Cl | H | H | H | H | $OCH_3$ | $OCH_3$ | N | |
| 4-Cl | H | H | H | H | $CH_3$ | $OCH_3$ | N | |
| 4-Cl | $CH_3$ | H | $CH_3$ | H | $CH_3$ | $CH_3$ | CH | |
| 4-Cl | $OCH_3$ | H | $OCH_3$ | H | $OCH_3$ | $OCH_3$ | CH | |

TABLE IV (continued)

| $R_1$ | $R_4$ | $R_5$ | $R_6$ | $R_{17}$ | X | Y | Z | m.p. (°C) |
|---|---|---|---|---|---|---|---|---|
| 4-Cl | H | H | H | H | $OCH_3$ | $OCH_3$ | N | |
| 4-Cl | H | $CH_3$ | H | H | $OCH_3$ | $OCH_3$ | CH | |
| 5-Cl | H | H | H | $CH_3$ | $CH_3$ | $OCH_3$ | CH | |
| 3-$CH_3$ | H | H | H | H | $CH_3$ | $CH_3$ | CH | |
| 5-$CH_3$ | H | H | H | H | $OCH_3$ | $CH_2OCH_3$ | N | |
| 4-$CH_3$ | H | H | H | H | $CH_3$ | $CH(OCH_3)_2$ | CH | |
| 4-$CH_3$ | H | H | H | H | $CH_3$ | $N(CH_3)_2$ | N | |
| 4-$CH_3$ | H | H | H | H | $OCH_3$ | $OCH_3$ | CH | |
| 4-$CH_3$ | $CH_3$ | $CH_3$ | $OCH_3$ | H | $OCH_3$ | $CH_3$ | CH | |
| 4-$OCH_3$ | H | H | H | $CH_3$ | $CH_3$ | $CH_3$ | N | |
| 3-$OCH_3$ | $CH_3$ | $CH_3$ | H | H | $OCH_3$ | $OCH_3$ | N | |
| 6-$OCH_3$ | H | H | H | H | $OCH_3$ | $OCH_3$ | N | |
| 6-$OCH_3$ | H | H | H | H | $CH_3$ | $OCH_3$ | CH | |
| 6-$OCH_3$ | H | H | H | H | $CH_3$ | $CH_3$ | CH | |
| 6-F | H | H | H | H | $OCH_3$ | $OCH_3$ | N | |
| 5-F | H | H | H | H | $CH_3$ | $OCH_3$ | N | |
| 3-F | H | H | H | H | $CH_3$ | $CH_2OCH_3$ | CH | |
| 3-F | H | H | H | H | $OCH_3$ | $CH(OCH_3)_2$ | CH | |
| 3-F | H | H | H | H | $CH_3$ | $N(CH_3)_2$ | N | |
| 3-F | H | H | H | $CH_3$ | $CH_3$ | $OCH_3$ | CH | |
| 3-F | $OCH_3$ | $CH_3$ | $OCH_3$ | H | $CH_3$ | $OCH_3$ | N | |

TABLE V

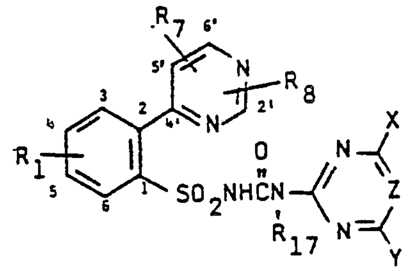

| $R_1$ | $R_7$ | $R_8$ | $R_{17}$ | X | Y | Z | m.p. (°C) |
|---|---|---|---|---|---|---|---|
| H | H | H | H | $CH_3$ | $CH_3$ | CH | |
| H | H | H | H | $CH_3$ | $OCH_3$ | CH | |
| H | H | H | H | $OCH_3$ | $OCH_3$ | CH | |
| H | H | H | H | $CH_3$ | $CH_3$ | N | |
| H | H | H | H | $CH_3$ | $OCH_3$ | N | |
| H | H | H | H | $OCH_3$ | $OCH_3$ | N | |
| H | H | H | H | $CH_3$ | $CH_2OCH_3$ | CH | |
| H | H | H | H | $CH_3$ | $CH(OCH_3)_2$ | CH | |
| H | H | H | H | $OCH_3$ | $N(CH_3)_2$ | N | |
| H | H | H | H | $OCH_3$ | $NH_2$ | CH | |
| H | H | H | H | Cl | $OCH_3$ | CH | |
| H | H | H | H | $OCH_3$ | $NHCH_3$ | CH | |
| H | H | H | H | $CH_3$ | $CH_2OCH_3$ | N | |
| H | H | H | H | Cl | $CH_3$ | CH | |
| H | H | H | H | $OCH_3$ | $CH(OCH_3)_2$ | N | |
| H | 2′-$CH_3$ | H | H | $CH_3$ | $CH_3$ | CH | |
| H | 2′-$CH_3$ | 6′-$OCH_3$ | H | $CH_3$ | $OCH_3$ | N | |
| H | 2′-$OCH_3$ | H | H | $OCH_3$ | $OCH_3$ | N | |
| H | 5′-$CH_3$ | H | H | $OCH_3$ | $OCH_3$ | CH | |
| H | 2′-$OCH_3$ | 5′-$CH_3$ | H | $CH_3$ | $CH_3$ | CH | |
| H | 6′-$CH_3$ | H | H | $OCH_3$ | $CH_3$ | CH | |
| H | H | H | $CH_3$ | $CH_3$ | $CH_3$ | CH | |
| H | H | H | $CH_3$ | $OCH_3$ | $OCH_3$ | CH | |
| H | 2′-$CH_3$ | H | H | $CH_3$ | $CH_3$ | N | |
| H | H | H | H | $OCH_3$ | $CH_2OCH_3$ | N | |
| H | 6′-$CH_3$ | H | H | $CH_3$ | $CH_3$ | CH | |

TABLE V (continued)

| $R_1$ | $R_7$ | $R_8$ | $R_{17}$ | X | Y | Z | m.p. (°C) |
|---|---|---|---|---|---|---|---|
| H | H | H | H | $OCH_3$ | $CH(OCH_3)_2$ | CH | |
| 4-F | H | H | H | $CH_3$ | $CH_3$ | N | |
| 4-F | H | H | H | $OCH_3$ | $CH_3$ | N | |
| 4-F | H | H | H | $CH_3$ | $N(CH_3)_2$ | CH | |
| 4-F | H | H | H | Cl | $OCH_3$ | CH | |
| 4-F | H | H | $CH_3$ | $OCH_3$ | $NH_2$ | N | |
| 4-F | H | H | H | $CH_3$ | $OCH_3$ | N | |
| 5-Cl | H | H | H | $OCH_3$ | $CH_3$ | CH | |
| 5-Cl | H | H | H | $CH_3$ | $OC_2H_5$ | CH | |
| 5-Cl | H | H | H | $OCH_3$ | $C_2H_5$ | N | |
| 5-Cl | H | H | H | Cl | $CH_2OCH_3$ | N | |
| 5-Cl | H | H | H | $OCH_3$ | $OCH_3$ | CH | |
| 5-Cl | H | H | $CH_3$ | $CH_3$ | $OCH_3$ | CH | |
| 3-$CH_3$ | H | H | H | $OCH_3$ | $NHCH_3$ | N | |
| 3-$CH_3$ | H | H | H | $CH_3$ | $C_2H_5$ | N | |
| 3-$CH_3$ | H | H | H | $OCH_3$ | $CH_3$ | CH | |
| 3-$CH_3$ | 2′-$CH_3$ | H | H | $CH_3$ | $CH_3$ | CH | |
| 3-$CH_3$ | H | H | H | $OCH_3$ | $CH_3$ | N | |
| 4-$OCH_3$ | H | H | H | $CH_3$ | $OCH_3$ | N | |
| 4-$OCH_3$ | H | H | H | $OCH_3$ | $OCH_3$ | CH | |
| 4-$OCH_3$ | H | H | H | $CH_3$ | $CH_3$ | CH | |
| 4-$OCH_3$ | H | H | H | $OCH_3$ | $OCH_3$ | N | |

TABLE VI

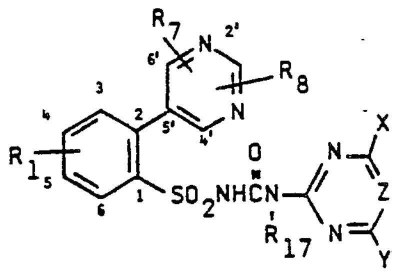

| $R_1$ | $R_7$ | $R_8$ | $R_{17}$ | X | Y | Z | m.p. (°C) |
|---|---|---|---|---|---|---|---|
| H | H | H | H | $CH_3$ | $CH_3$ | CH | |
| H | H | H | H | $CH_3$ | $OCH_3$ | CH | |
| H | H | H | H | $OCH_3$ | $OCH_3$ | CH | |
| H | H | H | H | $CH_3$ | $CH_3$ | N | |
| H | H | H | H | $CH_3$ | $OCH_3$ | N | |
| H | H | H | H | $OCH_3$ | $OCH_3$ | N | |
| H | H | H | H | $CH_3$ | $CH_2OCH_3$ | CH | |
| H | H | H | H | $CH_3$ | $CH(OCH_3)_2$ | CH | |
| H | H | H | H | $OCH_3$ | $N(CH_3)_2$ | N | |
| H | H | H | H | $OCH_3$ | $NH_2$ | CH | |
| H | H | H | H | Cl | $OCH_3$ | CH | |
| H | H | H | H | $OCH_3$ | $NHCH_3$ | CH | |
| H | H | H | H | $CH_3$ | $CH_2OCH_3$ | N | |
| H | H | H | H | Cl | $CH_3$ | CH | |
| H | H | H | H | $OCH_3$ | $CH(OCH_3)_2$ | N | |
| H | 2'-$OCH_3$ | H | H | $CH_3$ | $CH_3$ | CH | |
| H | 2'-$CH_3$ | 4'-$CH_3$ | H | $CH_3$ | $OCH_3$ | N | |
| H | 4'-$OCH_3$ | H | H | $CH_3$ | $CH_3$ | CH | |
| H | 2'-$CH_3$ | H | H | $OCH_3$ | $OCH_3$ | CH | |
| H | 4'-$CH_3$ | 6'-$CH_3$ | H | $OCH_3$ | $OCH_3$ | N | |
| H | 2'-$CH_3$ | H | H | $OCH_3$ | $OCH_3$ | N | |
| H | H | H | $CH_3$ | $CH_3$ | $CH_3$ | CH | |
| H | H | H | $CH_3$ | $OCH_3$ | $OCH_3$ | CH | |
| H | 2'-$CH_3$ | H | H | $CH_3$ | $CH_3$ | N | |
| H | H | H | H | $OCH_3$ | $CH_2OCH_3$ | N | |

TABLE VI (continued)

| $R_1$ | $R_7$ | $R_8$ | $R_{17}$ | X | Y | Z | m.p. (°C) |
|---|---|---|---|---|---|---|---|
| H | 4′-$CH_3$ | 6′-$CH_3$ | H | $CH_3$ | $CH_3$ | CH | |
| H | H | H | H | $OCH_3$ | $CH(OCH_3)_2$ | CH | |
| 5-F | H | H | H | $CH_3$ | $CH_3$ | N | |
| 5-F | H | H | H | $OCH_3$ | $CH_3$ | N | |
| 5-F | H | H | H | $CH_3$ | $N(CH_3)_2$ | CH | |
| 5-F | H | H | H | Cl | $OCH_3$ | CH | |
| 5-F | H | H | $CH_3$ | $OCH_3$ | $NH_2$ | N | |
| 5-F | H | H | H | $CH_3$ | $OCH_3$ | N | |
| 3-Cl | H | H | H | $OCH_3$ | $CH_3$ | CH | |
| 3-Cl | H | H | H | $CH_3$ | $OC_2H_5$ | CH | |
| 3-Cl | H | H | H | $OCH_3$ | $C_2H_5$ | N | |
| 3-Cl | H | H | H | Cl | $CH_2OCH_3$ | N | |
| 3-Cl | H | H | H | $OCH_3$ | $OCH_3$ | CH | |
| 3-Cl | H | H | $CH_3$ | $CH_3$ | $OCH_3$ | CH | |
| 4-$CH_3$ | H | H | H | $OCH_3$ | $NHCH_3$ | N | |
| 4-$CH_3$ | H | H | H | $CH_3$ | $C_2H_5$ | N | |
| 4-$CH_3$ | H | H | H | $OCH_3$ | $CH_3$ | CH | |
| 4-$CH_3$ | H | H | H | $CH_3$ | $CH_3$ | CH | |
| 4-$CH_3$ | 2′-$CH_3$ | H | H | $OCH_3$ | $CH_3$ | N | |
| 5-$OCH_3$ | H | H | H | $CH_3$ | $OCH_3$ | N | |
| 5-$OCH_3$ | H | H | H | $OCH_3$ | $OCH_3$ | CH | |
| 5-$OCH_3$ | H | H | H | $CH_3$ | $CH_3$ | CH | |
| 5-$OCH_3$ | H | H | H | $OCH_3$ | $OCH_3$ | N | |

TABLE VII

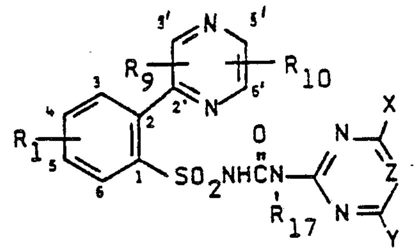

| $R_1$ | $R_9$ | $R_{10}$ | $R_{17}$ | X | Y | Z | m.p. (°C) |
|---|---|---|---|---|---|---|---|
| H | H | H | H | $CH_3$ | $CH_3$ | CH | 212—217°d |
| H | H | H | H | $CH_3$ | $OCH_3$ | CH | 178° |
| H | H | H | H | $OCH_3$ | $OCH_3$ | CH | 178—179° |
| H | H | H | H | $CH_3$ | $CH_3$ | N | |
| H | H | H | H | $CH_3$ | $OCH_3$ | N | |
| H | H | H | H | $OCH_3$ | $OCH_3$ | N | |
| H | H | H | H | $CH_3$ | $CH_2OCH_3$ | CH | |
| H | H | H | H | $CH_3$ | $CH(OCH_3)_2$ | CH | |
| H | H | H | H | $OCH_3$ | $N(CH_3)_2$ | N | |
| H | H | H | H | $OCH_3$ | $NH_2$ | CH | |
| H | H | H | H | Cl | $OCH_3$ | CH | |
| H | H | H | H | $OCH_3$ | $NHCH_3$ | CH | |
| H | H | H | H | $CH_3$ | $CH_2OCH_3$ | N | |
| H | H | H | H | Cl | $CH_3$ | CH | |
| H | H | H | H | $OCH_3$ | $CH(OCH_3)_2$ | N | |
| H | 3′-$CH_3$ | H | H | $CH_3$ | $OCH_3$ | CH | |
| H | 5′-$CH_3$ | 6′-$CH_3$ | H | $CH_3$ | $OCH_3$ | CH | |
| H | 5′-$CH_3$ | H | H | $CH_3$ | $CH_3$ | CH | |
| H | 6′-$CH_3$ | H | H | $OCH_3$ | $OCH_3$ | N | |
| H | 3′-$CH_3$ | 6′-$CH_3$ | H | $OCH_3$ | $CH_3$ | N | |
| H | 3′-$CH_3$ | H | H | $OCH_3$ | $OCH_3$ | CH | |
| H | H | H | $CH_3$ | $CH_3$ | $CH_3$ | CH | |
| H | H | H | $CH_3$ | $OCH_3$ | $OCH_3$ | CH | |
| H | 6′-$CH_3$ | H | H | $CH_3$ | $CH_3$ | N | |
| H | H | H | H | $OCH_3$ | $CH_2OCH_3$ | N | |
| H | 5′-$CH_3$ | 6′-$CH_3$ | H | $CH_3$ | $CH_3$ | CH | |

TABLE VII (continued)

| $R_1$ | $R_2$ | $R_3$ | $R_{17}$ | X | Y | Z | m.p. (°C) |
|---|---|---|---|---|---|---|---|
| H | H | H | H | $OCH_3$ | $CH(OCH_3)_2$ | CH | |
| 3-F | H | H | H | $CH_3$ | $CH_3$ | N | |
| 3-F | H | H | H | $OCH_3$ | $CH_3$ | N | |
| 3-F | H | H | H | $CH_3$ | $N(CH_3)_2$ | CH | |
| 3-F | H | H | H | Cl | $OCH_3$ | CH | |
| 3-F | H | H | $CH_3$ | $OCH_3$ | $NH_2$ | N | |
| 3-F | H | H | H | $CH_3$ | $OCH_3$ | N | |
| 4-Cl | H | H | H | $OCH_3$ | $CH_3$ | CH | |
| 4-Cl | H | H | H | $CH_3$ | $OC_2H_5$ | CH | |
| 4-Cl | H | H | H | $OCH_3$ | $C_2H_5$ | N | |
| 4-Cl | H | H | H | Cl | $CH_2OCH_3$ | N | |
| 4-Cl | 6′-$CH_3$ | H | H | $OCH_3$ | $OCH_3$ | CH | |
| 4-Cl | H | H | $CH_3$ | $CH_3$ | $OCH_3$ | CH | |
| 5-$CH_3$ | H | H | H | $OCH_3$ | $NHCH_3$ | N | |
| 5-$CH_3$ | H | H | H | $CH_3$ | $C_2H_5$ | N | |
| 5-$CH_3$ | H | H | H | $OCH_3$ | $CH_3$ | CH | |
| 5-$CH_3$ | H | H | H | $CH_3$ | $CH_3$ | CH | |
| 5-$CH_3$ | H | H | H | $OCH_3$ | $CH_3$ | N | |
| 6-$OCH_3$ | H | H | H | $CH_3$ | $OCH_3$ | N | |
| 6-$OCH_3$ | H | H | H | $OCH_3$ | $OCH_3$ | CH | |
| 6-$OCH_3$ | H | H | H | $CH_3$ | $CH_3$ | CH | |
| 6-$OCH_3$ | H | H | H | $OCH_3$ | $OCH_3$ | N | |

TABLE VIII

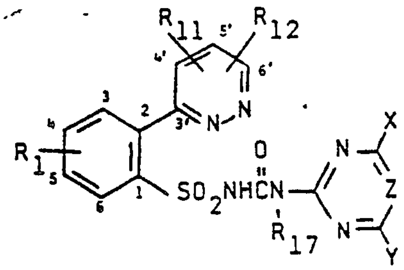

| $R_1$ | $R_{11}$ | $R_{12}$ | $R_{17}$ | X | Y | Z | m.p. (°C) |
|---|---|---|---|---|---|---|---|
| H | H | H | H | $CH_3$ | $CH_3$ | CH | |
| H | H | H | H | $CH_3$ | $OCH_3$ | CH | |
| H | H | H | H | $OCH_3$ | $OCH_3$ | CH | |
| H | H | H | H | $CH_3$ | $CH_3$ | N | |
| H | H | H | H | $CH_3$ | $OCH_3$ | N | |
| H | H | H | H | $OCH_3$ | $OCH_3$ | N | |
| H | H | H | H | $CH_3$ | $CH_2OCH_3$ | CH | |
| H | H | H | H | $CH_3$ | $CH(OCH_3)_2$ | CH | |
| H | H | H | H | $OCH_3$ | $N(CH_3)_2$ | N | |
| H | H | H | H | $OCH_3$ | $NH_2$ | CH | |
| H | H | H | H | Cl | $OCH_3$ | CH | |
| H | H | H | H | $OCH_3$ | $NHCH_3$ | CH | |
| H | H | H | H | $CH_3$ | $CH_2OCH_3$ | N | |
| H | H | H | H | Cl | $CH_3$ | CH | |
| H | H | H | H | $OCH_3$ | $CH(OCH_3)_2$ | N | |
| H | 6'-$CH_3$ | H | H | $CH_3$ | $OCH_3$ | N | |
| H | 4'-$CH_3$ | 6'-$CH_3$ | H | $OCH_3$ | $OCH_3$ | CH | |
| H | 6'-$CH_3$ | H | H | $OCH_3$ | $OCH_3$ | N | |
| H | 5'-$CH_3$ | H | H | $CH_3$ | $CH_3$ | N | |
| H | 4'-$CH_3$ | 5'-$CH_3$ | H | $OCH_3$ | $CH_3$ | CH | |
| H | 6'-$CH_3$ | H | H | $CH_3$ | $OCH_3$ | CH | |
| H | H | H | $CH_3$ | $CH_3$ | $CH_3$ | CH | |
| H | H | H | $CH_3$ | $OCH_3$ | $OCH_3$ | CH | |
| H | 6'-$CH_3$ | H | H | $CH_3$ | $CH_3$ | N | |
| H | H | H | H | $OCH_3$ | $CH_2OCH_3$ | N | |
| H | 4'-$CH_3$ | H | H | $CH_3$ | $CH_3$ | CH | |

TABLE VIII (continued)

| $R_1$ | $R_2$ | $R_3$ | $R_{17}$ | X | Y | Z | m.p. (°C) |
|---|---|---|---|---|---|---|---|
| H | H | H | H | $OCH_3$ | $CH(OCH_3)_2$ | CH | |
| 6-F | H | H | H | $CH_3$ | $CH_3$ | N | |
| 6-F | H | H | H | $OCH_3$ | $CH_3$ | N | |
| 6-F | H | H | H | $CH_3$ | $N(CH_3)_2$ | CH | |
| 6-F | H | H | H | Cl | $OCH_3$ | CH | |
| 6-F | H | H | $CH_3$ | $OCH_3$ | $NH_2$ | N | |
| 6-F | H | H | H | $CH_3$ | $OCH_3$ | N | |
| 5-Cl | H | H | H | $OCH_3$ | $CH_3$ | CH | |
| 5-Cl | H | H | H | $CH_3$ | $OC_2H_5$ | CH | |
| 5-Cl | 6'-$CH_3$ | H | H | $OCH_3$ | $C_2H_5$ | N | |
| 5-Cl | H | H | H | Cl | $CH_2OCH_3$ | N | |
| 5-Cl | H | H | H | $OCH_3$ | $OCH_3$ | CH | |
| 5-Cl | H | H | $CH_3$ | $CH_3$ | $OCH_3$ | CH | |
| 3-$CH_3$ | H | H | H | $OCH_3$ | $NHCH_3$ | N | |
| 3-$CH_3$ | H | H | H | $CH_3$ | $C_2H_5$ | N | |
| 3-$CH_3$ | H | H | H | $OCH_3$ | $CH_3$ | CH | |
| 3-$CH_3$ | H | H | H | $CH_3$ | $CH_3$ | CH | |
| 3-$CH_3$ | H | H | H | $OCH_3$ | $CH_3$ | N | |
| 4-$OCH_3$ | H | H | H | $CH_3$ | $OCH_3$ | N | |
| 4-$OCH_3$ | H | H | H | $OCH_3$ | $OCH_3$ | CH | |
| 4-$OCH_3$ | H | H | H | $CH_3$ | $CH_3$ | CH | |
| 4-$OCH_3$ | H | H | H | $OCH_3$ | $OCH_3$ | N | |

TABLE IX

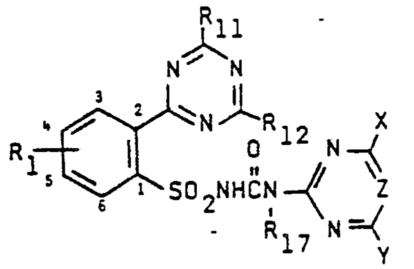

| $R_1$ | $R_{11}$ | $R_{12}$ | $R_{17}$ | X | Y | Z | m.p. (°C) |
|---|---|---|---|---|---|---|---|
| H | $CH_3$ | $CH_3$ | H | $CH_3$ | $CH_3$ | CH | |
| H | $CH_3$ | $CH_3$ | H | $CH_3$ | $OCH_3$ | CH | |
| H | $CH_3$ | $CH_3$ | H | $OCH_3$ | $OCH_3$ | CH | |
| H | $CH_3$ | $CH_3$ | H | $CH_3$ | $CH_3$ | N | |
| H | $CH_3$ | $CH_3$ | H | $CH_3$ | $OCH_3$ | N | |
| H | $CH_3$ | $CH_3$ | H | $OCH_3$ | $OCH_3$ | N | |
| H | $OCH_3$ | $OCH_3$ | H | $CH_3$ | $CH_3$ | CH | |
| H | $OCH_3$ | $OCH_3$ | H | Cl | $OCH_3$ | CH | |
| H | $OCH_3$ | $OCH_3$ | H | $OCH_3$ | $OCH_3$ | CH | |
| H | $OCH_3$ | $OCH_3$ | H | $CH_3$ | $CH_3$ | N | |
| H | $OCH_3$ | $OCH_3$ | H | $CH_3$ | $OCH_3$ | N | |
| H | $OCH_3$ | $OCH_3$ | H | $OCH_3$ | $OCH_3$ | N | |
| H | $OCH_3$ | $CH_3$ | H | $CH_3$ | $CH_3$ | CH | |
| H | $OCH_3$ | $CH_3$ | H | $CH_3$ | $OCH_3$ | CH | |
| H | $OCH_3$ | $CH_3$ | H | $OCH_3$ | $OCH_3$ | CH | |
| H | $OCH_3$ | $CH_3$ | H | $CH_3$ | $CH_3$ | N | |
| H | $OCH_3$ | $CH_3$ | H | $CH_3$ | $OCH_3$ | N | |
| H | $OCH_3$ | $CH_3$ | H | $OCH_3$ | $OCH_3$ | N | |
| H | $OCH_3$ | $CH_3$ | H | $CH_3$ | $CH_2OCH_3$ | CH | |
| H | $OCH_3$ | $CH_3$ | H | $CH_3$ | $N(CH_3)_2$ | CH | |
| H | $OCH_3$ | $CH_3$ | $CH_3$ | $OCH_3$ | $CH_3$ | CH | |
| H | $OCH_3$ | $CH_3$ | $CH_3$ | $CH_3$ | $CH(OCH_3)_2$ | CH | |
| H | $OCH_3$ | $CH_3$ | $CH_3$ | $CH_3$ | $CH_3$ | CH | |
| 3-Cl | $CH_3$ | $CH_3$ | H | $CH_3$ | $CH_3$ | CH | |
| 3-Cl | $CH_3$ | $CH_3$ | H | $CH_3$ | $OCH_3$ | CH | |

TABLE IX (continued)

| $R_1$ | $R_{11}$ | $R_{12}$ | $R_{17}$ | X | Y | Z | m.p. (°C) |
|---|---|---|---|---|---|---|---|
| 3-Cl | $CH_3$ | $CH_3$ | H | $OCH_3$ | $CH_2OCH_3$ | CH | |
| 4-Cl | $CH_3$ | $CH_3$ | H | $CH_3$ | $CH_3$ | N | |
| 5-Cl | $CH_3$ | $CH_3$ | H | $CH_3$ | $OCH_3$ | N | |
| 5-Cl | $CH_3$ | $CH_3$ | H | $OCH_3$ | $CH(OCH_3)_2$ | N | |
| 5-Cl | $OCH_3$ | $OCH_3$ | H | $CH_3$ | $OCH_3$ | CH | |
| 4-$CH_3$ | $OCH_3$ | $OCH_3$ | H | Cl | $CH_3$ | CH | |
| 3-$CH_3$ | $OCH_3$ | $OCH_3$ | H | $OCH_3$ | $OCH_3$ | CH | |
| 6-F | $OCH_3$ | $OCH_3$ | H | $CH_3$ | $CH_3$ | N | |
| 5-$OCH_3$ | $OCH_3$ | $OCH_3$ | H | $CH_3$ | $CH_3$ | N | |
| H | $OCH_3$ | $OCH_3$ | H | $OCH_3$ | $N(CH_3)_2$ | N | |
| H | $OCH_3$ | $CH_3$ | H | $CH_3$ | $NH_2$ | CH | |
| H | $OCH_3$ | $CH_3$ | H | $CH_3$ | $NHCH_3$ | CH | |
| H | $OCH_3$ | $CH_3$ | H | $OCH_3$ | $CH_2OCH_3$ | CH | |
| H | $OCH_3$ | $CH_3$ | H | $CH_3$ | $CH(OCH_3)_2$ | N | |
| H | $OCH_3$ | $CH_3$ | H | $CH_3$ | $NHCH_3$ | N | |
| H | $OCH_3$ | $CH_3$ | H | $OCH_3$ | $CH_2OCH_3$ | N | |
| H | $OCH_3$ | $CH_3$ | H | $CH_3$ | $CH(OCH_3)_2$ | CH | |
| H | $OCH_3$ | $CH_3$ | H | $CH_3$ | $N(CH_3)_2$ | CH | |
| H | $OCH_3$ | $CH_3$ | $CH_3$ | $OCH_3$ | $OCH_3$ | CH | |
| H | $OCH_3$ | $CH_3$ | $CH_3$ | $CH_3$ | $CH_3$ | CH | |
| H | $OCH_3$ | $CH_3$ | $CH_3$ | $CH_3$ | $OCH_3$ | CH | |

TABLE X

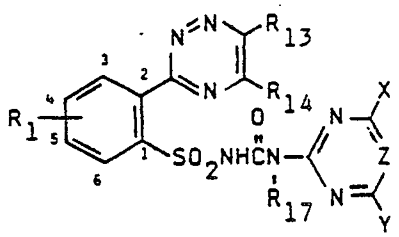

| R1 | R13 | R14 | R17 | X | Y | Z | m.p. (°C) |
|---|---|---|---|---|---|---|---|
| H | $CH_3$ | $CH_3$ | H | $CH_3$ | $CH_3$ | CH | |
| H | $CH_3$ | $CH_3$ | H | $CH_3$ | $OCH_3$ | CH | |
| H | $CH_3$ | $CH_3$ | H | $OCH_3$ | $OCH_3$ | CH | |
| H | $CH_3$ | $CH_3$ | H | $CH_3$ | $CH_3$ | N | |
| H | $CH_3$ | $CH_3$ | H | $CH_3$ | $OCH_3$ | N | |
| H | $CH_3$ | $CH_3$ | H | $OCH_3$ | $OCH_3$ | N | |
| H | $OCH_3$ | $CH_3$ | H | $CH_3$ | $CH_3$ | CH | |
| H | $OCH_3$ | $CH_3$ | H | $CH_3$ | $OCH_3$ | CH | |
| H | $OCH_3$ | $CH_3$ | H | $OCH_3$ | $OCH_3$ | CH | |
| H | $OCH_3$ | $CH_3$ | H | $CH_3$ | $CH_3$ | N | |
| H | $OCH_3$ | $CH_3$ | H | $CH_3$ | $OCH_3$ | N | |
| H | $OCH_3$ | $CH_3$ | H | $OCH_3$ | $OCH_3$ | N | |
| H | $OCH_3$ | $CH_3$ | H | $CH_3$ | $CH_2OCH_3$ | CH | |
| H | $OCH_3$ | $OCH_3$ | H | $CH_3$ | $CH_3$ | CH | |
| H | $OCH_3$ | $OCH_3$ | H | $OCH_3$ | $CH_2OCH_3$ | CH | |
| H | $OCH_3$ | $OCH_3$ | H | $CH_3$ | $OCH_3$ | N | |
| H | $OCH_3$ | $OCH_3$ | H | $OCH_3$ | $OCH_3$ | N | |
| H | $OCH_3$ | $OCH_3$ | H | Cl | $OCH_3$ | CH | |
| H | $OCH_3$ | $OCH_3$ | H | $OCH_3$ | $OCH_3$ | CH | |
| H | $CH_3$ | $CH_3$ | $CH_3$ | $CH_3$ | $CH_3$ | CH | |
| H | $CH_3$ | $OCH_3$ | $CH_3$ | $CH_3$ | $OCH_3$ | CH | |
| H | $OCH_3$ | $OCH_3$ | $CH_3$ | $OCH_3$ | $CH_3$ | N | |
| H | $CH_3$ | $CH_3$ | $CH_3$ | $OCH_3$ | $OCH_3$ | N | |
| 5-$CH_3$ | $CH_3$ | $CH_3$ | H | $CH_3$ | $CH_3$ | CH | |
| 4-Cl | $CH_3$ | $CH_3$ | H | $CH_3$ | $OCH_3$ | CH | |
| 4-Cl | $CH_3$ | $CH_3$ | H | $OCH_3$ | $OCH_3$ | CH | |

TABLE X (continued)

| $R_1$ | $R_{13}$ | $R_{14}$ | $R_{17}$ | X | Y | Z | m.p. (°C) |
|---|---|---|---|---|---|---|---|
| 4-Cl | $CH_3$ | $CH_3$ | H | $CH_3$ | $CH_3$ | N | |
| 6-Cl | $CH_3$ | $CH_3$ | H | $CH_3$ | $OCH_3$ | N | |
| 3-Cl | $CH_3$ | $CH_3$ | H | $OCH_3$ | $OCH_3$ | N | |
| 5-F | $OCH_3$ | $CH_3$ | H | $CH_3$ | $CH_3$ | CH | |
| 5-F | $OCH_3$ | $CH_3$ | H | $CH_3$ | $OCH_3$ | CH | |
| 5-F | $OCH_3$ | $CH_3$ | H | $OCH_3$ | $OCH_3$ | CH | |
| 3-$CH_3$ | $OCH_3$ | $CH_3$ | H | $CH_3$ | $CH_3$ | N | |
| 3-$CH_3$ | $OCH_3$ | $CH_3$ | H | $CH_3$ | $OCH_3$ | N | |
| 3-$CH_3$ | $OCH_3$ | $CH_3$ | H | $OCH_3$ | $OCH_3$ | N | |
| 4-$OCH_3$ | $OCH_3$ | $CH_3$ | H | $CH_3$ | $CH_2OCH_3$ | CH | |
| 4-$OCH_3$ | $OCH_3$ | $OCH_3$ | H | $CH_3$ | $CH_3$ | CH | |
| 4-$OCH_3$ | $OCH_3$ | $OCH_3$ | H | $OCH_3$ | $CH_2OCH_3$ | CH | |
| 3-F | $OCH_3$ | $OCH_3$ | H | $CH_3$ | $OCH_3$ | N | |
| 3-F | $OCH_3$ | $OCH_3$ | H | $OCH_3$ | $OCH_3$ | N | |
| 3-F | $OCH_3$ | $OCH_3$ | H | Cl | $OCH_3$ | CH | |
| 4-$CH_3$ | $OCH_3$ | $OCH_3$ | H | $OCH_3$ | $OCH_3$ | CH | |
| 4-$CH_3$ | $CH_3$ | $CH_3$ | $CH_3$ | $CH_3$ | $CH_3$ | CH | |
| 4-$CH_3$ | $CH_3$ | $OCH_3$ | $CH_3$ | $CH_3$ | $OCH_3$ | CH | |
| H | $OCH_3$ | $OCH_3$ | $CH_3$ | $OCH_3$ | $CH_3$ | N | |
| H | $CH_3$ | $CH_3$ | $CH_3$ | $OCH_3$ | $OCH_3$ | N | |

TABLE XI

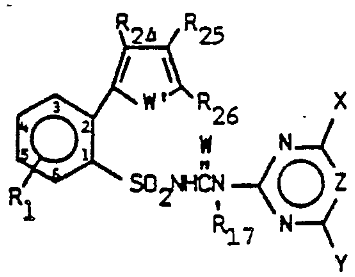

| $R_1$ | W | $R_{24}$ | $R_{25}$ | $R_{17}$ | $R_{26}$ | W' | Z | X | Y | m.p. (°C) |
|---|---|---|---|---|---|---|---|---|---|---|
| H | O | H | H | H | $-CO_2CH_3$ | O | CH | $CH_3O$ | $CH_3O$ | 215—217(d) |
| H | O | H | H | H | $-CO_2CH_3$ | O | N | $CH_3O$ | $CH_3O$ | 210—212(d) |
| H | O | H | H | H | $-CO_2CH_3$ | O | CH | $CH_3O$ | $CH_3$ | 200—202(d) |
| H | O | H | H | H | $-CO_2CH_3$ | O | N | $CH_3O$ | $CH_3$ | 198—199(d) |
| H | O | H | H | H | $-CO_2CH_3$ | O | CH | $CH_3$ | $CH_3$ | 197—199(d) |
| H | O | H | H | H | $-CO_2CH_3$ | O | N | $CH_3$ | $CH_3$ | 205—207(d) |
| H | O | H | H | H | H | O | CH | $CH_3O$ | $CH_3O$ | 168—183(d) |
| H | O | H | H | H | H | O | N | $CH_3O$ | $CH_3O$ | 175(d) |
| H | O | H | H | H | H | O | CH | $CH_3O$ | $CH_3$ | 162—168(d) |
| H | S | H | H | H | H | O | N | $CH_3O$ | $CH_3$ | |
| H | S | H | H | H | H | O | CH | $CH_3$ | $CH_3$ | |
| H | O | H | H | H | H | O | N | $CH_3$ | $CH_3$ | |
| H | O | H | H | $CH_3$ | H | O | N | $CH_3O$ | $CH_3O$ | |
| H | O | H | H | H | H | S | CH | $CH_3O$ | $CH_3O$ | 193—196°d |
| H | O | H | H | H | H | S | CH | $CH_3O$ | $CH_3$ | 218—219°d |
| H | O | H | H | H | H | S | CH | $CH_3$ | $CH_3$ | 211—215° |
| H | O | H | H | H | H | S | N | $CH_3O$ | $CH_3O$ | >260° |
| H | O | H | H | H | H | S | N | $CH_3O$ | $CH_3$ | 190—196°d |
| H | O | H | H | H | H | S | N | $CH_3$ | $CH_3$ | |
| H | O | H | H | H | H | O | N | $CH_3O$ | $CH_3$ | 165—186(d) |
| 5-F | O | H | H | H | H | O | N | $CH_3O$ | $CH_3$ | |
| 5-Cl | O | H | H | H | H | O | N | $CH_3O$ | $CH_3$ | |
| 5-Br | O | H | H | H | H | O | N | $CH_3O$ | $CH_3$ | |
| 5-$CF_3$ | O | H | H | H | H | O | N | $CH_3O$ | $CH_3$ | |
| H | O | H | H | H | H | O | CH | $CH_3$ | $CH_3$ | 162—178(d) |

TABLE XI (continued)

| $R_1$ | W | $R_{24}$ | $R_{25}$ | $R_{17}$ | $R_{26}$ | W' | Z | X | Y | m.p. (°C) |
|---|---|---|---|---|---|---|---|---|---|---|
| 5-$CH_3$ | O | H | H | H | H | O | N | $CH_3O$ | $CH_3$ | |
| 5-$CH_3O$ | O | H | H | H | H | O | N | $CH_3O$ | $CH_3$ | |
| 3-Cl | O | H | H | H | H | O | N | $CH_3O$ | $CH_3$ | |
| 4-Cl | O | H | H | H | H | O | N | $CH_3O$ | $CH_3$ | |
| 6-Cl | O | H | H | H | H | O | N | $CH_3O$ | $CH_3$ | |
| H | O | $CH_3$ | H | H | H | O | N | $CH_3O$ | $CH_3$ | |
| H | O | $C_2H_5$ | H | H | H | O | N | $CH_3O$ | $CH_3$ | |
| H | O | Cl | H | H | H | O | N | $CH_3O$ | $CH_3$ | |
| H | O | Br | H | H | H | O | N | $CH_3O$ | $CH_3$ | |
| H | O | $CH_3O$ | H | H | H | O | N | $CH_3O$ | $CH_3$ | |
| H | O | $C_2H_5O$ | H | H | H | O | N | $CH_3O$ | $CH_3$ | |
| H | O | H | $CH_3$ | H | H | O | N | $CH_3O$ | $CH_3$ | |
| H | O | H | Cl | H | H | O | N | $CH_3O$ | $CH_3$ | |
| H | O | H | $CH_3O$ | H | H | O | N | $CH_3O$ | $CH_3$ | |
| H | O | $CH_3$ | $CH_3$ | H | $CH_3$ | O | N | $CH_3O$ | $CH_3$ | |
| H | S | H | H | H | $CH_3$ | O | N | $CH_3O$ | $CH_3$ | |
| H | O | H | H | H | $C_2H_5$ | O | N | $CH_3O$ | $CH_3$ | |
| H | O | H | H | H | Cl | O | N | $CH_3O$ | $CH_3$ | |
| H | O | H | H | H | Br | O | N | $CH_3O$ | $CH_3$ | |
| H | O | H | H | H | $CH_3O$ | O | N | $CH_3O$ | $CH_3$ | |
| H | O | H | H | H | $C_2H_5O$ | O | N | $CH_3O$ | $CH_3$ | |
| H | O | H | H | H | H | O | N | $CH_3O$ | $CH_3$ | |
| H | O | H | H | H | H | O | CH | Cl | $CH_3O$ | |
| H | O | H | H | H | H | O | CH | Cl | $NH_2$ | |
| H | O | H | H | H | H | O | CH | $CH_3$ | $—CH_2OCH_3$ | |
| H | O | H | H | H | H | O | CH | $CH_3$ | $C_2H_5O$ | |
| H | O | H | H | H | H | O | CH | $CH_3$ | $CH(OCH_3)_2$ | |
| H | O | H | H | H | H | O | CH | $CH_3O$ | $C_2H_5—$ | |
| H | O | H | H | H | H | O | N | $CH_3O$ | $NH_2$ | |
| H | O | H | H | H | H | O | N | $CH_3O$ | $NHCH_3$ | |
| H | O | H | H | H | H | O | N | $CH_3O$ | $N(CH_3)_2$ | |

TABLE XI (continued)

| $R_1$ | W | $R_{24}$ | $R_{25}$ | $R_{17}$ | $R_{26}$ | W' | Z | X | Y | m.p. (°C) |
|---|---|---|---|---|---|---|---|---|---|---|
| H | O | H | H | H | H | O | CH | $CH_3$ | $CH(-OCH_2CH_2O-)$ | |
| H | O | H | H | H | H | O | CH | $CH_3$ | $OCH_2CF_3$ | |
| H | O | H | H | H | H | O | CH | $OCH_3$ | $CF_3$ | |
| H | O | H | H | H | H | O | CH | $CH_3$ | $OCH_2CH_2OCH_3$ | |
| H | O | H | H | H | H | O | N | $CH_3$ | $SCH_3$ | |

TABLE XII

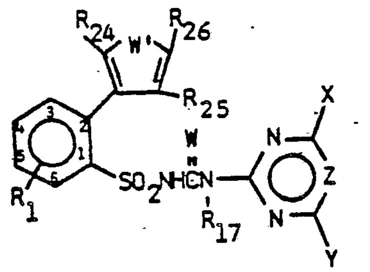

| $R_1$ | W | $R_{24}$ | $R_{25}$ | $R_{17}$ | $R_{26}$ | W' | Z | X | Y | m.p. (°C) |
|---|---|---|---|---|---|---|---|---|---|---|
| H | O | H | H | H | H | O | CH | $CH_3O$ | $CH_3O$ | |
| H | O | H | H | H | H | O | CH | $CH_3O$ | $CH_3$ | |
| H | O | H | H | H | H | O | CH | $CH_3$ | $CH_3$ | |
| H | O | H | H | H | H | O | N | $CH_3O$ | $CH_3O$ | |
| H | O | H | H | H | H | O | N | $CH_3O$ | $CH_3$ | |
| H | O | H | H | H | H | O | N | $CH_3$ | $CH_3$ | |
| H | O | H | H | H | H | S | N | $CH_3O$ | $CH_3$ | |
| H | O | H | H | H | H | S | N | $CH_3O$ | $CH_3O$ | |
| H | O | H | H | H | H | S | CH | $CH_3O$ | $CH_3O$ | |
| H | S | H | H | H | H | S | CH | $CH_3O$ | $CH_3$ | |
| H | O | H | H | H | H | S | CH | $CH_3$ | $CH_3$ | |
| H | O | H | H | $CH_3$ | H | O | N | $CH_3O$ | $CH_3O$ | |
| 5-Cl | O | H | H | H | H | O | N | $CH_3O$ | $CH_3O$ | |
| 5-Br | O | H | H | H | H | O | N | $CH_3O$ | $CH_3$ | |
| 5-$CH_3$ | O | H | H | H | H | O | N | $CH_3O$ | $CH_3$ | |
| 6-Cl | O | H | H | H | H | O | N | $CH_3O$ | $CH_3$ | |
| 6-$CH_3$ | O | H | H | H | H | O | N | $CH_3O$ | $CH_3$ | |
| H | O | $CH_3$ | H | H | H | O | N | $CH_3O$ | $CH_3$ | |

TABLE XII (continued)

| $R_1$ | W | $R_{24}$ | $R_{25}$ | $R_{17}$ | $R_{26}$ | W' | Z | X | Y | m.p. (°C) |
|---|---|---|---|---|---|---|---|---|---|---|
| H | O | $C_2H_5$ | H | H | H | O | N | $CH_3O$ | $CH_3$ | |
| H | O | $CH_3$ | $CH_3$ | H | $CH_3$ | O | N | $CH_3O$ | $CH_3$ | |
| H | O | H | $CH_3$ | H | H | O | N | $CH_3O$ | $CH_3$ | |
| H | S | H | H | H | $C_2H_5$ | O | N | $CH_3O$ | $CH_3$ | |
| H | O | H | H | H | $CO_2CH_3$ | O | N | $CH_3O$ | $CH_3$ | |
| H | O | H | H | H | H | O | N | $CH_3O$ | $CH_3$ | |
| H | O | H | H | H | H | O | CH | $CH_3O$ | $CH_3O$ | |
| H | O | H | H | H | H | O | CH | $CH_3O$ | $CH_3$ | |
| H | O | H | H | H | H | O | CH | Cl | $CH_3O$ | |
| H | O | H | H | H | H | O | CH | $CH_3$ | $CH_2OCH_3$ | |
| H | S | H | H | H | H | O | CH | $CH_3$ | $CH(OCH_3)_2$ | |
| H | O | H | H | H | H | O | N | $CH_3O$ | $NH_2$ | |
| H | O | H | H | H | H | O | N | $CH_3O$ | $NHCH_3$ | |
| H | O | H | H | H | H | O | N | $CH_3O$ | $N(CH_3)_2$ | |
| H | O | H | H | H | H | O | CH | $CH_3$ | $OCH_2CF_3$ | |
| H | O | H | H | H | H | O | CH | $OCH_3$ | $CH(-OCH_2CH_2O-)$ | |

TABLE XIII

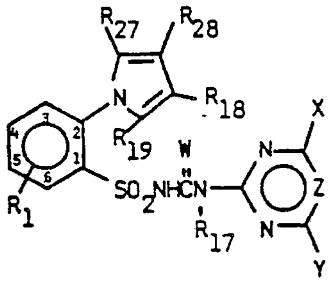

| R1 | W | R27 | R28 | R17 | R18 | R19 | Z | X | Y | m.p. (°C) |
|---|---|---|---|---|---|---|---|---|---|---|
| H | O | H | H | H | H | H | CH | $CH_3O$ | $CH_3O$ | |
| H | O | H | H | H | H | H | CH | $CH_3O$ | $CH_3$ | |
| H | O | H | H | H | H | H | CH | $CH_3$ | $CH_3$ | |
| H | S | H | H | H | H | H | N | $CH_3O$ | $CH_3O$ | |
| H | O | H | H | H | H | H | N | $CH_3O$ | $CH_3$ | |
| H | O | H | H | H | H | H | N | $CH_3$ | $CH_3$ | |
| H | O | $CH_3$ | H | H | H | $CH_3$ | CH | $CH_3O$ | $CH_3O$ | |
| H | O | $CH_3$ | H | H | H | $CH_3$ | CH | $CH_3O$ | $CH_3$ | |
| H | O | $CH_3$ | H | H | H | $CH_3$ | CH | $CH_3$ | $CH_3$ | |
| H | O | $CH_3$ | H | H | H | $CH_3$ | N | $CH_3O$ | $CH_3O$ | |
| H | O | $CH_3$ | H | H | H | $CH_3$ | N | $CH_3O$ | $CH_3$ | |
| H | O | H | H | $CH_3$ | H | H | N | $CH_3O$ | $CH_3O$ | |
| 5-Cl | O | H | H | H | H | H | N | $CH_3O$ | $CH_3O$ | |
| 5-F | O | H | H | H | H | H | N | $CH_3O$ | $CH_3$ | |
| 5-Br | O | H | H | H | H | H | N | $CH_3O$ | $CH_3$ | |
| 5-$CH_3$ | O | H | H | H | H | H | N | $CH_3O$ | $CH_3$ | |
| 5-$CF_3$ | O | H | H | H | H | H | N | $CH_3O$ | $CH_3$ | |
| 5-$OCH_3$ | O | H | H | H | H | H | N | $CH_3O$ | $CH_3$ | |
| 3-Cl | O | H | H | H | H | H | N | $CH_3O$ | $CH_3$ | |
| 4-Cl | O | H | H | H | H | H | N | $CH_3O$ | $CH_3$ | |
| 6-Cl | O | H | H | H | H | H | N | $CH_3O$ | $CH_3$ | |
| H | O | $CH_3$ | $CH_3$ | H | $CH_3$ | $CH_3$ | N | $CH_3O$ | $CH_3$ | |
| H | O | $CH_3$ | H | H | H | H | N | $CH_3O$ | $CH_3$ | |
| H | O | H | $CH_3$ | H | H | H | N | $CH_3O$ | $CH_3$ | |
| H | O | H | $CH_3$ | H | $CH_3$ | H | N | $CH_3O$ | $CH_3$ | |
| H | O | $CH_3$ | $CH_3$ | H | H | H | N | $CH_3O$ | $CH_3$ | |

TABLE XIII (continued)

| R1 | W | R27 | R28 | R17 | R18 | R19 | Z | X | Y | m.p. (°C) |
|---|---|---|---|---|---|---|---|---|---|---|
| H | O | H | H | H | H | H | CH | $CH_3$ | $C_2H_5O$ | |
| H | S | H | H | H | H | H | CH | $CH_3$ | $CH_2OCH_3$ | |
| H | O | H | H | H | H | H | CH | $CH_3$ | $CH(OCH_3)_2$ | |
| H | O | H | H | H | H | H | CH | Cl | $CH_3O$ | |
| H | O | H | H | H | H | H | N | $CH_3O$ | $N(CH_3)_2$ | |
| H | O | H | H | H | H | H | N | $CH_3O$ | $NH_2$ | |

TABLE XIV

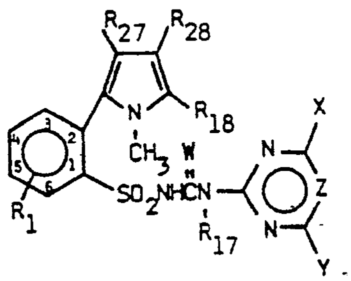

| R1 | W | R27 | R28 | R17 | R18 | Z | X | Y | m.p. (°C) |
|---|---|---|---|---|---|---|---|---|---|
| H | O | H | H | H | H | CH | $CH_3O$ | $CH_3O$ | |
| H | O | H | H | H | H | CH | $CH_3O$ | $CH_3$ | |
| H | O | H | H | H | H | CH | $CH_3$ | $CH_3$ | |
| H | O | H | H | H | H | N | $CH_3O$ | $CH_3O$ | |
| H | O | H | H | H | H | N | $CH_3O$ | $CH_3$ | |
| H | O | H | H | H | $CH_3$ | CH | $CH_3O$ | $CH_3O$ | |
| H | O | H | H | H | $CH_3$ | CH | $CH_3O$ | $CH_3$ | |
| H | S | H | H | H | $CH_3$ | N | $CH_3O$ | $CH_3O$ | |
| H | O | H | H | H | $CH_3$ | N | $CH_3O$ | $CH_3$ | |
| H | O | $CH_3$ | $CH_3$ | H | $CH_3$ | N | $CH_3O$ | $CH_3$ | |
| H | O | $CH_3$ | $CH_3$ | H | H | N | $CH_3O$ | $CH_3$ | |
| H | S | $CH_3$ | H | H | H | N | $CH_3O$ | $CH_3$ | |
| H | O | H | $CH_3$ | H | H | N | $CH_3O$ | $CH_3$ | |
| H | O | H | H | $CH_3$ | H | N | $CH_3O$ | $CH_3O$ | |
| 5-F | O | H | H | H | H | N | $CH_3O$ | $CH_3O$ | |
| 5-Br | O | H | H | H | H | N | $CH_3O$ | $CH_3O$ | |
| 5-$CH_3$ | O | H | H | H | H | N | $CH_3O$ | $CH_3O$ | |

TABLE XIV (continued)

| $R_1$ | W | $R_{27}$ | $R_{28}$ | $R_{17}$ | $R_{18}$ | Z | X | Y | m.p. (°C) |
|---|---|---|---|---|---|---|---|---|---|
| 5-$CH_3O$ | O | H | H | H | H | N | $CH_3O$ | $CH_3O$ | |
| 5-$CF_3$ | O | H | H | H | H | N | $CH_3O$ | $CH_3O$ | |
| 5-Cl | O | H | H | H | H | N | $CH_3O$ | $CH_3O$ | |
| H | O | H | H | H | H | CH | Cl | $CH_3O$ | |
| H | O | H | H | H | H | CH | $CH_3$ | $CH_2OCH_3$ | |
| H | O | H | H | H | H | CH | $CH_3$ | $CH(OCH_3)_2$ | |
| H | O | H | H | H | H | N | $CH_3$ | $C_2H_5O$ | |
| H | O | H | H | H | H | N | $CH_3O$ | $N(CH_3)_2$ | |
| H | O | H | H | H | H | CH | Cl | $NH_2$ | |

TABLE XV

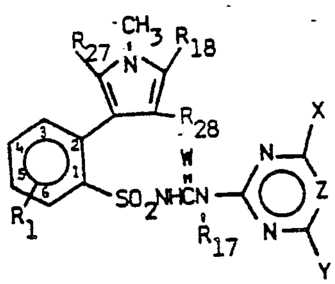

| $R_1$ | W | $R_{27}$ | $R_{28}$ | $R_{17}$ | $R_{18}$ | Z | X | Y | m.p. (°C) |
|---|---|---|---|---|---|---|---|---|---|
| H | O | H | H | H | H | CH | $CH_3O$ | $CH_3O$ | |
| H | O | H | H | H | H | CH | $CH_3O$ | $CH_3$ | |
| H | O | H | H | H | H | CH | $CH_3$ | $CH_3$ | |
| H | O | H | H | H | H | N | $CH_3O$ | $CH_3O$ | |
| H | O | H | H | H | H | N | $CH_3O$ | $CH_3$ | |
| H | O | $CH_3$ | H | H | $CH_3$ | CH | $CH_3O$ | $CH_3O$ | |
| H | O | $CH_3$ | H | H | $CH_3$ | CH | $CH_3O$ | $CH_3$ | |
| H | S | $CH_3$ | H | H | $CH_3$ | N | $CH_3O$ | $CH_3O$ | |
| H | S | $CH_3$ | H | H | $CH_3$ | N | $CH_3O$ | $CH_3$ | |
| H | O | $CH_3$ | $CH_3$ | H | $CH_3$ | N | $CH_3O$ | $CH_3$ | |
| H | O | H | H | $CH_3$ | H | N | $CH_3O$ | $CH_3O$ | |
| H | O | H | $CH_3$ | H | H | N | $CH_3O$ | $CH_3$ | |
| H | O | H | H | H | $CH_3$ | N | $CH_3O$ | $CH_3$ | |
| 5-F | O | H | H | H | H | N | $CH_3O$ | $CH_3$ | |

TABLE XV (continued)

| $R_1$ | W | $R_{27}$ | $R_{28}$ | $R_{17}$ | $R_{18}$ | Z | X | Y | m.p. (°C) |
|---|---|---|---|---|---|---|---|---|---|
| 3-Br | O | H | H | H | H | N | $CH_3O$ | $CH_3$ | |
| 4-$CH_3$ | O | H | H | H | H | N | $CH_3O$ | $CH_3$ | |
| 6-Cl | O | H | H | H | H | N | $CH_3O$ | $CH_3$ | |
| 5-$CF_3$ | O | H | H | H | H | N | $CH_3O$ | $CH_3$ | |
| 5-$CH_3O$ | O | H | H | H | H | N | $CH_3O$ | $CH_3$ | |
| H | O | H | H | H | H | CH | Cl | $CH_3O$ | |
| H | O | H | H | H | H | CH | $CH_3$ | $CH(OCH_3)_2$ | |
| H | O | H | H | H | H | CH | $CH_3O$ | $CH_2OCH_3$ | |
| H | O | H | H | H | H | N | $CH_3O$ | $N(CH_3)_2$ | |
| H | O | H | H | H | H | N | $CH_3$ | $NHCH_3$ | |
| H | O | H | H | H | H | N | $CH_3O$ | $NH_2$ | |
| H | S | H | H | H | H | CH | $CH_3$ | $CH(—OCH_2CH_2O—)$ | |
| H | O | H | H | H | H | CH | $OCH_3$ | $CF_3$ | |

TABLE XVI

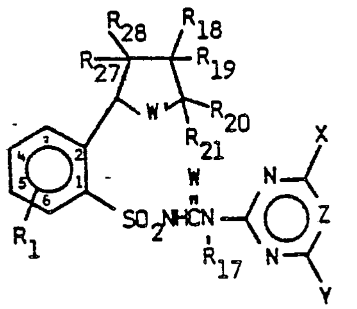

| $R_1$ | W | $R_{17}$ | $R_{27}$ | $R_{28}$ | $R_{18}$ | $R_{19}$ | $R_{20}$ | $R_{21}$ | W' | Z | X | Y | m.p. (°C) |
|---|---|---|---|---|---|---|---|---|---|---|---|---|---|
| H | O | H | H | H | H | H | H | H | O | CH | $CH_3O$ | $CH_3O$ | |
| H | O | H | H | H | H | H | H | H | O | CH | $CH_3O$ | $CH_3$ | |
| H | O | H | H | H | H | H | H | H | O | CH | $CH_3$ | $CH_3$ | |
| H | O | H | H | H | H | H | H | H | O | N | $CH_3O$ | $CH_3O$ | |
| H | O | H | H | H | H | H | H | H | O | N | $CH_3O$ | $CH_3$ | |
| H | O | H | H | H | H | H | H | H | O | N | $CH_3$ | $CH_3$ | |
| H | O | H | H | H | H | H | H | H | S | CH | $CH_3O$ | $CH_3O$ | |
| H | O | H | H | H | H | H | H | H | S | CH | $CH_3O$ | $CH_3$ | |
| H | O | H | H | H | H | H | H | H | S | CH | $CH_3$ | $CH_3$ | |
| H | O | H | H | H | H | H | H | H | S | N | $CH_3O$ | $CH_3O$ | |
| H | S | H | H | H | H | H | H | H | S | N | $CH_3O$ | $CH_3$ | |
| H | O | H | H | H | H | H | H | H | S | N | $CH_3$ | $CH_3$ | |
| H | O | $CH_3$ | H | H | H | H | H | H | O | N | $CH_3O$ | $CH_3O$ | |
| H | O | H | $CH_3$ | $CH_3$ | H | H | H | H | O | N | $CH_3O$ | $CH_3$ | |
| H | O | H | H | H | $CH_3$ | $CH_3$ | H | H | O | N | $CH_3O$ | $CH_3$ | |
| H | O | H | H | H | H | H | $CH_3$ | $CH_3$ | O | N | $CH_3O$ | $CH_3$ | |
| H | O | H | $CH_3$ | H | $CH_3$ | H | $CH_3$ | H | O | N | $CH_3O$ | $CH_3$ | |
| H | O | H | $CH_3$ | H | H | H | H | H | O | N | $CH_3O$ | $CH_3$ | |
| H | O | H | H | H | $CH_3$ | H | H | H | O | N | $CH_3O$ | $CH_3$ | |
| H | O | H | H | H | H | H | $CH_3$ | H | O | N | $CH_3O$ | $CH_3$ | |
| H | O | H | $CH_3$ | H | $CH_3$ | H | $CH_3$ | $CH_3$ | O | N | $CH_3O$ | $CH_3$ | |
| 5-F | O | H | H | H | H | H | H | H | O | N | $CH_3O$ | $CH_3$ | |
| 3-Br | O | H | H | H | H | H | H | H | O | N | $CH_3O$ | $CH_3$ | |
| 4-$CH_3$ | O | H | H | H | H | H | H | H | O | N | $CH_3O$ | $CH_3$ | |
| 6-Cl | O | H | H | H | H | H | H | H | O | N | $CH_3O$ | $CH_3$ | |

TABLE XVI (continued)

| $R_1$ | W | $R_{17}$ | $R_{27}$ | $R_{28}$ | $R_{18}$ | $R_{19}$ | $R_{20}$ | $R_{21}$ | W' | Z | X | Y | m.p. (°C) |
|---|---|---|---|---|---|---|---|---|---|---|---|---|---|
| 5-$CF_3$ | O | H | H | H | H | H | H | H | O | N | $CH_3O$ | $CH_3$ | |
| 5-$CH_3$ | O | H | H | H | H | H | H | H | O | N | $CH_3O$ | $CH_3$ | |
| 5-$CH_3O$ | O | H | H | H | H | H | H | H | O | N | $CH_3O$ | $CH_3$ | |
| 5-Cl | O | H | H | H | H | H | H | H | O | N | $CH_3O$ | $CH_3$ | |
| H | O | H | H | H | H | H | H | H | O | CH | Cl | $CH_3O$ | |
| H | O | H | H | H | H | H | H | H | O | CH | $CH_3$ | $CH(OCH_3)_2$ | |
| H | S | H | H | H | H | H | H | H | O | CH | $CH_3O$ | $—CH_2OCH_3$ | |
| H | O | H | H | H | H | H | H | H | O | N | $CH_3O$ | $N(CH_3)_2$ | |
| H | O | H | H | H | H | H | H | H | O | N | $CH_3O$ | $NHCH_3$ | |
| H | O | H | H | H | H | H | H | H | O | N | $CH_3$ | $NH_2$ | |
| H | O | H | H | H | H | H | H | H | O | N | $CH_3$ | $C_2H_5O—$ | |
| H | O | H | H | H | H | H | H | H | O | N | $CH_3O$ | $C_2H_5—$ | |
| H | O | H | H | H | H | H | H | H | O | CH | $CH_3$ | $OCH_2CH_2OCH_3$ | |

TABLE XVII

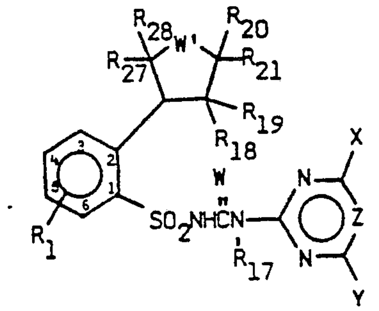

| $R_1$ | W | $R_{17}$ | $R_{27}$ | $R_{28}$ | $R_{18}$ | $R_{19}$ | $R_{20}$ | $R_{21}$ | W' | Z | X | Y | m.p. (°C) |
|---|---|---|---|---|---|---|---|---|---|---|---|---|---|
| H | O | H | H | H | H | H | H | H | O | CH | $CH_3O$ | $CH_3O$ | |
| H | O | H | H | H | H | H | H | H | O | CH | $CH_3O$ | $CH_3$ | |
| H | S | H | H | H | H | H | H | H | O | CH | $CH_3$ | $CH_3$ | |
| H | O | H | H | H | H | H | H | H | O | N | $CH_3O$ | $CH_3O$ | |
| H | O | H | H | H | H | H | H | H | O | N | $CH_3O$ | $CH_3$ | |
| H | O | H | H | H | H | H | H | H | O | N | $CH_3$ | $CH_3$ | |
| H | O | $CH_3$ | H | H | H | H | H | H | O | N | $CH_3O$ | $CH_3$ | |
| H | O | H | H | H | H | H | H | H | S | CH | $CH_3O$ | $CH_3O$ | |
| H | O | H | H | H | H | H | H | H | S | CH | $CH_3O$ | $CH_3$ | |
| H | S | H | H | H | H | H | H | H | S | CH | $CH_3$ | $CH_3$ | |
| H | O | H | H | H | H | H | H | H | S | N | $CH_3O$ | $CH_3O$ | |
| H | O | H | H | H | H | H | H | H | S | N | $CH_3O$ | $CH_3$ | |
| H | O | H | H | H | H | H | H | H | S | N | $CH_3$ | $CH_3$ | |
| H | O | H | $CH_3$ | $CH_3$ | H | H | H | H | O | N | $CH_3O$ | $CH_3$ | |
| H | O | H | H | H | H | H | $CH_3$ | $CH_3$ | O | N | $CH_3O$ | $CH_3$ | |
| H | O | H | $CH_3$ | H | $CH_3$ | H | $CH_3$ | H | O | N | $CH_3O$ | $CH_3$ | |
| H | O | H | $CH_3$ | $CH_3$ | H | H | $CH_3$ | $CH_3$ | O | N | $CH_3O$ | $CH_3$ | |
| 5-F | O | H | H | H | H | H | H | H | O | N | $CH_3O$ | $CH_3$ | |
| 5-Br | O | H | H | H | H | H | H | H | O | N | $CH_3O$ | $CH_3$ | |
| 5-$CH_3$ | O | H | H | H | H | H | H | H | O | N | $CH_3O$ | $CH_3$ | |
| 5-$CF_3$ | O | H | H | H | H | H | H | H | O | N | $CH_3O$ | $CH_3$ | |
| 5-Cl | O | H | H | H | H | H | H | H | O | N | $CH_3O$ | $CH_3$ | |
| H | O | H | H | H | H | H | H | H | O | CH | $CH_3$ | $CH_3$ | |
| 5-$CH_3O$ | O | H | H | H | H | H | H | H | O | N | $CH_3O$ | $CH_3$ | |
| 3-Cl | O | H | H | H | H | H | H | H | O | N | $CH_3O$ | $CH_3$ | |

TABLE XVII (continued)

| $R_1$ | W | $R_{17}$ | $R_{27}$ | $R_{28}$ | $R_{18}$ | $R_{19}$ | $R_{20}$ | $R_{21}$ | W' | Z | X | Y | m.p. (°C) |
|---|---|---|---|---|---|---|---|---|---|---|---|---|---|
| 4-Cl | O | H | H | H | H | H | H | H | O | N | $CH_3O$ | $CH_3$ | |
| 6-Cl | O | H | H | H | H | H | H | H | O | N | $CH_3O$ | $CH_3$ | |
| H | O | H | H | H | H | H | H | H | O | CH | Cl | $CH_3O$ | |
| H | O | H | H | H | H | H | H | H | O | CH | $CH_3O$ | $CH_2OCH_3$ | |
| H | O | H | H | H | H | H | H | H | O | CH | $CH_3O$ | $CH(OCH_3)_2$ | |
| H | O | H | H | H | H | H | H | H | O | N | $CH_3O$ | $N(CH_3)_2$ | |
| H | O | H | H | H | H | H | H | H | O | N | $CH_3$ | $NHCH_3$ | |
| H | O | H | H | H | H | H | H | H | O | N | $CH_3O$ | $NH_2$ | |
| H | O | H | H | H | H | H | H | H | O | N | $CH_3$ | $C_2H_5O$ | |
| H | O | H | H | H | H | H | H | H | O | N | $CH_3O$ | $C_2H_5$ | |
| H | O | H | H | H | H | H | H | H | O | N | $CH_3$ | $OCH_2CF_3$ | |

TABLE XVIII

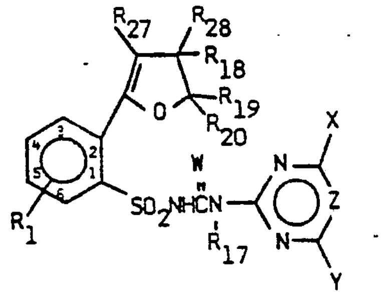

| $R_1$ | W | $R_{17}$ | $R_{27}$ | $R_{28}$ | $R_{18}$ | $R_{19}$ | $R_{20}$ | Z | X | Y | m.p. (°C) |
|---|---|---|---|---|---|---|---|---|---|---|---|
| H | O | H | H | H | H | H | H | CH | $CH_3O$ | $CH_3O$ | |
| H | O | H | H | H | H | H | H | CH | $CH_3O$ | $CH_3$ | |
| H | O | H | H | H | H | H | H | CH | $CH_3$ | $CH_3$ | |
| H | O | H | H | H | H | H | H | N | $CH_3O$ | $CH_3O$ | |
| H | O | H | H | H | H | H | H | N | $CH_3O$ | $CH_3$ | |
| H | O | H | H | H | H | H | H | N | $CH_3$ | $CH_3$ | |
| H | O | $CH_3$ | H | H | H | H | H | N | $CH_3O$ | $CH_3$ | |
| H | S | H | $CH_3$ | H | H | H | H | N | $CH_3O$ | $CH_3$ | |
| H | O | H | $CH_3$ | $CH_3$ | H | H | H | N | $CH_3O$ | $CH_3$ | |
| H | O | H | H | $CH_3$ | $CH_3$ | H | H | N | $CH_3O$ | $CH_3$ | |
| H | O | H | H | H | H | $CH_3$ | $CH_3$ | N | $CH_3O$ | $CH_3$ | |
| H | O | H | $CH_3$ | H | $CH_3$ | $CH_3$ | H | N | $CH_3O$ | $CH_3$ | |
| H | O | H | H | $CH_3$ | $CH_3$ | $CH_3$ | $CH_3$ | N | $CH_3O$ | $CH_3$ | |
| H | O | H | $CH_3$ | $CH_3$ | H | H | $CH_3$ | N | $CH_3O$ | $CH_3$ | |
| 5-F | O | H | H | H | H | H | H | N | $CH_3O$ | $CH_3$ | |
| 5-Cl | O | H | H | H | H | H | H | N | $CH_3O$ | $CH_3$ | |
| 5-$CH_3$ | O | H | H | H | H | H | H | N | $CH_3O$ | $CH_3$ | |
| 5-Br | O | H | H | H | H | H | H | N | $CH_3O$ | $CH_3$ | |
| 5-$CF_3$ | O | H | H | H | H | H | H | N | $CH_3O$ | $CH_3$ | |
| 5-$CH_3O$ | O | H | H | H | H | H | H | N | $CH_3O$ | $CH_3$ | |
| 3-Cl | O | H | H | H | H | H | H | N | $CH_3O$ | $CH_3$ | |
| 4-Cl | O | H | H | H | H | H | H | N | $CH_3O$ | $CH_3$ | |

TABLE XVIII (continued)

| $R_1$ | W | $R_{17}$ | $R_{27}$ | $R_{28}$ | $R_{18}$ | $R_{19}$ | $R_{20}$ | $R_{21}$ | W′ | Z | X | Y | m.p. (°C) |
|---|---|---|---|---|---|---|---|---|---|---|---|---|---|
| 6-Cl | O | H | H | H | H | H | H | H | O | N | $CH_3O$ | $CH_3$ | |
| H | O | H | H | H | H | H | H | H | O | CH | Cl | $CH_3O$ | |
| H | O | H | H | H | H | H | H | H | O | CH | Cl | $NH_2$ | |
| H | O | H | H | H | H | H | H | H | O | CH | $CH_3$ | $CH_2OCH_3$ | |
| H | O | H | H | H | H | H | H | H | O | CH | $CH_3O$ | $CH(OCH_3)_2$ | |
| H | O | H | H | H | H | H | H | H | O | N | $CH_3O$ | $N(CH_3)_2$ | |
| H | O | H | H | H | H | H | H | H | O | N | $CH_3$ | $NH_2$ | |
| H | O | H | H | H | H | H | H | H | O | N | $CH_3O$ | $C_2H_5O$ | |
| H | S | H | H | H | H | H | H | H | O | N | $CH_3O$ | $C_2H_5$ | |
| H | O | H | H | H | H | H | H | H | O | N | $CH_3O$ | $NH(CH_3)_2$ | |

TABLE XIX

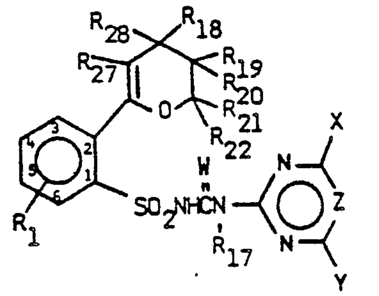

| $R_1$ | W | $R_{17}$ | $R_{27}$ | $R_{28}$ | $R_{18}$ | $R_{19}$ | $R_{20}$ | $R_{21}$ | $R_{22}$ | Z | X | Y | m.p. (°C) |
|---|---|---|---|---|---|---|---|---|---|---|---|---|---|
| H | O | H | H | H | H | H | H | H | H | CH | $CH_3O$ | $CH_3O$ | |
| H | S | H | H | H | H | H | H | H | H | CH | $CH_3O$ | $CH_3$ | |
| H | O | H | H | H | H | H | H | H | H | CH | $CH_3$ | $CH_3$ | |
| H | O | H | H | H | H | H | H | H | H | N | $CH_3O$ | $CH_3O$ | |
| H | O | H | H | H | H | H | H | H | H | N | $CH_3O$ | $CH_3$ | |
| H | O | H | H | H | H | H | H | H | H | N | $CH_3$ | $CH_3$ | |
| H | O | $CH_3$ | H | H | H | H | H | H | H | N | $CH_3O$ | $CH_3O$ | |
| H | O | H | $CH_3$ | H | H | H | H | H | H | N | $CH_3O$ | $CH_3$ | |
| H | O | H | H | $CH_3$ | $CH_3$ | H | H | H | H | N | $CH_3O$ | $CH_3$ | |
| H | O | H | H | H | H | $CH_3$ | $CH_3$ | H | H | N | $CH_3O$ | $CH_3$ | |
| H | O | H | H | H | H | H | H | $CH_3$ | $CH_3$ | N | $CH_3O$ | $CH_3$ | |
| H | O | H | $CH_3$ | H | H | H | H | $CH_3$ | $CH_3$ | N | $CH_3O$ | $CH_3$ | |
| H | S | H | $CH_3$ | $CH_3$ | H | $CH_3$ | H | $CH_3$ | H | N | $CH_3O$ | $CH_3$ | |
| 5-Cl | O | H | H | H | H | H | H | H | H | N | $CH_3O$ | $CH_3$ | |
| 5-F | O | H | H | H | H | H | H | H | H | N | $CH_3O$ | $CH_3$ | |
| 5-$CH_3$ | O | H | H | H | H | H | H | H | H | N | $CH_3O$ | $CH_3$ | |
| 5-Br | O | H | H | H | H | H | H | H | H | N | $CH_3O$ | $CH_3$ | |
| 5-$CF_3$ | O | H | H | H | H | H | H | H | H | N | $CH_3O$ | $CH_3$ | |
| 5-$CH_3O$ | O | H | H | H | H | H | H | H | H | N | $CH_3O$ | $CH_3$ | |
| 3-Cl | O | H | H | H | H | H | H | H | H | N | $CH_3O$ | $CH_3$ | |
| 4-Cl | O | H | H | H | H | H | H | H | H | N | $CH_3O$ | $CH_3$ | |
| 6-Cl | O | H | H | H | H | H | H | H | H | N | $CH_3O$ | $CH_3$ | |
| H | O | H | H | H | H | H | H | H | H | CH | Cl | $CH_3O$ | |
| H | O | H | H | H | H | H | H | H | H | CH | Cl | $NH_2$ | |
| H | O | H | H | H | H | H | H | H | H | CH | $CH_3$ | $CH_2OCH_3$ | |

TABLE XIX (continued)

| $R_1$ | W | $R_{17}$ | $R_{27}$ | $R_{28}$ | $R_{18}$ | $R_{19}$ | $R_{20}$ | $R_{21}$ | $R_{22}$ | Z | X | Y | m.p. (°C) |
|---|---|---|---|---|---|---|---|---|---|---|---|---|---|
| H | O | H | H | H | H | H | H | H | H | CH | $CH_3$ | $CH(OCH_3)_2$ | |
| H | O | H | H | H | H | H | H | H | H | N | $CH_3O$ | $N(CH_3)_2$ | |
| H | O | H | H | H | H | H | H | H | H | N | $CH_3$ | $NH_2$ | |
| H | O | H | H | H | H | H | H | H | H | N | $CH_3O$ | $NHCH_3$ | |
| H | O | H | H | H | H | H | H | H | H | N | $CH_3O$ | $C_2H_5O$ | |
| H | O | H | H | H | H | H | H | H | H | N | $CH_3O$ | $C_2H_5$ | |
| H | O | H | H | H | H | H | H | H | H | CH | $CH_3$ | $SCH_3$ | |

TABLE XX

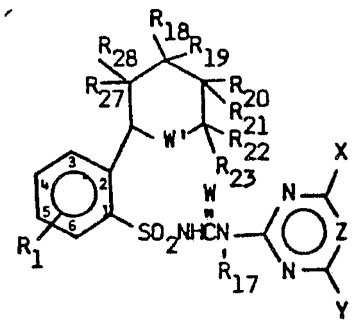

| $R_1$ | W | W' | $R_{27}$ | $R_{28}$ | $R_{17}$ | $R_{18}$ | $R_{19}$ | $R_{20}$ | $R_{21}$ | $R_{22}$ | $R_{23}$ | Z | X | Y | m.p. (°C) |
|---|---|---|---|---|---|---|---|---|---|---|---|---|---|---|---|
| H | O | O | H | H | H | H | H | H | H | H | H | CH | $CH_3$ | $CH_3$ | |
| H | O | O | H | H | H | H | H | H | H | H | H | CH | $CH_3$ | $OCH_3$ | |
| H | O | O | H | H | H | H | H | H | H | H | H | CH | $OCH_3$ | $OCH_3$ | |
| H | O | O | H | H | H | H | H | H | H | H | H | N | $CH_3$ | $OCH_3$ | |
| H | S | O | H | H | H | H | H | H | H | H | H | N | $OCH_3$ | $OCH_3$ | |
| H | O | O | H | H | H | H | H | H | H | H | H | N | $OCH_3$ | $OCH_3$ | |
| H | O | S | H | H | $CH_3$ | H | H | H | H | H | H | N | $CH_3$ | $OCH_3$ | |
| H | S | S | H | H | H | H | H | H | H | H | H | CH | Cl | $OCH_3$ | |
| H | O | S | H | H | H | H | H | H | H | H | H | CH | $OCH_3$ | $CF_3$ | |
| H | O | S | H | H | H | H | $CH_3$ | H | H | H | H | CH | $CH_3$ | $SCH_3$ | |
| H | O | O | $CH_3$ | $CH_3$ | H | H | H | H | H | H | H | N | $CH_3$ | $OCH_2CF_3$ | |
| H | O | O | $CH_3$ | H | H | $CH_3$ | H | H | H | H | H | N | $OCH_3$ | $OC_2H_5$ | |
| H | O | O | H | H | H | H | H | $CH_3$ | H | H | H | N | $OCH_3$ | $CH_3$ | |
| H | O | O | $CH_3$ | $CH_3$ | H | H | H | H | H | $CH_3$ | $CH_3$ | CH | $CH_3$ | $C_2H_5$ | |

TABLE XX (continued)

| $R_1$ | W | W' | $R_{27}$ | $R_{28}$ | $R_{17}$ | $R_{18}$ | $R_{19}$ | $R_{20}$ | $R_{21}$ | $R_{22}$ | $R_{23}$ | Z | X | Y | m.p. (°C) |
|---|---|---|---|---|---|---|---|---|---|---|---|---|---|---|---|
| H | O | O | H | H | H | H | H | H | $CH_3$ | H | H | CH | $OCH_3$ | $CH_3$ | |
| H | O | O | H | H | H | H | H | H | H | $CH_3$ | H | CH | $CH_3$ | $CH_3$ | |
| H | O | O | H | H | H | H | H | H | H | H | H | N | $OCH_3$ | $OCH_2CH_2OCH_3$ | |
| H | O | S | H | H | H | H | H | H | H | H | H | N | $CH_3$ | $OCH_3$ | |
| H | O | S | H | H | H | H | H | H | H | H | H | N | $CH_3$ | $CH(-OCH_2CH_2O-)$ | |
| 5-Cl | O | O | H | H | H | H | H | H | H | H | H | CH | $OCH_3$ | $CH_3$ | |
| 5-$CH_3$ | O | O | H | H | H | H | H | H | H | H | H | CH | Cl | $OCH_3$ | |
| 6-Cl | O | O | H | H | H | H | H | H | H | H | H | CH | $OCH_3$ | $CH_2OCH_3$ | |
| 4-$CH_3$ | O | O | H | H | H | H | H | H | H | H | H | CH | $CH_3$ | $OCH_3$ | |

TABLE XXI

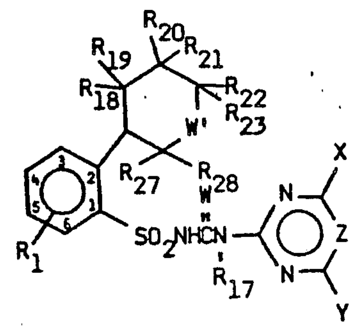

| $R_1$ | W | W' | $R_{27}$ | $R_{28}$ | $R_{17}$ | $R_{18}$ | $R_{19}$ | $R_{20}$ | $R_{21}$ | $R_{22}$ | $R_{23}$ | Z | X | Y | m.p. (°C) |
|---|---|---|---|---|---|---|---|---|---|---|---|---|---|---|---|
| H | O | O | H | H | H | H | H | H | H | H | H | CH | $CH_3$ | $CH_3$ | |
| H | O | O | H | H | H | H | H | H | H | H | H | CH | $CH_3$ | $OCH_3$ | |
| H | O | O | H | H | H | H | H | H | H | H | H | CH | $OCH_3$ | $OCH_3$ | |
| H | O | O | H | H | H | H | H | H | H | H | H | N | $CH_3$ | $OCH_3$ | |
| H | S | O | H | H | H | H | H | H | H | H | H | N | $OCH_3$ | $OCH_3$ | |
| H | O | O | H | H | H | H | H | H | H | H | H | N | $OCH_3$ | $OCH_3$ | |
| H | O | S | H | H | $CH_3$ | H | H | H | H | H | H | N | $CH_3$ | $OCH_3$ | |
| H | S | S | H | H | H | H | H | H | H | H | H | CH | Cl | $OCH_3$ | |
| H | O | S | H | H | H | H | H | H | H | H | H | CH | $OCH_3$ | $CF_3$ | |
| H | O | S | H | H | H | H | $CH_3$ | H | H | H | H | CH | $CH_3$ | $SCH_3$ | |
| H | O | O | $CH_3$ | $CH_3$ | H | H | H | H | H | H | H | N | $CH_3$ | $OCH_2CF_3$ | |
| H | O | O | $CH_3$ | H | H | $CH_3$ | H | H | H | H | H | N | $OCH_3$ | $OC_2H_5$ | |
| H | O | O | H | H | H | H | H | $CH_3$ | H | H | H | N | $OCH_3$ | $CH_3$ | |
| H | O | O | $CH_3$ | $CH_3$ | H | H | H | H | H | $CH_3$ | $CH_3$ | CH | $CH_3$ | $C_2H_5$ | |
| H | O | O | H | H | H | H | H | H | $CH_3$ | H | H | CH | $OCH_3$ | $CH_3$ | |

TABLE XXI (continued)

| $R_1$ | W | W' | $R_{27}$ | $R_{28}$ | $R_{17}$ | $R_{18}$ | $R_{19}$ | $R_{20}$ | $R_{21}$ | $R_{22}$ | $R_{23}$ | Z | X | Y | m.p. (°C) |
|---|---|---|---|---|---|---|---|---|---|---|---|---|---|---|---|
| H | O | O | H | H | H | H | H | H | H | $CH_3$ | H | CH | $CH_3$ | $CH_3$ | |
| H | O | O | H | H | H | H | H | H | H | H | H | N | $OCH_3$ | $OCH_2CH_2OCH_3$ | |
| H | O | S | H | H | H | H | H | H | H | H | H | N | $CH_3$ | $OCH_3$ | |
| H | O | S | H | H | H | H | H | H | H | H | H | N | $CH_3$ | $CH(—OCH_2CH_2O—)$ | |
| 5-Cl | O | O | H | H | H | H | H | H | H | H | H | CH | $OCH_3$ | $CH_3$ | |
| 5-$CH_3$ | O | O | H | H | H | H | H | H | H | H | H | CH | Cl | $OCH_3$ | |
| 6-Cl | O | O | H | H | H | H | H | H | H | H | H | CH | $OCH_3$ | $CH_2OCH_3$ | |
| 4-$CH_3$ | O | O | H | H | H | H | H | H | H | H | H | CH | $CH_3$ | $OCH_3$ | |

TABLE XXII

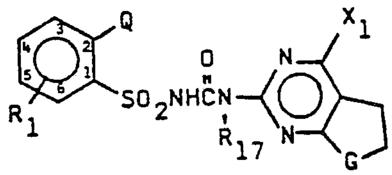

| Q | $R_1$ | $R_{17}$ | $X_1$ | G | m.p. (°C) |
|---|---|---|---|---|---|
| 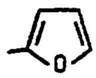 | H | H | $CH_3$ | $CH_2$ | |
| 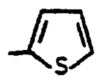 | H | H | $CH_3$ | O | |
| 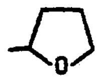 | H | H | $CH_3$ | O | |
| 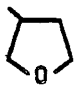 | H | H | $CH_3$ | O | |
| 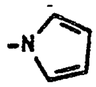 | H | H | $CH_3$ | O | |
| 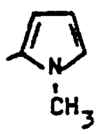 | H | H | $OCH_3$ | O | |
| 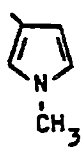 | H | H | $OCH_3$ | $CH_2$ | |
| 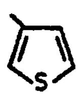 | 5-Cl | H | $CH_3$ | O | |
| 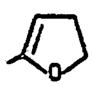 | H | H | $CH_3$ | O | |
| 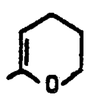 | H | $CH_3$ | $CH_3$ | O | |
| 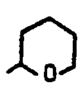 | H | H | Cl | O | |
| 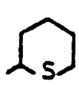 | H | H | $CH_3$ | O | |
| 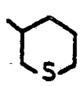 | H | H | $CH_3$ | O | |
| 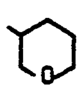 | H | H | $CH_3$ | $CH_2$ | |

TABLE XXII (continued)

| Q | $R_1$ | $R_{17}$ | $X_1$ | G | m.p. (°C) |
|---|---|---|---|---|---|
| 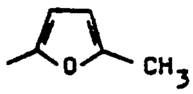 | H | H | $CH_3$ | O | |
| 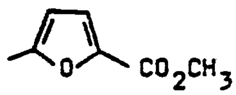 | H | H | $CH_3$ | O | |
| 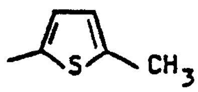 | H | H | $CH_3$ | O | |
| 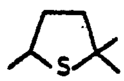 | H | H | $CH_3$ | O | |

TABLE XXIII

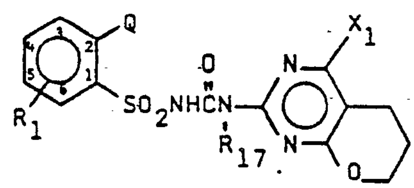

| Q | $R_1$ | $R_{17}$ | $X_1$ | m.p. (°C) |
|---|---|---|---|---|
| 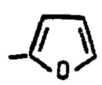 | H | H | $CH_3$ | |
| 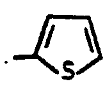 | H | H | $CH_3$ | |
| 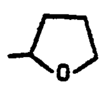 | H | H | $CH_3$ | |
| 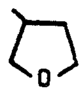 | H | H | $CH_3$ | |
| 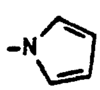 | H | H | $CH_3$ | |
| 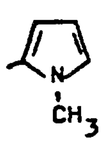 | H | H | $OCH_3$ | |
| 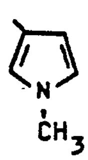 | H | H | $OCH_3$ | |
| 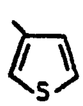 | 5-Cl | H | $CH_3$ | |

TABLE XXIII (continued)

| Q | $R_1$ | $R_{17}$ | $X_1$ | m.p. (°C) |
|---|---|---|---|---|
| 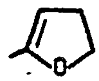 | H | $CH_3$ | $CH_3$ | |
| 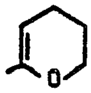 | H | $CH_3$ | $CH_3$ | |
| 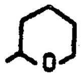 | H | H | Cl | |
| 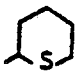 | H | H | $CH_3$ | |
| 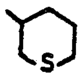 | H | H | $CH_3$ | |
| 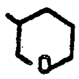 | H | H | $CH_3$ | |
| 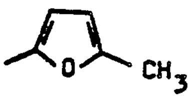 | H | H | $CH_3$ | |
| 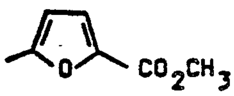 | H | H | $CH_3$ | |
| 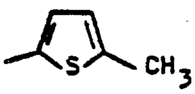 | H | H | $CH_3$ | |
| 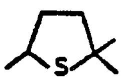 | H | H | $CH_3$ | |

TABLE XXIV

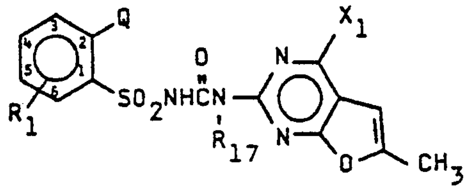

| Q | $R_1$ | $R_{17}$ | $X_1$ | m.p. (°C) |
|---|---|---|---|---|
| 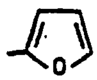 | H | H | $CH_3$ | |
| 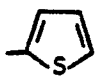 | H | H | $CH_3$ | |
| 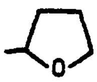 | H | H | $CH_3$ | |
| 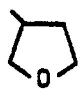 | H | H | $CH_3$ | |
| 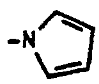 | H | H | $CH_3$ | |
| 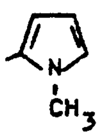 | H | H | $OCH_3$ | |
| 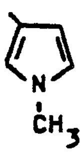 | H | H | $OCH_3$ | |
| 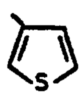 | 5-Cl | H | $CH_3$ | |
| 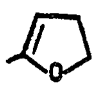 | H | H | $CH_3$ | |
| 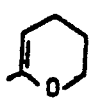 | H | $CH_3$ | $CH_3$ | |
| 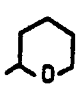 | H | H | Cl | |
| 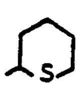 | H | H | $CH_3$ | |
| 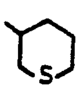 | H | H | $CH_3$ | |
| 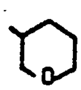 | H | H | $CH_3$ | |

TABLE XXIV (continued)

| Q | $R_1$ | $R_{17}$ | $X_1$ | m.p. (°C) |
|---|---|---|---|---|
| 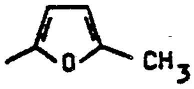 | H | H | $CH_3$ | |
| 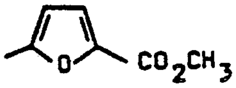 | H | H | $CH_3$ | |
| 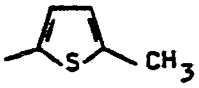 | H | H | $CH_3$ | |
| 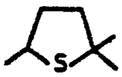 | H | H | $CH_3$ | |

TABLE XXV

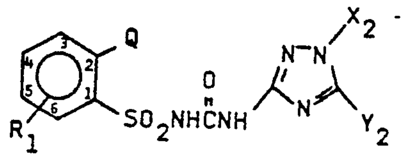

| Q | $R_1$ | $X_2$ | $Y_2$ | m.p. (°C) |
|---|---|---|---|---|
| 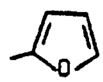 | H | $CH_3$ | $OCH_3$ | |
| 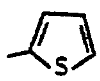 | H | $C_2H_5$ | $OCH_3$ | |
| 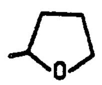 | H | $CH_3$ | $SCH_3$ | |
| 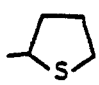 | H | $C_2H_5$ | $SCH_3$ | |
| 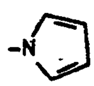 | H | $CH_2CF_3$ | $SC_2H_5$ | |
| 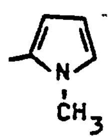 | H | $CH_2CF_3$ | $SC_2H_5$ | |
| 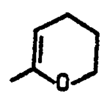 | 5-F | $CH_3$ | $SCH_3$ | |
| 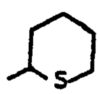 | H | $CH_2CH_2CH_3$ | $OC_2H_5$ | |
| 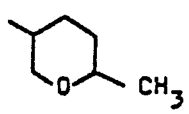 | H | $C_2H_5$ | $OCH_3$ | |

Formulations

Useful formulations of the compounds of Formula I can be prepared in conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of (a) about 0.1% to 20% surfactant(s) and (b) about 1% to 99.9% solid or liquid inert diluent(s). More specifically, they will contain these ingredients in the following approximate proportions:

TABLE XXVI

| | Active Ingredient | Weight Percent* Diluent(s) | Surfactant(s) |
|---|---|---|---|
| Wettable Powders | 20—90 | 0—74 | 1—10 |
| Oil Suspensions, Emulsions, Solutions, (including Emulsifiable Concentrates) | 3—50 | 40—95 | 0—15 |
| Aqueous Suspension | 10—50 | 40—84 | 1—20 |
| Dusts | 1—25 | 70—99 | 0—5 |
| Granules and Pellets | 0.1—95 | 5—99.9 | 0—15 |
| High Strength Compositions | 90—99 | 0—10 | 0—2 |

*Active ingredient plus at least one of a Surfactant or a Diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide", 2nd Ed., Interscience, New York, 1950. Solubility under 0.1% is preferred for suspension concentrates; solution concentrates are preferably stable against phase separation at 0°C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", *Chemical Engineering,* December 4, 1967, pp 147ff. and "Perry's Chemical Engineers' Handbook", 5th Ed., McGraw-Hill, New York, 1973, pp. 8—57ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138—140, 162—164, 166, 167 and 169—182;

H. Gysin and E. Knusli, U.S. Patent 2,891,855, June 23, 1959, Col. 3, line 66 through Col. 5, line 17 and Examples 1—4;

G. C. Klingman, "Weed Control as a Science", John wiley and Sons, Inc., New York, 1961, pp. 81—96; and

J. D. Fryer and S. A. Evans, "Weed Control Handbook", 5th Ed., Blackwell Scientific Publications, Oxford, 1968, pp. 101—103.

In the following examples, all parts are by weight unless otherwise indicated.

Example 8

Wettable Powder

| | |
|---|---|
| 2-(2-Carbomethoxyfur-5-yl)-N-[(4,6-dimethoxypyrimidin-2-yl)aminocarbonyl]benzenesulfonamide | 80% |
| sodium alkylnaphthalenesulfonate | 2% |
| sodium ligninsulfonate | 2% |
| synthetic amorphous silica | 3% |
| kaolinite | 13% |

The ingredients are blended, hammer-milled until all the solids are essentially under 50 microns, reblended, and packaged.

Example 9

Wettable Powder

| | |
|---|---|
| N-[(4-methoxy-6-methylpyrimidin-2-yl)-amino-carbonyl]-2-(tetrahydrofuran-2-yl)benzenesulfonamide | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles essentially all below 10 microns in diameter. The product is reblended before packaging.

Example 10

Granule

| | |
|---|---|
| Wettable Powder of Example 9 | 5% |
| attapulgite granules | 95% |
| (U.S.S. 20—40 mesh; 0.84—0.42 mm) | |

A slurry of wettable powder containing ≈25% solids is sprayed on the surface of attapulgite granules in a double-cone blender. The granules are dried and packaged.

Example 11

Extruded Pellet

| | |
|---|---|
| N-[(4,6-dimethoxypyrimidin-2-yl)aminocarbonyl]-2-(tetrahydrofuran-2-yl)benzenesulfonamide | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

Example 12

Oil Suspension

| | |
|---|---|
| N-[(4-chloro-6-methoxypyrimidin-2-yl)aminocarbonyl]-2-(tetrahydrofuran-2-yl)benzenesulfonamide | 25% |
| polyoxyethylene sorbitol hexaoleate | 5% |
| highly aliphatic hydrocarbon oil | 70% |

The ingredients are ground together in a sand mill until the solid particles have been reduced to under about 5 microns. The resulting thick suspension may be applied directly, but preferably after being extended with oils or emulsified in water.

Example 13

Wettable Powder

| | |
|---|---|
| N-[(4,6-dimethoxy-1,3,5-triazin-2-yl)aminocarbonyl]-2-(tetrahydrofuran-2-yl)benzenesulfonamide | 20% |
| sodium alkylnaphthalenesulfonate | 4% |
| sodium ligninsulfonate | 4% |
| low viscosity methyl cellulose | 3% |
| attapulgite | 69% |

The ingredients are thoroughly blended. After grinding in a hammer-mill to produce particles essentially all below 100 microns, the material is reblended and sifted through a U.S.S. No. 50 sieve (0.3 mm opening) and packaged.

Example 14

Low Strength Granule

| | |
|---|---|
| N-[(4,6-dimethylpyrimidin-2-yl)aminocarbonyl]-2-(tetrahydrofuran-2-yl)benzenesulfonamide | 1% |
| N,N-dimethylformamide | 9% |
| attapulgite granules (U.S.S. 20—40 sieve) | 90% |

The active ingredient is dissolved in the solvent and the solution is sprayed upon dedusted granules in a double cone blender. After spraying of the solution has been completed, the blender is allowed to run for a short period and then the granules are packaged.

Example 15

Aqueous Suspension

| | |
|---|---|
| N-[(4-methoxy-6-methylpyrimidin-2-yl)aminocarbonyl]-2-(tetrahydrofuran-2-yl)benzenesulfonamide | 40% |
| polyacrylic acid thickener | 0.3% |
| dodecylphenol polyethylene glycol ether | 0.5% |
| disodium phosphate | 1% |
| monosodium phosphate | 0.5% |
| polyvinyl alcohol | 1.0% |
| water | 56.7% |

The ingredients are blended and ground together in a sand mill to produce particles essentially all under 5 microns in size.

Example 16

Solution

| | |
|---|---|
| N-[(4-methoxy-6-methyl-1,3,5-triazin-2-yl)aminocarbonyl]-2-(tetrahydrofuran-2-yl)benzenesulfonamide, sodium salt | 5% |
| water | 95% |

The salt is added directly to the water with stirring to produce the solution, which may then be packaged for use.

Example 17

Low Strength Granule

| | |
|---|---|
| N-[(4-chloro-6-methoxypyrimidin-2-yl)aminocarbonyl]-2-(tetrahydrofuran-2-yl)benzenesulfonamide | 0.1% |
| attapulgite granules | 99.9% |
| (U.S.S. 20—40 mesh) | |

The active ingredient is dissolved in a solvent and the solution is sprayed upon dedusted granules in a double-cone blender. After spraying of the solution has been completed, the material is warmed to evaporate the solvent. The material is allowed to cool and then packaged.

Example 18

Granule

| | |
|---|---|
| N-[(4,6-dimethoxypyrimidin-2-yl)aminocarbonyl]-2-(tetrahydrofuran-2-yl)benzenesulfonamide | 80% |
| wetting agent | 1% |
| crude ligninsulfonate salt (containing 5—20% of the natural sugars) | 10% |
| attapulgite clay | 9% |

The ingredients are blended and milled to pass through a 100 mesh screen. This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged, screened to the desired size range, generally 14—100 mesh (1410—149 microns), and packaged for use.

Example 19

High Strength Concentrate

| | |
|---|---|
| 2-(2-Carbomethoxyfur-5-yl)-N-[(4,6-dimethyl-1,3,5-triazin-2-yl)aminocarbonyl]benzenesulfonamide | 99% |
| silica aerogel | 0.5% |
| synthetic amorphous silica | 0.5% |

The ingredients are blended and ground in a hammer-mill to produce a material essentially all passing a U.S.S. No. 50 screen (0.3 mm opening). The concentrate may be formulated further if necessary).

Example 20

Wettable Powder

| | |
|---|---|
| N-[(4,6-dimethoxy-1,3,5-triazin-2-yl)aminocarbonyl]-2-(tetrahydrofuran-2-yl)benzenesulfonamide | 90% |
| dioctyl sodium sulfosuccinate | 0.1% |
| synthetic fine silica | 9.9% |

The ingredients are blended and ground in a hammer-mill to produce particles essentially all below 100 microns. The material is sifted through a U.S.S. No. 50 screen and then packaged.

Example 21

Wettable Powder

| | |
|---|---|
| N-[(4,6-dimethylpyrimidin-2-yl)aminocarbonyl]-2-tetrahydrofuran-2-yl)benzenesulfonamide | 40% |
| sodium ligninsulfonate | 20% |
| montmorillonite clay | 40% |

The ingredients are thoroughly blended, coarsely hammer-milled and then air-milled to produce particles essentially all below 10 microns in size. The material is reblended and then packaged.

Example 22

Oil Suspension

| | |
|---|---|
| N-[(4-methoxy-6-methylpyrimidin-2-yl)aminocarbonyl]-2-(tetrahydrofuran-2-yl)benzenesulfonamide | 35% |
| blend of polyalcohol carboxylic esters and oil soluble petroleum sulfonates | 6% |
| xylene | 59% |

The ingredients are combined and ground together in a sand mill to produce particles essentially all below 5 microns. The product can be used directly, extended with oils, or emulsified in water.

Example 23

Dust

| | |
|---|---|
| N-[(4,6-dimethoxypyrimidin-2-yl)aminocarbonyl]-2-tetrahydrofuran-2-yl)benzenesulfonamide | 10% |
| attapulgite | 10% |
| Pyrophyllite | 80% |

The active ingredient is blended with attapulgite and then passed through a hammer-mill to produce particles substantially all below 200 microns. The ground concentrate is then blended with powdered pyrophyllite until homogeneous.

Utility

The compounds of the present invention are highly active herbicides. They can be used for broad-spectrum pre- and/or post-emergence weed control in areas where complete control of all vegetation is desired, such as around fuel storage tanks, ammunition depots, industrial storage areas, parking lots, drive-in theaters, around billboards, highway and railroad structures. Alternatively, the subject compounds are useful for plant growth modification, particularly in retarding the growth of undesired vegetation.

Rates of application for the compounds of this invention are ordinarily determined by a number of factors, including their use as either herbicides or plant growth modifiers, the types of weeds to be controlled, weather and climate, the formulation to be used, the mode of application, amount of foliage present, etc. In general terms, the subject compounds should be applied at levels of around 0.001 to 5 kg/ha, lower rates being preferred for lighter soils and/or those having a low organic matter content, for

situations where only short-term persistence is required, or for plant growth modification.

The compounds of the invention may be used in combination with any other commercial herbicide, examples of which are those of the triazine, triazole, uracil, urea, amide, diphenylether, carbamate and bipyridylium types.

The herbicidal and plant growth modifying properties of the subject compounds were discovered in a number of greenhouse tests, the results of which may be seen in the following examples.

## Compounds

Compound 1

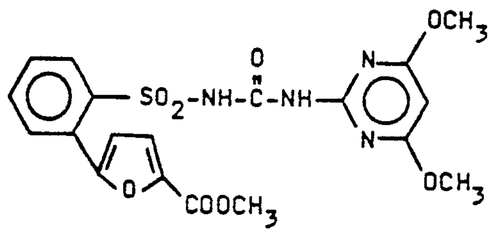

Compound 2

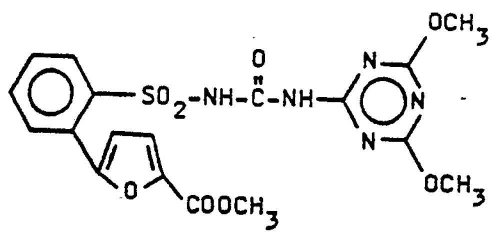

Compound 3

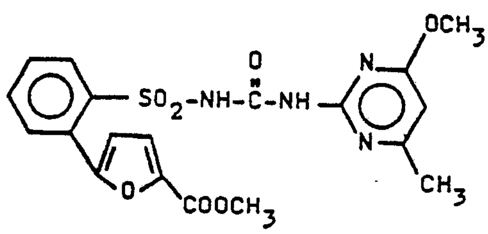

Compound 4

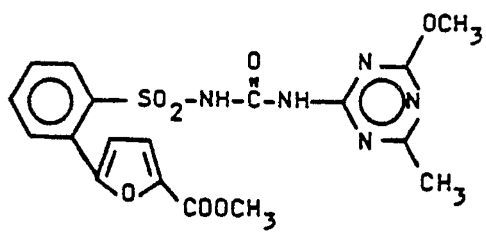

Compound 5

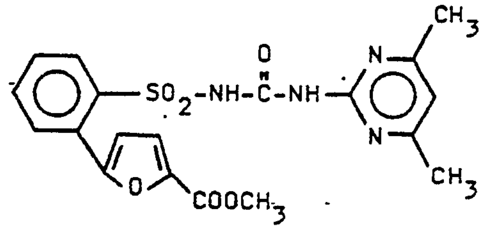

Compounds (continued)

Compound 6

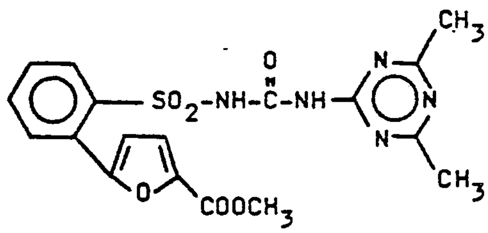

Compound 7

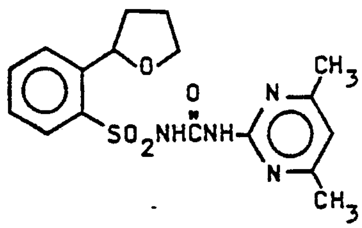

Compound 8

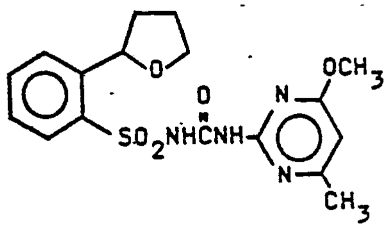

Compound 9

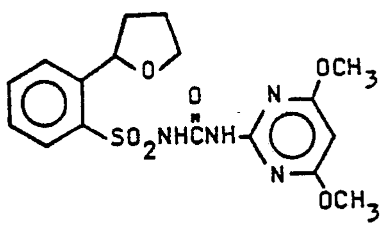

Compound 10

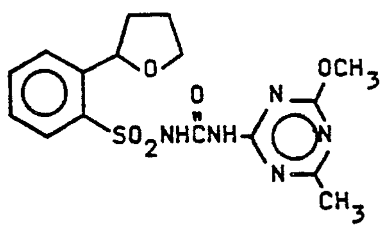

Compound 11

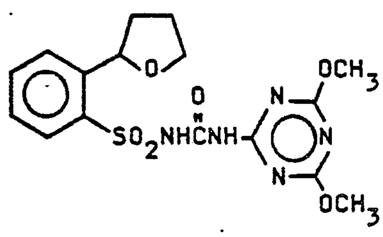

Compounds (continued)

Compound 12

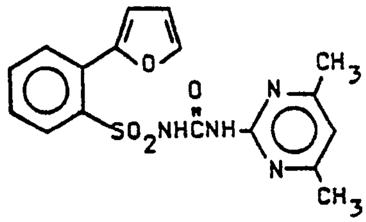

Compound 13

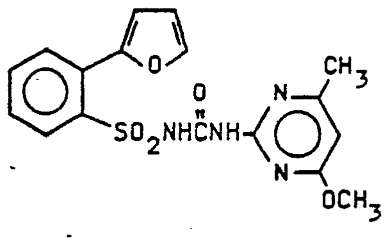

Compound 14

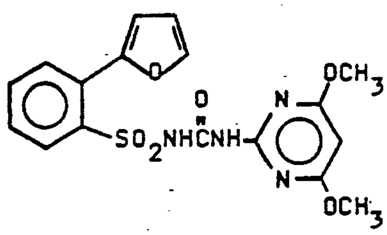

Compound 15

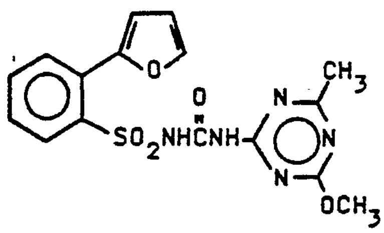

Compound 16

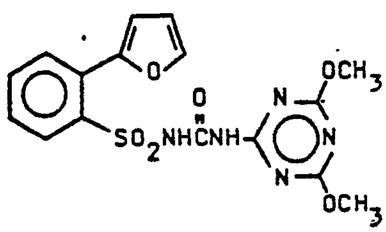

Compound 17

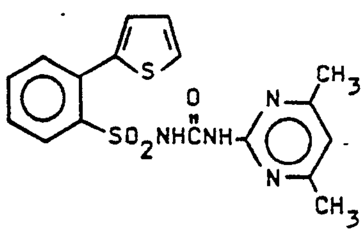

Compounds (continued)

Compound 18

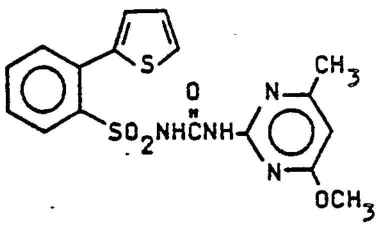

Compound 19

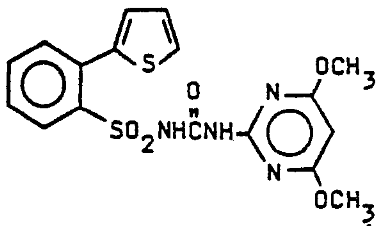

Compound 20

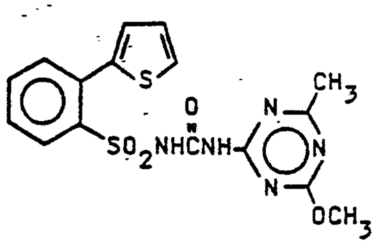

Compound 21

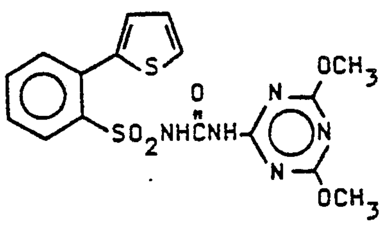

Compound 22

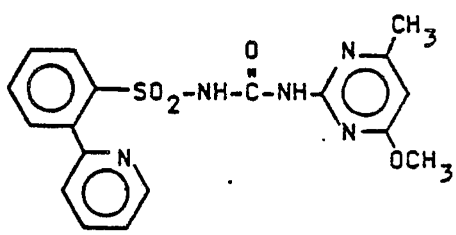

Compound 23

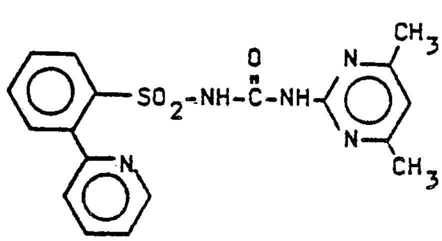

Compounds (continued)

Compound 24

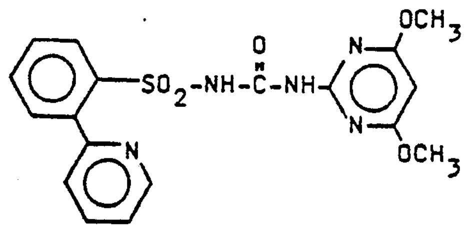

Compound 25

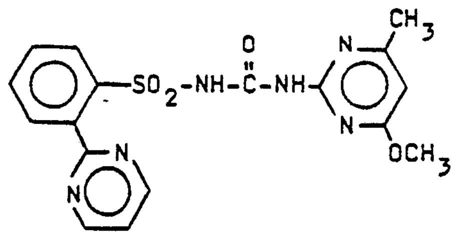

*Test A*

Seeds of crabgrass (*Digitaria* spp.), barnyardgrass (*Echinochloa crusgalli*), wild oats (*Avena fatua*), sicklepod (*Cassia obtusifolia*), morningglory (*Ipomoea* spp.), cocklebur (*Xanthium* spp.), sorghum, corn, soybean, rice, wheat and nutsedge tubers (*Cyperus rotundus*) were planted in a growth medium and treated pre-emergence with a nonphytotoxic solvent solution of the compounds of Table A. At the same time, cotton having five leaves (including cotyledonary ones), bush beans with the third trifoliolate leaf expanding, crabgrass with two leaves, barnyardgrass with two leaves, wild oats with two leaves, sicklepod with three leaves (including cotyledonary ones), morningglory with four leaves (including the cotyledonary ones), cocklebur with four leaves (including the cotyledonary ones), sorghum with four leaves, corn with four leaves, soybean with two cotyledonary leaves, rice with three leaves, wheat with one leaf, and nutsedge with three to five leaves were sprayed with a nonphytotoxic solvent solution of the compounds of Table A. Other containers of the above mentioned weeds and crops were treated pre- or post-emergence with the same nonphytotoxic solvent so as to provide a solvent control. A set of untreated control plants was also included for comparison. Pre-emergence and post-emergence treated plants and controls were maintained in a greenhouse for sixteen days, then all treated plants were compared with their respective controls and rated visually for response to treatment. Several of the compounds tested were essentially innocuous to wheat but, at the same rate of application, provided effective weed control.

The following rating system was used:

0 = no effect;
10 = maximum effect;
C = chlorosis or necrosis;
E = emergence inhibition;
G = growth retardation;
H = formative effects;
S = albinism;
U = increased chlorophyl;
X = axillary stimulation; and
6Y = abscised buds or flowers.

The test data are summarized in Table A.

TABLE A

| | Cmpd. 1 | Cmpd. 2 | Cmpd. 3 | Cmpd. 4 |
|---|---|---|---|---|
| Rate kg/ha | 2 | 2 | 2 | 2 |
| POST-EMERGENCE | | | | |
| Bush bean | 1H, 5G | 4S, 8G, 6Y | 6C, 9G, 6Y | 6C, 9G, 6Y |
| Cotton | 5C, 9G | 3C, 3H, 8G | 5C, 9G | 5C, 9G |
| Morningglory | 1C, 4G | 1C | 3C, 9G | 4C, 9G |
| Cocklebur | 3C, 7H | 2C, 7H | 5C, 9G | 9C |
| Sicklepod | 2C | 2C | 4C | 4C |
| Nutsedge | 2C, 9G | 2C, 5G, 5X | 9G | 2C, 9G |
| Crabgrass | 6G | 2C | 2C, 9G | 1C, 2G |
| Barnyardgrass | 9H | 1C | 3C, 9H | 2C, 5H |
| Wild Oats | O | O | 2C, 8G, 5X | O |
| Wheat | O | O | 3C, 9G | 2G |
| Corn | O | 1C, 3G | 2U, 9G | 2C, 9H |
| Soybean | 1C, 3G | 1C, 1H | 4C, 8G | 4C, 8G |
| Rice | 2C, 9G | O | 5C, 9G | 5G |
| Sorghum | 2C, 7G | 1C, 4G | 1C, 9G | 3C, 8H |
| PRE-EMERGENCE | | | | |
| Morningglory | 1C | 2C | 8G | 8G |
| Cocklebur | 8H | 9H | — | 9H |
| Sicklepod | 1C | 2C | 9C | 2C, 7H |
| Nutsedge | 9G | 10E | 10E | 10E |
| Crabgrass | 2C | 3C | 2C, 6G | 2C |
| Barnyardgrass | 5G | 3C | 3C, 8H | 5C |
| Wild Oats | 2G | 2G | 3C, 9G | 1C, 6G |
| Wheat | 2G | 2G | 3C, 9G | 4G |
| Corn | 2C, 6G | 2C, 8G | 9G | 2U, 9G |
| Soybean | O | 1C, 1H | 5H | 7H |
| Rice | 3C, 7H | 2C, 3G | 10E | 2C, 8G |
| Sorghum | 2C, 7G | 2C, 7G | 5C, 9H | 2C, 9H |

TABLE A (continued)

| | Cmpd. 5 | Cmpd. 6 | Cmpd. 7 | Cmpd. 8 |
|---|---|---|---|---|
| Rate kg/ha | 2 | 2 | 0.05 | 0.05 |
| POST-EMERGENCE | | | | |
| Bush bean | 5C, 9G, 6Y | 1C, 2G | 5C, 9G, 6Y | 6C, 9G, 6Y |
| Cotton | 6C, 9G | 2C, 3H | 4C, 9G | 5C, 9G |
| Morningglory | 2C, 6G | 1C | 10C | 10C |
| Cocklebur | 5C, 9G | 3C, 6H | 10C | 10C |
| Sicklepod | 1C, 2G | O | 4C, 6G | 4C, 8G |
| Nutsedge | 1C, 9G | 2G | 2C, 8G | 2C, 9G |
| Crabgrass | 2C, 8G | 1C, 3G | 2C, 6G | 2C, 9G |
| Barnyardgrass | 2C, 8H | O | 5C, 9H | 5C, 9H |
| Wild Oats | 2C, 6G | O | 4C, 9G | 5C, 9G |
| Wheat | 3C, 9G | O | 3U, 9G | 4C, 9G |
| Corn | 2C, 9G | 3C, 7G | 5U, 9G | 5U, 9G |
| Soybean | 2C, 4H | 2C, 5G | 6C, 9G | 5C, 9G |
| Rice | 3C, 9G | 2C, 7G | 5C, 9G | 5C, 9G |
| Sorghum | 2C, 9G | 2C, 9G | 3C, 9G | 5C, 9H |
| Sugar beet | — | — | 3C, 9H | 9C |
| PRE-EMERGENCE | | | | |
| Morningglory | 8G | 1C, 3G | 5C, 9G | 9G |
| Cocklebur | 9H | 9H | 9H | 9H |
| Sicklepod | 1C, 5G | 2C | 9G | 9G |
| Nutsedge | 10E | 9G | 9G | 10E |
| Crabgrass | 3C | O | 2G | 3C, 6G |
| Barnyardgrass | 3C, 6H | 3C | 2C, 9H | 5C, 9H |
| Wild Oats | 2C, 9H | 2C, 8G | 2C, 9G | 5C, 9G |
| Wheat | 4C, 9H | 5G | 2C, 9G | 4C, 9G |
| Corn | 10E | 2C, 8G | 9G | 4C, 9G |
| Soybean | 8H | 1C | 2C, 5H | 9H |
| Rice | 10E | 9H | 10E | 10E |
| Sorghum | 10E | 3C, 9G | 5C, 9H | 5C, 9H |
| Sugar beet | | | 9G | 10E |

TABLE A (continued)

| | Cmpd. 9 | Cmpd. 10 | Cmpd. 11 | Cmpd. 12 |
|---|---|---|---|---|
| Rate kg/ha | 0.05 | 0.05 | 0.05 | 0.05 |
| POST-EMERGENCE | | | | |
| Bush bean | 5C, 9G, 6Y | 6C, 9G, 6Y | 5C, 9G, 6Y | 5C, 9G, 6Y |
| Cotton | 6C, 9G | 4C, 8G | 5C, 9G | O |
| Sorghum | 4C, 9G | 2C, 9G | 9G | 2C, 9G |
| Corn | 6U, 9G | 2U, 9G | 1U, 9H | 2C, 9G |
| Soybean | 10C | 9C | 4C, 8G | 5C, 9H |
| Wheat | 3C, 9G | 2C, 9G | 1C | 7G |
| Wild Oats | 4C, 9G | 2C, 9G | 1C, 5G | 2C, 9G |
| Rice | 6C, 9G | 9C | 9C | 6C, 9G |
| Barnyardgrass | 5C, 9H | 5C, 9H | 2C, 9H | 5C, 9H |
| Crabgrass | 4C, 9G | 2C, 8G | 1C, 3G | 2C, 5G |
| Morningglory | 4C, 7H | 5C, 8H | 10C | 5G |
| Cocklebur | 10C | 10C | 10C | 2C, 8H |
| Sicklepod | 5C, 9G | 3C, 8H | 3C, 5H | 2C, 5G |
| Nutsedge | 5C, 9G | 3C, 8G | 3G | 2C, 7G |
| Sugar beet | 9C | 9C | 9C | 2C, 7H |
| PRE-EMERGENCE | | | | |
| Sorghum | 5C, 9H | 5C, 9H | 5C, 9H | 3C, 9H |
| Corn | 2C, 9H | 2C, 9G | 2C, 9H | 3C, 9G |
| Soybean | 9H | 9H | 2C, 5H | 5H |
| Wheat | 1C, 9G | 2C, 9G | O | 2C, 9G |
| Wild Oats | 3C, 9G | 4C, 9G | 5H | 3C, 7G |
| Rice | 10E | 10E | 10E | 2C, 9H |
| Barnyardgrass | 5C, 9H | 5C, 9H | 2C, 7H | 5C, 9H |
| Crabgrass | 2C, 9G | 2C, 8G | O | 2C |
| Morningglory | 9G | 9C | 9C | 8G |
| Cocklebur | — | — | 9H | 9H |
| Sicklepod | 9G | 9G | 3C, 9G | 9G |
| Nutsedge | 10E | 5C, 9G | O | 2C, 9G |
| Sugar beet | 10E | 10E | 10E | 9G |

TABLE A (continued)

| | Cmpd. 13 | Cmpd. 14 | Cmpd. 15 | Cmpd. 16 |
|---|---|---|---|---|
| Rate kg/ha | 0.05 | 0.05 | 0.05 | 0.05 |
| POST-EMERGENCE | | | | |
| Bush bean | 6C, 9G, 6Y | 5C, 9G, 6Y | 5C, 9G, 6Y | 5C, 9G, 6Y |
| Cotton | 2C, 3G | 2C, 5G | 3C, 6G | 2C, 5G |
| Sorghum | 2C, 9G | 9G | 1C, 9G | O |
| Corn | 2C, 9G | 2C, 9G | 2C, 9G | O |
| Soybean | 5C, 9G | 9C | 9C | 9C |
| Wheat | 2C, 7G | O | 1C, 6G | O |
| Wild Oats | 2C, 7G | O | 1C, 6G | O |
| Rice | 5C, 9G | 5C, 9G | 6C, 9G | O |
| Barnyardgrass | 5C, 8H | 5C, 9H | 2C, 9H | 1H |
| Crabgrass | 2C, 4G | 1C, 6G | 2C, 6G | O |
| Morningglory | 3C, 5G | 2C, 7G | 2C, 8G | 5C, 9G |
| Cocklebur | 3C, 9H | 3C, 8H | 3C, 8H | 4G |
| Sicklepod | 3C, 6G | 3C, 9H | 2C, 6G | 2C, 8H |
| Nutsedge | 5G | 9G | 1C, 8G | 5G |
| Sugar beet | 10C | 5C, 9G | 2C, 9H | 4C, 9G |
| PRE-EMERGENCE | | | | |
| Sorghum | 5C, 9G | 5C, 9H | 9H | 2G |
| Corn | 3C, 9H | 2C, 9G | 9G | 1C, 6G |
| Soybean | 8H | 8H | 9H | 8H |
| Wheat | 2C, 9G | 1C, 6G | 2C, 9G | O |
| Wild Oats | 3C, 8G | 1C, 3G | 3C, 8G | 2C |
| Rice | 10E | 9H | 9H | 2G |
| Barnyardgrass | 5C, 9H | 5C, 9H | 5C, 9H | 2C |
| Crabgrass | 2C, 4G | 2C, 6G | 4C, 8G | O |
| Morningglory | 9G | 7G | 8G | 9G |
| Cocklebur | 9H | 9H | 9H | 9H |
| Sicklepod | 8G | 9G | 9G | 8G |
| Nutsedge | 10E | 10E | 10E | 5G |
| Sugar beet | 10E | 10E | 10E | 10E |

TABLE A (continued)

| | Cmpd. 17 | Cmpd. 18 | Cmpd. 19 | Cmpd. 20 | Cmpd. 21 |
|---|---|---|---|---|---|
| Rate kg/ha | 0.05 | 0.05 | 0.05 | 0.05 | 0.05 |
| POST-EMERGENCE | | | | | |
| Bush bean | 5C, 8G, 6Y | 5S, 9G, 6Y | 5C, 9G, 6Y | 9C | 9C |
| Cotton | 3C, 5G | 4C, 4H, 8G | 6C, 9G | 6C, 9G | 3C, 9G |
| Sorghum | 2C, 8H | 3C, 9H | 9H | 2G | 2C |
| Corn | 2C, 6H | 3C, 8H | 2C, 9G | 2G | O |
| Soybean | 4C, 9G | 3C, 9G | 3C, 9G | 5C, 9G | 5C, 9G |
| Wheat | 1H | 1C | 3G | O | O |
| Wild Oats | 1H | 2C | 3G | O | O |
| Rice | 2C, 8G | 2C, 8G | 2C, 7G | O | O |
| Barnyardgrass | 2C, 6H | 5C, 9H | 9C | 2H | O |
| Crabgrass | 1C | 5G | 2C, 4G | O | 2C |
| Morningglory | 5C, 9G | 3C, 8G | 5C, 9H | 10C | 9C |
| Cocklebur | 2C, 5G | 2C, 8H | 5C, 9G | 9C | 10C |
| Sicklepod | 3C | 2C, 4G | 5C, 8H | 4C, 5G | 3C, 4H |
| Nutsedge | 2C, 6G | 3C, 7G | 2C, 8G | 5G | O |
| Sugar beet | — | — | — | — | — |
| PRE-EMERGENCE | | | | | |
| Sorghum | 4G | 3C, 9H | 4C, 9H | 2G | O |
| Corn | 3C, 6G | 2C, 8H | 9G | 1C, 4G | O |
| Soybean | 1C | 3C, 8H | 9H | 9H | O |
| Wheat | 5G | 8G | 7G | O | O |
| Wild Oats | 2C, 4G | 2C, 8G | 2C, 8G | O | O |
| Rice | O | 3C, 6G | 10E | 1C, 4G | O |
| Barnyardgrass | 3G | 3C, 9H | 3C, 9H | 2G | O |
| Crabgrass | 3G | 2C, 5G | 3C, 8G | 2G | 2G |
| Morningglory | 5G | 8H | 9G | 9G | 8G |
| Cocklebur | 8H | 9H | 9H | 9H | 9H |
| Sicklepod | 8G | 9G | 9G | 9G | 9G |
| Nutsedge | 2G | 8G | 10E | 3G | O |
| Sugar beet | — | — | — | — | — |

TABLE A (continued)

| | Cmpd. 22 | Cmpd. 23 | Cmpd. 24 | Cmpd. 25 |
|---|---|---|---|---|
| Rate kg/ha | 0.05 | 0.05 | 0.05 | 0.05 |
| POST-EMERGENCE | | | | |
| Bush bean | 4C, 9G, 6Y | 4H, 6Y | 6C, 9G, 6Y | 9C |
| Cotton | 4C, 9G | 5C, 7H | 5C, 9G | 4C, 9G |
| Morningglory | 9C | 4C, 8H | 5C, 9G | 10C |
| Cocklebur | 9C | 4C, 8H | 4C, 9G | 10C |
| Sicklepod | 5C, 7H | 2C | 3C, 9G | 9C |
| Nutsedge | 2C, 9G | 3C, 8G | 2C, 9G | 6C, 9G |
| Crabgrass | 3C, 8G | 2C, 4H | 2C, 8H | 6C, 9G |
| Barnyardgrass | 3C, 9H | 3C, 8H | 5C, 9H | 9C |
| Wild Oats | 3C, 9G | 3C, 8H | 2C, 9G | 9C |
| Wheat | 2C, 9G | 2C, 3H | 2C, 7G | 9C |
| Corn | 3C, 9G | 4C, 9H | 3C, 9H | 10C |
| Soybean | 5C, 9H | 3C, 9G | 5C, 9G | 9C |
| Rice | 5C, 9G | 6C, 9G | 5C, 9G | 6C, 9G |
| Sorghum | 3C, 9H | 5C, 9H | 2C, 9H | 6C, 9G |
| Sugar beet | 4C, 8G | 2C, 4G | 2C, 9G | 9C |
| PRE-EMERGENCE | | | | |
| Morningglory | 9C | 2C, 6H | 9C | 3C, 9H |
| Cocklebur | 9H | 5H, 2C | 9H | — |
| Sicklepod | 9C | 3H | 2C, 9G | 2C, 9G |
| Nutsedge | 3G | O | 10E | 10E |
| Crabgrass | 1C, 2H | 4G | 2C, 5G | 3C, 7G |
| Barnyardgrass | 5C, 9H | 2C, 4G | 4C, 9H | 3C, 9H |
| Wild Oats | 5C, 9G | 2C, 5G | 2C, 9G | 3C, 9H |
| Wheat | 3C, 9G | 2C, 3G | 2C, 9G | 3C, 9H |
| Corn | 3C, 9G | 3C, 5G | 3C, 9H | 10E |
| Soybean | 4C, 7H | 2C | 3C, 9H | 9H |
| Rice | 5C, 9H | 3C, 5G | 10E | 10E |
| Sorghum | 5C, 9H | 3C, 7H | 5C, 9H | 5C, 9H |
| Sugar beet | 5C, 9G | 3C, 5H | 9G | 10E |

**Claims for the Contracting States: BE CH DE FR IT LI LU NL SE**

1. A compound of the formula:

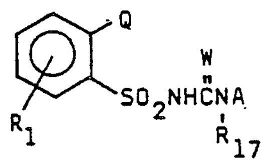

I

or

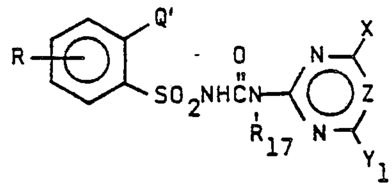

Ia

wherein

Q is

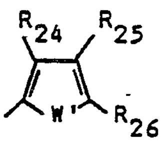

,

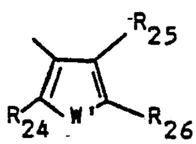

,

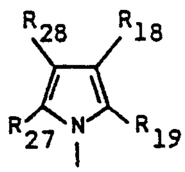

,

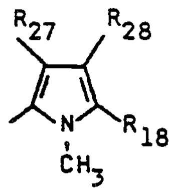

,

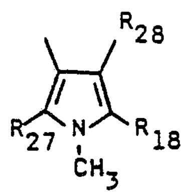

,

Q-1 Q-2 Q-3 Q-4 Q-5

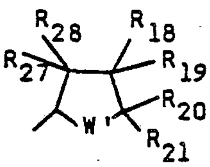

,

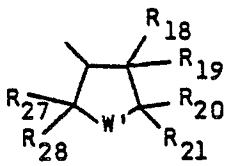

,

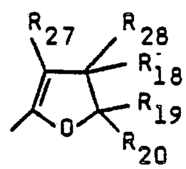

,

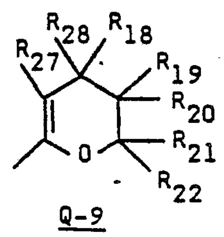

,

Q-6 Q-7 Q-8 Q-9

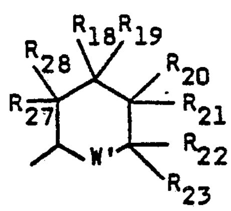

or

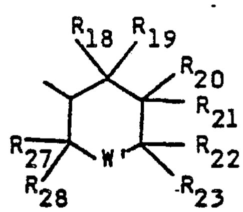

;

Q-10 Q-11

where Q' is

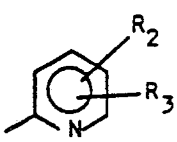

,

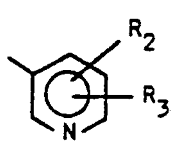

,

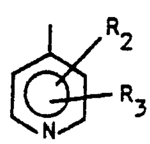

.

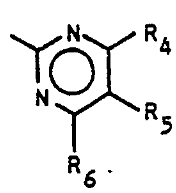

,

Q'-1 Q'-2 Q'-3 Q'-4

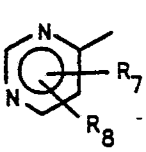

,

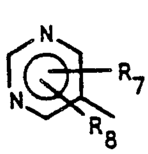

,

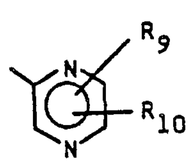

,

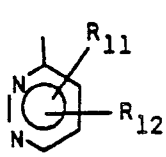

,

Q'-5 Q'-6 Q'-7 Q'-8

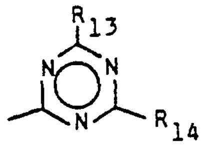 or 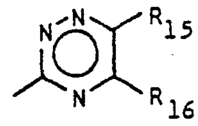 ;

Q'-9 Q'-10

W is O or S;
W' is O or S;
R is H, F, Cl, $CH_3$ or $OCH_3$;
$R_1$ is H, F, Cl, Br, $CH_3$, $CF_3$ or $OCH_3$;
$R_2$, $R_3$, $R_5$, $R_9$, $R_{10}$, $R_{11}$, $R_{12}$ and $R_{17}$ are independently H or $CH_3$;
$R_4$, $R_6$, $R_7$ and $R_8$ are independently H, $CH_3$ or $OCH_3$;
$R_{13}$, $R_{14}$, $R_{15}$ and $R_{16}$ are independently $CH_3$ or $OCH_3$;
$R_{18}$ is H or $CH_3$;
$R_{19}$ is H or $CH_3$;
$R_{20}$ is H or $CH_3$;
$R_{21}$ is H or $CH_3$;
$R_{22}$ is H or $CH_3$;
$R_{23}$ is H or $CH_3$;
$R_{24}$ is H, $CH_3$, $C_2H_5$, Cl, Br, $OCH_3$ or $OC_2H_5$;
$R_{25}$ is H, $CH_3$, $C_2H_5$, Cl, Br, $OCH_3$ or $OC_2H_5$;
$R_{26}$ is H, $CH_3$, $C_2H_5$, Cl, Br, $OCH_3$, $OC_2H_5$ or $CO_2CH_3$;
$R_{27}$ is H or $CH_3$;
$R_{28}$ is H or $CH_3$;
A is

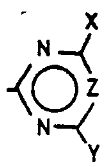 , 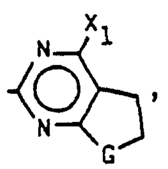 , 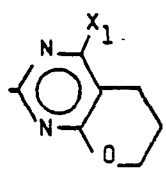 , 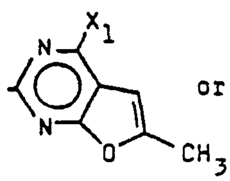 or 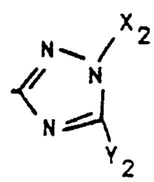 ;

X is $CH_3$, $OCH_3$ or Cl;
Y is $CH_3$, $C_2H_5$, $CH_2OCH_3$, $OCH_3$, $OC_2H_5$, $CH(OCH_3)_2$, $SCH_3$, $CF_3$, $NH_2$, $NHCH_3$, $N(CH_3)_2$, $OCH_2CH_2OCH_3$, $OCH_2CF_3$ or

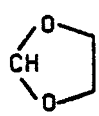 ;

$Y_1$ is $CH_3$, $C_2H_5$, $CH_2OCH_3$, $OCH_3$, $OC_2H_5$, $CH(OCH_3)_2$, $NH_2$, $NHCH_3$ or $N(CH_3)_2$;
Z is CH or N;
$X_1$ is $CH_3$, $OCH_3$ or Cl;
G is O or $CH_2$;
$X_2$ is $C_1$—$C_3$ alkyl or $CH_2CF_3$;
$Y_2$ is $CH_3O$, $C_2H_5O$, $CH_3S$ or $C_2H_5S$; and their agriculturally suitable salts; provided that
1) when W is S, then $R_{17}$ is H,
A is

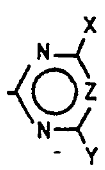 ,

Y is $CH_3$, $OCH_3$, $C_2H_5$, $CH_2OCH_3$, $CH(OCH_3)_2$ or

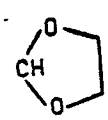 ;

2) when Q is

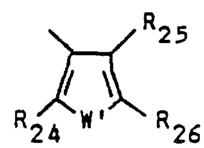

,

then $R_{24}$ is H, $CH_3$ or $C_2H_5$; $R_{25}$ is H, $CH_3$ or $C_2H_5$; and $R_{26}$ is H, $CH_3$, $C_2H_5$ or $—CO_2CH_3$;

3) when one of $R_{24}$, $R_{25}$, $R_{26}$ is other than H, then the other two substituents are H or $CH_3$;

4) the total number of carbon atoms in $R_{18}$, $R_{19}$, $R_{20}$, $R_{21}$, $R_{22}$, $R_{23}$, $R_{27}$ and $R_{28}$ is less than or equal to 4;

5) when X is Cl, then Z is CH and Y or $Y_1$ is $OCH_3$, $NH_2$, $OC_2H_5$, $NHCH_3$ or $N(CH_3)_2$; and

6) when R is $CH_3$, then R is in the 3-, 4- or 5-position of the benzene ring, relative to the sulfonamido group in the 1-position.

2. Compounds of Claim 1, Formula I, where W is O.

3. Compounds of Claim 2 where $R_1$ and $R_{17}$ are H.

4. Compounds of Claim 3 where $R_{24}$ is H, $CH_3$ or $C_2H_5$; $R_{25}$ is H, $CH_3$ or $C_2H_5$; and $R_{26}$ is H, $CH_3$, $C_2H_5$ or $—CO_2CH_3$.

5. Compounds of Claim 4 where Y is $CH_3$, $CH_2OCH_3$, $OCH_3$, $OC_2H_5$, $CH(OCH_3)_2$ or

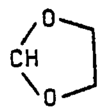

,

and

A is

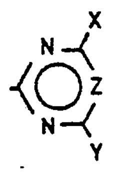

;

6. Compounds of Claim 1, Formula Ia, where R and $R_{17}$ are H.

7. Compounds of Claim 6 where $Y_1$ is $CH_3$, $OCH_3$, $CH_2OCH_3$ or $N(CH_3)_2$.

8. Compounds of Claim 4 where W′ = O.

9. Compounds of Claim 4 where W′ = S.

10. Compounds of Claim 7 where Q′ is a substituted pyridine.

11. Compounds of Claim 7 where Q′ is a substituted pyrimidine.

12. Compounds of Claim 7 where Q′ is a substituted pyrazine.

13. Compounds of Claim 7 where Q′ is a substituted triazine.

14. A compound of Claim 1 selected from N - [(4,6 - dimethylpyrimidin - 2 - yl)aminocarbonyl] - 2 - (tetrahydrofuran - 2 - yl)benzenesulfonamide; N - [(4 - methoxy - 6 - methylpyrimidin - 2 - yl)aminocarbonyl] - 2 - (tetrahydrofuran - 2 - yl)benzenesulfonamide; N - [(4,6 - dimethoxypyrimidin - 2 - yl)aminocarbonyl] - 2 - (tetrahydrofuran - 2 - yl)benzenesulfonamide; N - [(4 - chloro - 6 - methoxypyrimidin - 2 - yl)aminocarbonyl] - 2 - (tetrahydrofuran - 2 - yl)benzenesulfonamide; N - [(4,6 - dimethoxy - 1,3,5 - triazin - 2 - yl)aminocarbonyl] - 2 - (tetrahydrofuran - 2 - yl)benzenesulfonamide; N - [(4 - methoxy - 6 - methyl - 1,3,5 - triazin - 2 - yl)aminocarbonyl] - 2 - (tetrahydrofuran - 2 - yl)benzenesulfonamide; or an agriculturally suitable salt thereof.

15. A compound of Claim 1 selected from N - [(4 - methoxy - 6 - methylpyrimidin - 2 - yl)aminocarbonyl] - 2 - (2 - pyridinyl)benzenesulfonamide; N - [(4,6 - dimethylpyrimidin - 2 - yl)aminocarbonyl] - 2 - (2 - pyridinyl)benzenesulfonamide; N - [(4,6 - dimethoxy - pyrimidin - 2 - yl)aminocarbonyl] - 2 - (2 - pyridinyl)benzenesulfonamide; or an agriculturally suitable salt thereof.

16. A compound of Claim 1 which is N - [(4 - methoxy - 6 - methylpyrimidin - 2 - yl)aminocarbonyl] - 2 - (2 - pyrimidinyl)benzenesulfonamide; or an agriculturally suitable salt thereof.

17. A composition suitable for controlling the growth of undesired vegetation comprising an effective amount of a herbicidal compound and at least one of the following: surfactant, solid or liquid diluent, characterised in that said herbicidal compound comprises a compound of any of claims 1 to 16.

18. A method for controlling the growth of undesired vegetation by applying to the locus to be protected an effective amount of a herbicidal compound characterised in that said herbicidal compound comprises a compound of any of claims 1 to 16.

19. A method for regulating the growth of plants by applying to the locus of such plants an effective but substantially non-phytotoxic amount of a plant growth regulant characterised in that said plant growth regulant comprises a compound of any of claims 1 to 16.

20. A method for the preparation of a compound of claim 1 which comprises

(a) reacting a benzenesulfonamide of formula

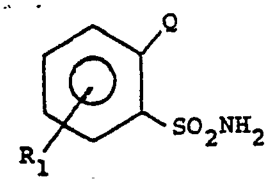

(II)

with a methyl heteroaryl carbamate of formula

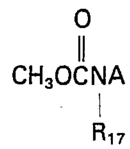

(III)

in the presence of an equimolar amount of trimethylaluminum to obtain a compound of formula (I) wherein $R_1$, $R_{17}$, Q and A are as defined in claim 1 and W is O; or

(b) reacting a benzenesulfonyl isocyanate or isothiocyanate of formula

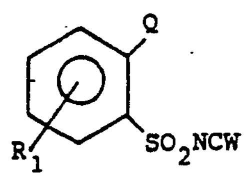

(IV)

with an aminoheterocycle of formula

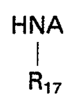

(V)

wherein $R_1$, $R_{17}$, W and A are as defined in claim 1 and Q is selected from Q—1, Q—2 or Q—6 to Q—11; or

(c) reacting said sulfonamide of formula (II) with a heterocyclic isothiocyanate of formula

SCN—A (Va)

wherein A is as defined in claim 1; or

(d) reacting a sulfonamide of formula

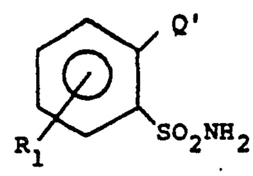

with a methyl pyrimidinyl carbamate or methyl triazinyl carbamate of formula

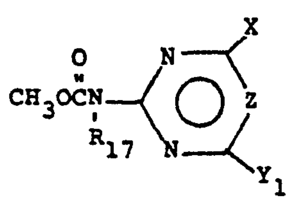

in the presence of an equimolar amount of trimethylaluminum wherein $R_1$, $R_{17}$, Q', X, Y and Z are as defined in claim 1, to obtain a product of formula (Ia).

**Claims for the Contracting State: AT**

1. A process for the preparation of a compound of the formula:

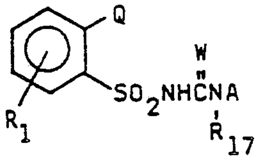

I

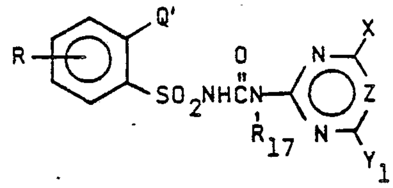

Ia

wherein

Q is

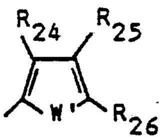
,
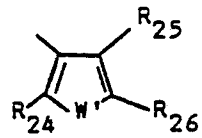
,
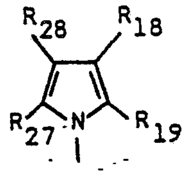
,
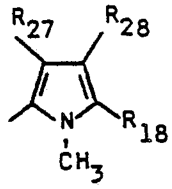
,
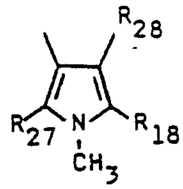
,

Q-1 Q-2 Q-3 Q-4 Q-5

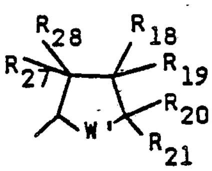
,
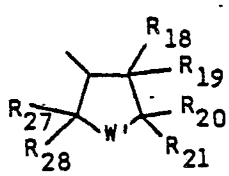
,
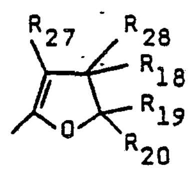
,
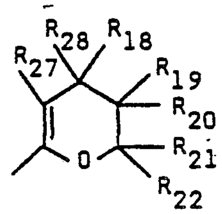
,

Q-6 Q-7 Q-8 Q-9

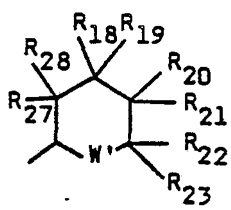
or
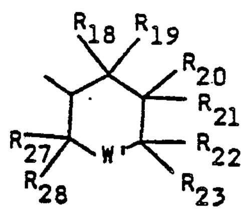
;

Q-10 Q-11

where Q' is

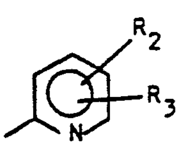
,
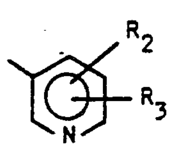
,
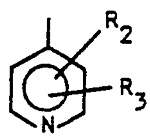
,
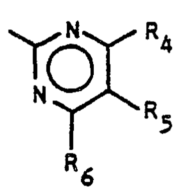
,
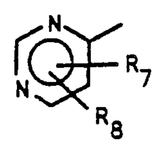
,

Q'-1 Q'-2 Q'-3 Q'-4 Q'-5

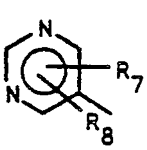
,
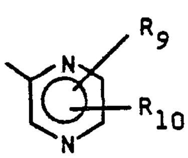
,
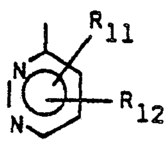
,
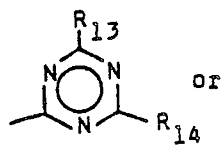
or
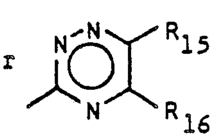
;

Q'-6 Q'-7 Q'-8 Q'-9 Q'-10

W is O or S;
W' is O or S;
R is H, F, Cl, $CH_3$ or $OCH_3$;
$R_1$ is H, F, Cl, Br, $CH_3$, $CF_3$ or $OCH_3$;
$R_2$, $R_3$, $R_5$, $R_9$, $R_{10}$, $R_{11}$, $R_{12}$ and $R_{17}$ are independently H or $CH_3$;
$R_4$, $R_6$, $R_7$ and $R_8$ are independently H, $CH_3$ or $OCH_3$;
$R_{13}$, $R_{14}$, $R_{15}$ and $R_{16}$ are independently $CH_3$ or $OCH_3$;
$R_{18}$ is H or $CH_3$;
$R_{19}$ is H or $CH_3$;
$R_{20}$ is H or $CH_3$;
$R_{21}$ is H or $CH_3$;
$R_{22}$ is H or $CH_3$;
$R_{23}$ is H or $CH_3$;
$R_{24}$ is H, $CH_3$, $C_2H_5$, Cl, Br, $OCH_3$ or $OC_2H_5$;
$R_{25}$ is H, $CH_3$, $C_2H_5$, Cl, Br, $OCH_3$ or $OC_2H_5$;
$R_{26}$ is H, $CH_3$, $C_2H_5$, Cl, Br, $OCH_3$, $OC_2H_5$ or $CO_2CH_3$;
$R_{27}$ is H or $CH_3$;
$R_{28}$ is H or $CH_3$;
A is

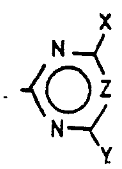 , 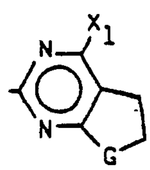 , 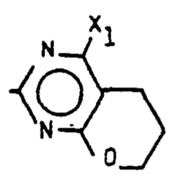 , 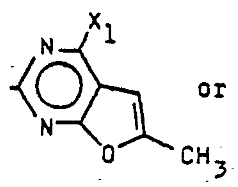 or 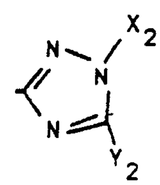 ;

X is $CH_3$, $OCH_3$ or Cl;
Y is $CH_3$, $C_2H_5$, $CH_2OCH_3$, $OCH_3$, $OC_2H_5$, $CH(OCH_3)_2$, $SCH_3$, $CF_3$, $NH_2$, $NHCH_3$, $N(CH_3)_2$, $OCH_2CH_2OCH_3$, $OCH_2CF_3$ or

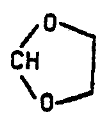 ;

$Y_1$ is $CH_3$, $C_2H_5$, $CH_2OCH_3$, $OCH_3$, $OC_2H_5$, $CH(OCH_3)_2$, $NH_2$, $NHCH_3$ or $N(CH_3)_2$;
Z is CH or N;
$X_1$ is $CH_3$, $OCH_3$ or Cl;
G is O or $CH_2$;
$X_2$ is $C_1$—$C_3$ alkyl or $CH_2CF_3$;
$Y_2$ is $CH_3O$, $C_2H_5O$, $CH_3S$ or $C_2H_5S$; and their agriculturally suitable salts; provided that
1) when W is S, then $R_{17}$ is H,
A is

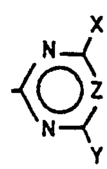 ,

Y is $CH_3$, $OCH_3$, $C_2H_5$, $CH_2OCH_3$, $CH(OCH_3)_2$ or

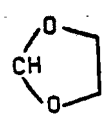 ;

2) when Q is

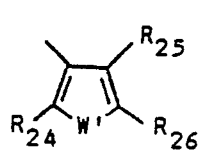 ,

then $R_{24}$ is H, $CH_3$ or $C_2H_5$; $R_{25}$ is H, $CH_3$ or $C_2H_5$; and $R_{26}$ is H, $CH_3$, $C_2H_5$ or —$CO_2CH_3$;

3) when one of $R_{24}$, $R_{25}$, $R_{26}$ is other than H, then the other two substituents are H or $CH_3$;

4) the total number of carbon atoms in $R_{18}$, $R_{19}$, $R_{20}$, $R_{21}$, $R_{22}$, $R_{23}$, $R_{27}$ and $R_{28}$ is less than or equal to 4;

5) when X is Cl, then Z is CH and Y or $Y_1$ is $OCH_3$, $NH_2$, $OC_2H_5$, $NHCH_3$ or $N(CH_3)_2$; and

6) when R is $CH_3$, then R is in the 3-, 4- or 5-position of the benzene ring, relative to the sulfonamido group in the 1-position; which comprises

(a) reacting a benzenesulfonamide of formula

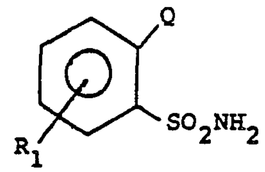

(II)

with a methyl heteroaryl carbamate of formula

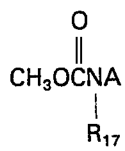

(III)

in the presence of an equimolar amount of trimethylaluminum to obtain a compound of formula (I) wherein $R_1$, $R_{17}$, Q and A are as defined above and W is O; or

(b) reacting a benzenesulfonyl isocyanate or isothiocyanate of formula

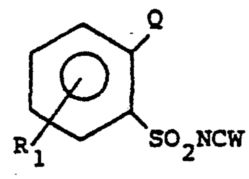

(IV)

with an aminoheterocycle of formula

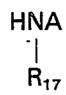

(V)

wherein $R_1$, $R_{17}$, W and A are as defined above and Q is selected from Q—1, Q—2 or Q—6 to Q—11; or

(c) reacting said sulfonamide of formula (II) with a heterocyclic isothiocyanate of formula

SCN—A (Va)

wherein A is as defined above; or

(d) reacting a sulfonamide of formula

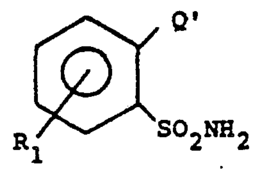

with a methyl pyrimidinyl carbamate or methyl triazinyl carbamate of formula

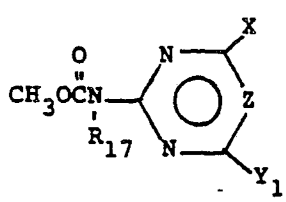

in the presence of an equimolar amount of trimethylaluminum wherein $R_1$, $R_{17}$, Q′, X, Y and Z are as defined above, to obtain a product of formula (Ia).

2. A process of Claim 1, where the product is of Formula I, where W is O.

3. A process of Claim 2 where $R_1$ and $R_{17}$ are H.

4. A process of Claim 3 where $R_{24}$ is H, $CH_3$ or $C_2H_5$; $R_{25}$ is H, $CH_3$ or $C_2H_5$; and $R_{26}$ is H, $CH_3$, $C_2H_5$ or $-CO_2CH_3$.

5. A process of Claim 4 where Y is $CH_3$, $CH_2OCH_3$, $OCH_3$, $OC_2H_5$, $CH(OCH_3)_2$ or

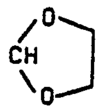 ;

and

A is

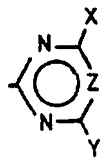 ,

6. A process of Claim 1, where the product is of Formula Ia, where R and $R_{17}$ are H.

7. A process of Claim 6 where $Y_1$ is $CH_3$, $OCH_3$, $CH_2OCH_3$ or $N(CH_3)_2$.

8. A process of Claim 4 where W' = O.

9. A process of Claim 4 where W' = S.

10. A process of Claim 7 where Q' is a substituted pyridine.

11. A process of Claim 7 where Q' is a substituted pyrimidine.

12. A process of Claim 7 where Q' is a substituted pyrazine.

13. A process of Claim 7 where Q' is a substituted triazine.

14. A process of Claim 1 wherein the product is a compound selected from N - [(4,6 - dimethylpyrimidin - 2 - yl)aminocarbonyl] - 2 - (tetrahydrofuran - 2 - yl)benzenesulfonamide; N - [(4 - methoxy - 6 - methylpyrimidin - 2 - yl)aminocarbonyl] - 2 - (tetrahydrofuran - 2 - yl)benzenesulfonamide; N - [(4,6 - dimethoxypyrimidin - 2 - yl)aminocarbonyl] - 2 - (tetrahydrofuran - 2 - yl)benzenesulfonamide; N - [(4 - chloro - 6 - methoxypyrimidin - 2 - yl)aminocarbonyl] - 2 - (tetrahydrofuran - 2 - yl)benzenesulfonamide; N - [(4,6 - dimethoxy - 1,3,5 - triazin - 2 - yl)aminocarbonyl] - 2 - (tetrahydrofuran - 2 - yl)benzenesulfonamide; N - [(4 - methoxy - 6 - methyl - 1,3,5 - triazin - 2 - yl)aminocarbonyl] - 2 - (tetrahydrofuran - 2 - yl)benzenesulfonamide; or an agriculturally suitable salt thereof.

15. A process of Claim 1 wherein the product is a compound selected from N - [(4 - methoxy - 6 - methylpyrimidin - 2 - yl)aminocarbonyl) - 2 - (2 - pyridinyl)benzenesulfonamide; N - [(4,6 - dimethylpyrimidin - 2 - yl)aminocarbonyl] - 2 - (2 - pyridinyl)benzenesulfonamide; N - [(4,6 - dimethoxy - pyrimidin - 2 - yl)aminocarbonyl] - 2 - (2 - pyridinyl)benzenesulfonamide; or an agriculturally suitable salt thereof.

16. A process of Claim 1 wherein the product is N - [(4 - methoxy - 6 - methylpyrimidin - 2 - yl)aminocarbonyl] - 2 - (2 - pyrimidinyl)benzenesulfonamide; or an agriculturally suitable salt thereof.

17. A composition suitable for controlling the growth of undesired vegetation comprising an effective amount of a herbicidal compound and at least one of the following: surfactant, solid or liquid diluent, characterised in that said herbicidal compound comprises a compound of Formula (I) or (Ia) as defined in any of claims 1 to 16.

18. A method for controlling the growth of undesired vegetation by applying to the locus to be protected an effective amount of a herbicidal compound characterised in that said herbicidal compound comprises a compound of Formula (I) or (Ia) as defined in any of claims 1 to 16.

19. A method for regulating the growth of plants by applying to the locus of such plants an effective but substantially non-phytotoxic amount of a plant growth regulant characterised in that said plant growth regulant comprises a compound of Formula (I) or (Ia) as defined in any of claims 1 to 16.

**Patentansprüche für die Vertragsstaaten: BE CH DE FR IT LI LU NL SE**

1. Verbindung der Formel

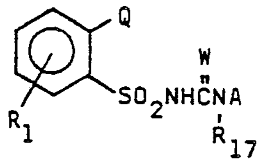 oder 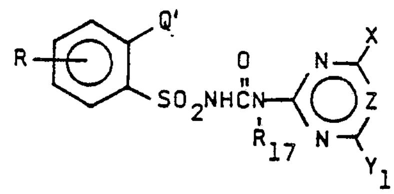

I    Ia

worin

Q

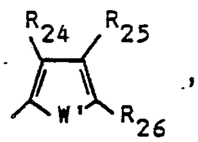,

Q-1

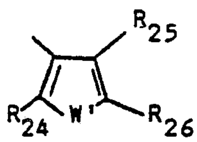

Q-2

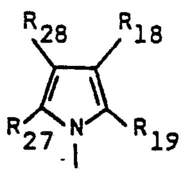,

Q-3

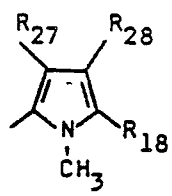,

Q-4

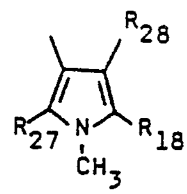,

Q-5

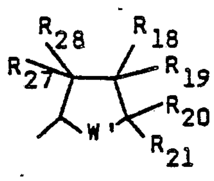,

Q-6

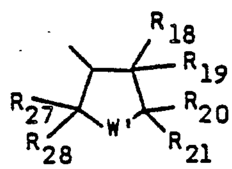,

Q-7

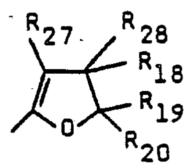,

Q-8

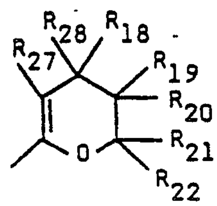,

Q-9

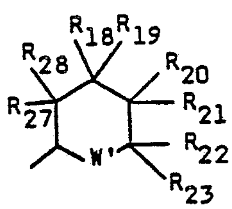 oder 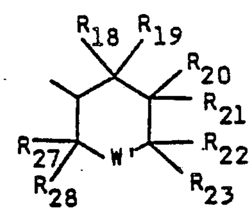 ist;

Q-10    Q-11

wobei Q'

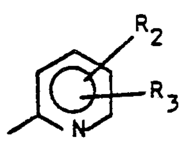,

Q'-1

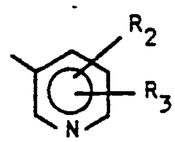,

Q'-2

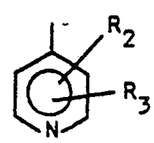,

Q'-3

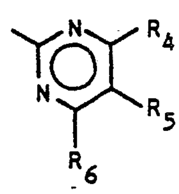,

Q'-4

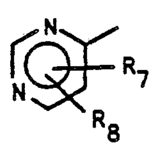,

Q'-5

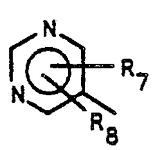,

Q'-6

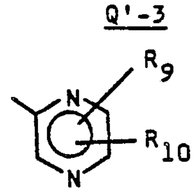,

Q'-7

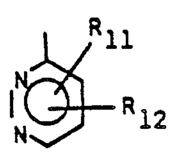,

Q'-8

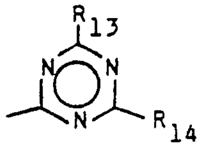 oder 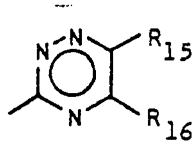 ist;

Q'-9 Q'-10

W O oder S ist;
W' O oder S ist;
R H, F, Cl, $CH_3$ oder $OCH_3$ ist;
$R_1$ H, F, Cl, Br, $CH_3$, $CF_3$ oder $OCH_3$ ist;
$R_2$, $R_3$, $R_5$, $R_9$, $R_{10}$, $R_{11}$, $R_{12}$ und $R_{17}$ unabhängig voneinander H oder $CH_3$ sind;
$R_4$, $R_6$, $R_7$ und $R_8$ unabhängig voneinander H, $CH_3$ oder $OCH_3$ sind;
$R_{13}$, $R_{14}$, $R_{15}$ und $R_{16}$ unabhängig voneinander $CH_3$ oder $OCH_3$ sind;
$R_{18}$ H oder $CH_3$ ist;
$R_{19}$ H oder $CH_3$ ist;
$R_{20}$ H oder $CH_3$ ist;
$R_{21}$ H oder $CH_3$ ist;
$R_{22}$ H oder $CH_3$ ist;
$R_{23}$ H oder $CH_3$ ist;
$R_{24}$ H, $CH_3$, $C_2H_5$, Cl, Br, $OCH_3$ oder $OC_2H_5$ ist;
$R_{25}$ H, $CH_3$, $C_2H_5$, Cl, Br, $OCH_3$ oder $OC_2H_5$ ist;
$R_{26}$ H, $CH_3$, $C_2H_5$, Cl, Br, $OCH_3$, $OC_2H_5$ oder $CO_2CH_3$ ist;
$R_{27}$ H oder $CH_3$ ist;
$R_{28}$ H oder $CH_3$ ist;
A ist

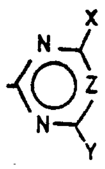, 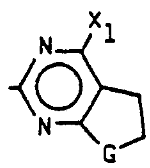, 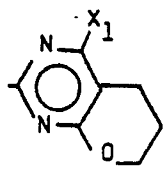, 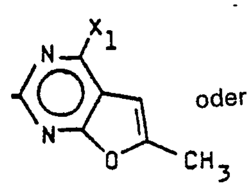 oder 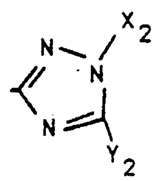;

X $CH_3$, $OCH_3$ oder Cl ist;
Y $CH_3$ $C_2H_5$, $CH_2OCH_3$, $OCH_3$, $OC_2H_5$, $CH(OCH_3)_2$, $SCH_3$, $CF_3$, $NH_2$, $NHCH_3$, $N(CH_3)_2$, $OCH_2CH_2OCH_3$, $OCH_2CF_3$ oder

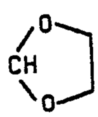

ist;
$Y_1$ $CH_3$, $C_2H_5$, $CH_2OCH_3$, $OCH_3$, $OC_2H_5$, $CH(OCH_3)_2$, $NH_2$, $NHCH_3$ oder $N(CH_3)_2$ ist;
Z CH oder N ist;
$X_1$ $CH_3$, $OCH_3$ oder Cl ist;
G O oder $CH_2$ ist;
$X_2$ $C_{1-3}$-Alkyl oder $CH_2CF_3$ ist;
$Y_2$ $CH_3O$, $C_2H_5O$, $CH_3S$ oder $C_2H_5S$ ist;
und ihre landwirtschaftlich geeigneten Salze; mit der Massgabe, dass
1) wenn W S ist, dann
$R_{17}$ H ist;
A

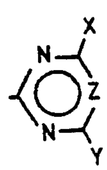

ist;
Y $CH_3$, $OCH_3$, $C_2H_5$, $CH_2OCH_3$, $CH(OCH_3)_2$ oder

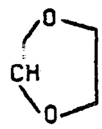

ist;

2) wenn Q

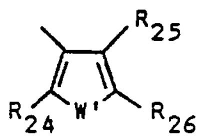

,

ist, dann

$R_{24}$ H, $CH_3$ oder $C_2H_5$ ist;

$R_{25}$ H, $CH_3$ oder $C_2H_5$ ist; und

$R_{26}$ H, $CH_3$, $C_2H_5$ oder $—CO_2CH_3$ ist;

3) wenn eines aus $R_{24}$, $R_{25}$, $R_{26}$ verschieden von H ist, dann die anderen beiden Substituenten H oder $CH_3$ sind;

4) die Gesamtzahl der Kohlenstoffatome in $R_{18}$, $R_{19}$, $R_{20}$, $R_{21}$, $R_{22}$, $R_{23}$, $R_{27}$ und $R_{28}$ weniger oder gleich 4 ist;

5) wenn X Cl ist, dann

Z CH ist und

Y oder $Y_1$ $OCH_3$, $NH_2$, $OC_2H_5$, $NHCH_3$ oder $N(CH_3)_2$ ist; und

6) wenn R $CH_3$ ist, dann

R in der 3-, 4- oder 5-Stellung des Benzolrings, relativ zur Sulfonamidgruppe in der 1-Stellung, angeordnet ist.

2. Verbindungen nach Anspruch 1, Formel I, worin W O ist.

3. Verbindungen nach Anspruch 2, worin $R_1$ und $R_{17}$ H sind.

4. Verbindungen nach Anspruch 3, worin

$R_{24}$ H, $CH_3$ oder $C_2H_5$ ist;

$R_{25}$ H, $CH_3$ oder $C_2H_5$ ist;

$R_{26}$ H, $CH_3$, $C_2H_5$ oder $—CO_2CH_3$ ist.

5. Verbindungen nach Anspruch 4, worin Y $CH_3$, $CH_2OCH_3$, $OCH_3$, $OC_2H_5$, $CH(OCH_3)_2$ oder

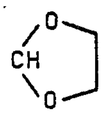

ist und

A

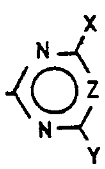

ist.

6. Verbindungen nach Anspruch 1, Formel Ia, worin R und $R_{17}$ H sind.

7. Verbindungen nach Anspruch 6, worin $Y_1$ $CH_3$, $OCH_3$, $CH_2OCH_3$ oder $N(CH_3)_2$ ist.

8. Verbindungen nach Anspruch 4, worin W' = O.

9. Verbindungen nach Anspruch 4, worin W' = S.

10. Verbindungen nach Anspruch 7, worin Q' ein substituiertes Pyridin ist.

11. Verbindungen nach Anspruch 7, worin Q' ein substituiertes Pyrimidin ist.

12. Verbindungen nach Anspruch 7, worin Q' ein substituiertes Pyrazin ist.

13. Verbindungen nach Anspruch 7, worin Q' ein substituiertes Triazin ist.

14. Verbindung nach Anspruch 1, ausgewählt aus N - [(4,6 - Dimethylpyrimidin - 2 - yl)aminocarbonyl] - 2 - (tetrahydrofuran - 2 - yl)benzolsulfonamid; N - [(4 - Methoxy - 6 - methylpyrimidin - 2 - yl)aminocarbonyl] - 2 - (tetrahydrofuran - 2 - yl)benzolsulfonamid; N - [(4,6 - Dimethoxypyrimidin - 2 - yl)aminocarbonyl] - 2 - (tetrahydrofuran - 2 - yl)benzolsulfonamid; N - [(4 - Chlor - 6 - methoxypyrimidin - 2 - yl)aminocarbonyl] - 2 - (tetrahydrofuran - 2 - yl)benzolsulfonamid; N - [(4,6 - Dimethoxy - 1,3,5 - triazin - 2 - yl)aminocarbonyl] - 2 - (tetrahydrofuran - 2 - yl)benzolsulfonamid; N - [(4 - Methoxy - 6 - methyl - 1,3,5 - triazin - 2 - yl)aminocarbonyl] - 2 - (tetrahydrofuran - 2 - yl)benzolsulfonamid; oder ein landwirtschaftlich geeignetes Salz davon.

15. Verbindung nach Anspruch 1, ausgewählt aus N - [(4 - Methoxy - 6 - methylpyrimidin - 2 - yl)aminocarbonyl] - 2 - (2 - pyridinyl)benzolsulfonamid; N - [(4,6 - Dimethylpyrimidin - 2 -

yl)aminocarbonyl] - 2 - (2 - pyridinyl)benzolsulfonamid; N - [(4,6 - Dimethoxypyrimidin - 2 - yl)aminocarbonyl] - 2 - (2 - pyridinyl)benzolsulfonamid; oder ein landwirtschaftlich geeignetes Salz davon.

16. Verbindung nach Anspruch 1, welche N - [(4 - Methoxy - 6 - methylpyrimidin - 2 - yl)aminocarbonyl] - 2 - (2 - pyrimidinyl)benzolsulfonamid; oder ein landwirtschaftlich geeignetes Salz davon ist.

17. Zur Bekämpfung des Wachstum unerwünschter Vegetation geeignete Zusammensetzung, enthaltend eine wirksame Menge einer herbiziden Verbindung und wenigstens eines der folgenden: oberflächenaktives Mittel, festes oder flüssiges Verdünnungsmittel, dadurch gekennzeichnet, dass die herbizide Verbindung eine Verbindung nach einem der Ansprüche 1 bis 16 umfasst.

18. Verfahren zur Bekämpfung des Wachstums unerwünschter Vegetation durch Anwendung einer wirksamen Menge einer herbiziden Verbindung auf den zu schützenden Ort, dadurch gekennzeichnet, dass die herbizide Verbindung eine Verbindung nach einem der Ansprüche 1 bis 16 umfasst.

19. Verfahren zur Regluierung des Wachstums von Pflanzen durch Anwendung einer wirksamen, jedoch im wesentlichen nicht phytotoxischen Menge eines Pflanzenwachstumsregulans auf den Ort solcher Pflanzen, dadurch gekennzeichnet, dass das Pflanzenwachstumsregulans eine Verbindung nach einem der Ansprüche 1 bis 16 umfasst.

20. Verfahren zur Herstellung einer Verbindung nach Anspruch 1 durch

(a) Reagieren eines benzolsulfonamids der Formel

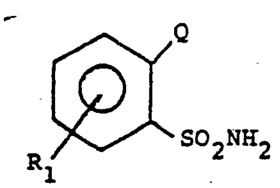

(II)

mit einem Methylheteroarylcarbamat der Formel

O
||
$CH_3OCNA$
|
$R_{17}$

(III)

in Gegenwart einer äquimolaren Menge Trimethylaluminium unter Erhalt einer Verbindung der Formel (I), worin $R_1$, $R_{17}$, Q und A wie in Anspruch 1 definiert sind und W O ist; oder

(b) Reagieren eines Benzolsulfonylisocyanats oder Isothiocyanats der Formel

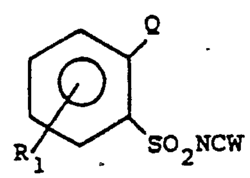

(IV)

mit einem Aminoheterocyclus der Formel

HNA
|
$R_{17}$

(V)

worin $R_1$, $R_{17}$, W und A wie in Anspruch 1 definiert sind und Q ausgewählt ist Q—1, Q—2 oder Q—6 bis Q—11; oder

(c) Reagieren dieses Sulfonamids der Formel (II) mit einem heterocyclischen Isothiocyanat der Formel

SCN—A (Va)

worin A wie in Anspruch 1 definiert ist; oder

(d) Reagieren eines Sulfonamids der Formel

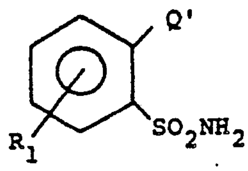

mit einem Methylpyrimidinylcarbamat oder Methyltriazinylcarbamat der Formel

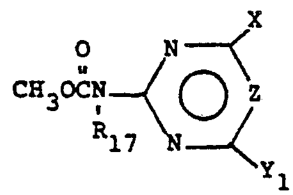

in Gegenwart einer äquimolaren Menge Trimethylaluminium, worin $R_1$, $R_{17}$, Q', X, Y und Z wie in Anspruch 1 definiert sind, unter Erhalt eines Produkts der Formel (Ia).

**Patentansprüche für den Vertragsstaat: AT**

1. Verfahren zur Herstellung einer Verbindung der Formel

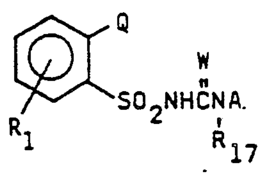

oder

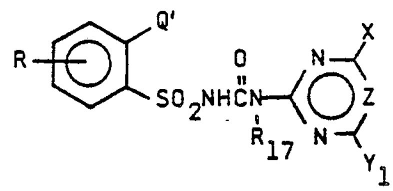

I

Ia

worin

Q

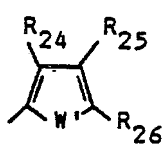

,

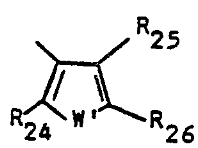

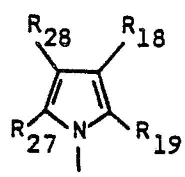

,

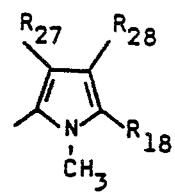

,

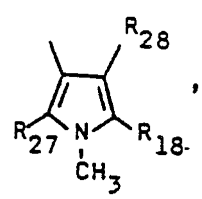

,

Q-1 Q-2 Q-3 Q-4 Q-5

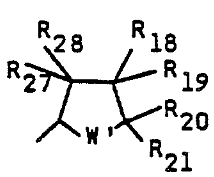

,

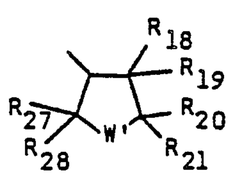

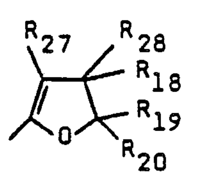

,

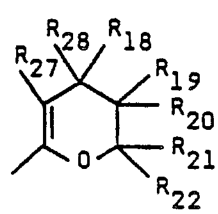

,

Q-6 Q-7 Q-8 Q-9

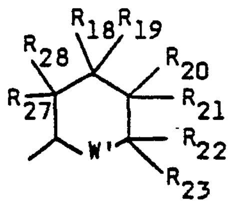 oder 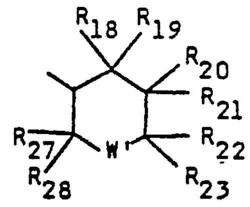 ist;

Q-10 Q-11

wobei Q'

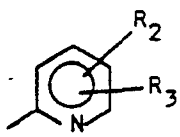 , 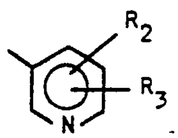 , 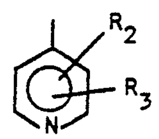 , 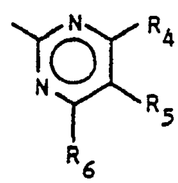 ,

Q'-1 Q'-2 Q'-3 Q'-4

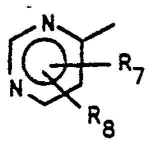 , 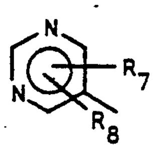 , 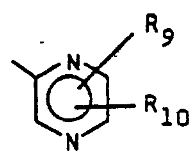 , 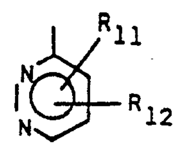 ,

Q'-5 Q'-6 Q'-7 Q'-8

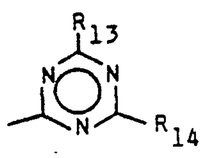 oder 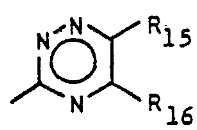 ;

Q'-9 Q'-10

W O oder S ist;
W' O oder S ist;
R H, F, Cl, $CH_3$ oder $OCH_3$ ist;
$R_1$ H, F, Cl, Br, $CH_3$, $CF_3$ oder $OCH_3$ ist;
$R_2$, $R_3$, $R_5$, $R_9$, $R_{10}$, $R_{11}$, $R_{12}$ und $R_{17}$ unabhängig voneinander H oder $CH_3$ sind;
$R_4$, $R_6$, $R_7$ und $R_8$ unabhängig voneinander H, $CH_3$ oder $OCH_3$ sind;
$R_{13}$, $R_{14}$, $R_{15}$ und $R_{16}$ unabhängig voneinander $CH_3$ oder $OCH_3$ sind;
$R_{18}$ H oder $CH_3$ ist;
$R_{19}$ H oder $CH_3$ ist;
$R_{20}$ H oder $CH_3$ ist;
$R_{21}$ H oder $CH_3$ ist;
$R_{22}$ H oder $CH_3$ ist;
$R_{23}$ H oder $CH_3$ ist;
$R_{24}$ H, $CH_3$, $C_2H_5$, Cl, Br, $OCH_3$ oder $OC_2H_5$ ist;
$R_{25}$ H, $CH_3$, $C_2H_5$, Cl. Br, $OCH_3$ oder $OC_2H_5$ ist;
$R_{26}$ H, $CH_3$, $C_2H_5$, Cl, Br, $OCH_3$, $OC_2H_5$ oder $CO_2CH_3$ ist;
$R_{27}$ H oder $CH_3$ ist;
$R_{28}$ H oder $CH_3$ ist;
A

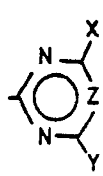 , 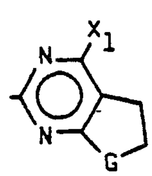 , 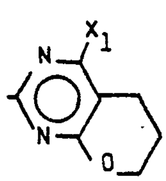 , 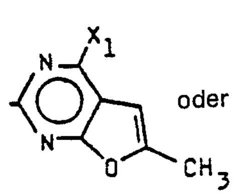 oder 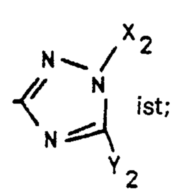 ist;

X $CH_3$, $OCH_3$ oder Cl ist;

Y $CH_3$ $C_2H_5$, $CH_2OCH_3$, $OCH_3$, $OC_2H_5$, $CH(OCH_3)_2$, $SCH_3$, $CF_3$, $NH_2$, $NHCH_3$, $N(CH_3)_2$, $OCH_2CH_2OCH_3$, $OCH_2CF_3$ oder

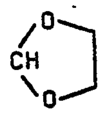

ist;

$Y_1$ $CH_3$, $C_2H_5$, $CH_2OCH_3$, $OCH_3$, $OC_2H_5$, $CH(OCH_3)_2$, $NH_2$, $NHCH_3$ oder $N(CH_3)_2$ ist;

Z CH oder N ist;

$X_1$ $CH_3$, $OCH_3$ oder Cl ist;

G O oder $CH_2$ ist;

$X_2$ $C_{1-3}$-Alkyl oder $CH_2CF_3$ ist;

$Y_2$ $CH_3O$, $C_2H_5O$, $CH_3S$ oder $C_2H_5S$ ist;

und ihrer landwirtschaftlich geeigneten Salze; mit der Massgabe, dass

1) wenn W S ist, dann

$R_{17}$ H ist;

A

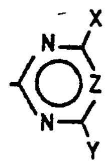

ist;

Y $CH_3$, $OCH_3$, $C_2H_5$, $CH_2OCH_3$, $CH(OCH_3)_2$ oder

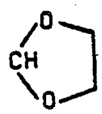

ist;

2) wenn Q

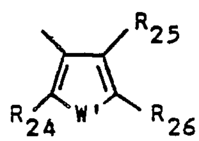

ist, dann

$R_{24}$ H, $CH_3$ oder $C_2H_5$ ist;

$R_{25}$ H, $CH_3$ oder $C_2H_5$ ist; und

$R_{26}$ H, $CH_3$, $C_2H_5$ oder $-CO_2CH_3$ ist;

3) wenn eines aus $R_{24}$, $R_{25}$, $R_{26}$ verschieden von H ist, dann die anderen beiden Substituenten H oder $CH_3$ sind;

4) die Gesamtzahl der Kohlenstoffatome in $R_{18}$, $R_{19}$, $R_{20}$, $R_{21}$, $R_{22}$, $R_{23}$, $R_{27}$ und $R_{28}$ weniger oder gleich 4 ist;

5) wenn X Cl ist, dann

Z CH ist und

Y oder $Y_1$ $OCH_3$, $NH_2$, $OC_2H_5$, $NHCH_3$ oder $N(CH_3)_2$ ist; und

6) wenn R $CH_3$ ist, dann

R in der 3-, 4- oder 5-Stellung des Benzolrings, relativ zur Sulfonamidgruppe in der 1-Stellung, angeordnet ist; durch

(a) Reagieren eines benzolsulfonamids der Formel

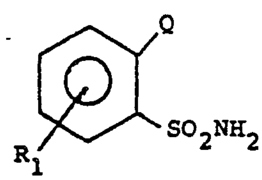

(II)

mit einem Methylheteroarylcarbamat der Formel

$$\begin{array}{c} O \\ \| \\ CH_3OCNA \\ | \\ R_{17} \end{array} \qquad \text{(III)}$$

in Gegenwart einer äquimolaren Menge Trimethylaluminium unter Erhalt einer Verbindung der Formel (I), worin $R_1$, $R_{17}$, Q und A wie in Anspruch 1 definiert sind und W O ist; oder

(b) Reagieren eines Benzolsulfonylisocyanats oder Isothiocyanats der Formel

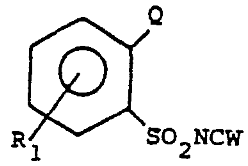

(IV)

mit einem Aminoheterocyclus der Formel

$$\begin{array}{c} HNA \\ | \\ R_{17} \end{array} \qquad \text{(V)}$$

worin $R_1$, $R_{17}$, W und A wie in Anspruch 1 definiert sind und Q ausgewählt ist Q—1, Q—2 oder Q—6 bis Q—11; oder

(c) Reagieren dieses Sulfonamids der Formel (II) mit einem heterocyclischen Isothiocyanat der Formel

$$SCN—A \qquad \text{(Va)}$$

worin A wie oben definiert ist; oder

(d) Reagieren eines Sulfonamids der Formel

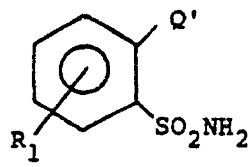

mit einem Methylpyrimidinylcarbamat oder Methyltriazinylcarbamat der Formel

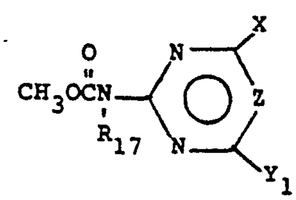

in Gegenwart einer äquimolaren Menge Trimethylaluminium, worin $R_1$, $R_{17}$, Q', X, Y und Z wie oben definiert sind, unter Erhalt eines Produkts der Formel (Ia).

2. Verfahren nach Anspruch 1, worin das Produkt die Formel I aufweist, worin W O ist.

3. Verfahren nach Anspruch 2, worin $R_1$ und $R_{17}$ H sind.

4. Verfahren nach Anspruch 3, worin

$R_{24}$ H, $CH_3$ oder $C_2H_5$ ist;

$R_{25}$ H, $CH_3$ oder $C_2H_5$ ist; und

$R_{26}$ H, $CH_3$, $C_2H_5$ oder $—CO_2CH_3$ ist.

5. Verfahren nach Anspruch 4, worin Y $CH_3$, $CH_2OCH_3$, $OCH_3$, $OC_2H_5$, $CH(OCH_3)_2$ oder

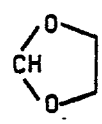

ist und

A 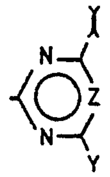 ,

ist.

6. Verfahren nach Anspruch 1, worin das Produkt die Formel Ia aufweist, worin R und $R_{17}$ H sind.

7. Verfahren nach Anspruch 6, worin $Y_1$ $CH_3$, $OCH_3$, $CH_2OCH_3$ oder $N(CH_3)_2$ ist.

8. Verfahren nach Anspruch 4, worin W' = O.

9. Verfahren nach Anspruch 4, worin W' = S.

10. Verfahren nach Anspruch 7, worin Q' ein substituiertes Pyridin ist.

11. Verfahren nach Anspruch 7, worin Q' ein substituiertes Pyrimidin ist.

12. Verfahren nach Anspruch 7, worin Q' ein substituiertes Pyrazin ist.

13. Verfahren nach Anspruch 7, worin Q' ein substituiertes Triazin ist.

14. Verfahren nach Anspruch 1, worin das Produkt eine Verbindung ist, ausgewählt aus N - [(4,6 - Dimethylpyrimidin - 2 - yl)aminocarbonyl] - 2 - (tetrahydrofuran - 2 - yl)benzolsulfonamid; N - [(4 - Methoxy - 6 - methylpyrimidin - 2 - yl)aminocarbonyl] - 2 - (tetrahydrofuran - 2 - yl)benzolsulfonamid; N - [(4,6 - Dimethoxypyrimidin - 2 - yl)aminocarbonyl] - 2 - (tetrahydrofuran - 2 - yl)benzolsulfonamid; N - [(4 - Chlor - 6 - methoxypyrimidin - 2 - yl)amino-carbonyl] - 2 - (tetrahydrofuran - 2 - yl)benzolsulfonamid; N - [(4,6 - Dimethoxy - 1,3,5 - triazin - 2 - yl)aminocarbonyl] - 2 - (tetrahydrofuran - 2 - yl)-benzolsulfonamid; N - [(4 - Methoxy - 6 - methyl - 1,3,5 - triazin - 2 - yl)aminocarbonyl] - 2 - (tetrahydrofuran - 2 - yl)benzolsulfonamid; oder ein landwirtschaftlich geeignetes Salz davon.

15. Verfahren nach Anspruch 1, worin das Produkt eine Verbindung ist, ausgewählt aus N - [(4 - Methoxy - 6 - methylpyrimidin - 2 - yl)aminocarbonyl] - 2 - (2 - pyridinyl)benzolsulfonamid; N - [(4,6 - Dimethylpyrimidin - 2 - yl)aminocarbonyl] - 2 - (2 - pyridinyl)benzolsulfonamid; N - [(4,6 - Dimethoxy - pyrimidin - 2 - yl)aminocarbonyl] - 2 - (2 - pyridinyl)benzolsulfonamid; oder ein landwirtschaftlich geeignetes Salz davon.

16. Verfahren nach Anspruch 1, worin das Produkt N - [(4 - Methoxy - 6 - methylpyrimidin - 2 - yl)aminocarbonyl] - 2 - ( 2 -pyrimidinyl)benzolsulfonamid; oder ein landwirtschaftlich geeignetes Salz davon ist.

17. Zur Bekämpfung des Wachstum unerwünschter Vegetation geeignete Verbindung, enthaltend eine wirksame Menge einer herbiziden Verbindung und wenigstens eines der folgenden: oberflächenaktives Mittel, festes oder flüssiges Verdünnungsmittel, dadurch gekennzeichnet, dass die herbizide Verbindung eine Verbindung der Formeln (I) oder (Ia), wie in einem der Ansprüche 1 bis 16 definiert, umfasst.

18. Verfahren zur Bekämpfung des Wachstums unerwünschter Vegetation durch Anwendung einer wirksamen Menge einer herbiziden Verbindung auf den zu schützenden Ort, dadurch gekennzeichnet, dass die herbizide Verbindung eine Verbindung der Formeln (I) oder (Ia), wie in einem der Ansprüche 1 bis 16 definiert, umfasst.

19. Verfahren zur Regulierung des Wachstums von Pflanzen durch Anwendung einer wirksamen, jedoch im wesentlichen nicht phytotoxischen Menge eines Pflanzenwachstumsregulans auf den Ort solcher Pflanzen, dadurch gekennzeichnet, dass das Pflanzenwachstumsregulans eine Verbindung der Formeln (I) oder (Ia), wie in einem der Ansprüche 1 bis 16 definiert, enthält.

**Revendications pour les Etats contractants: BE CH DE FR IT LI LU NL SE**

1. Un composé de la formule

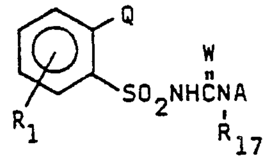

I

ou

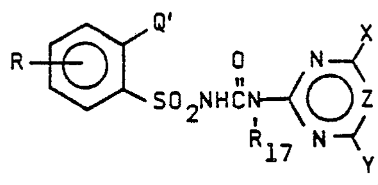

Ia

où

Q est

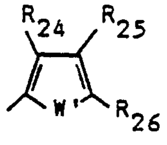,

Q-1

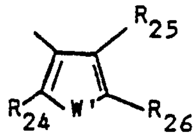,

Q-2

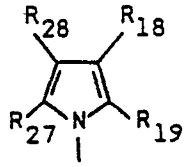,

Q-3

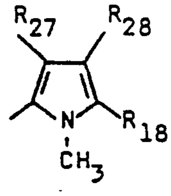,

Q-4

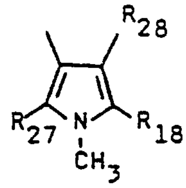,

Q-5

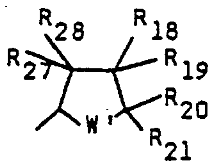,

Q-6

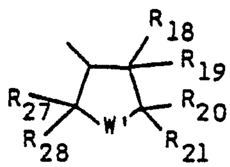,

Q-7

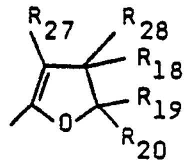,

Q-8

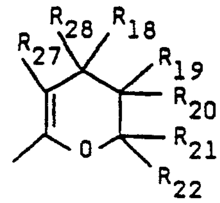,

Q-9

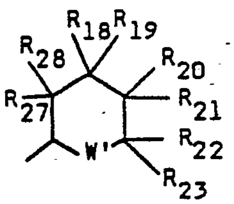

Q-10

ou

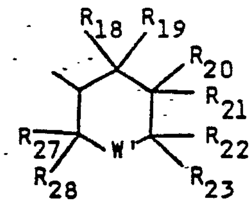;

Q-11

ou Q' est

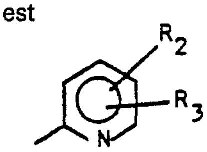,

Q'-1

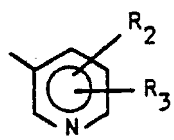,

Q'-2

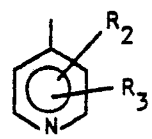,

Q'-3

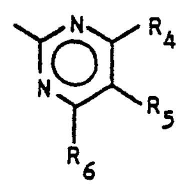,

Q'-4

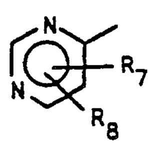,

Q'-5

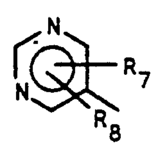,

Q'-6

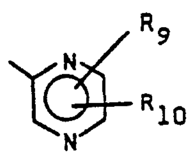,

Q'-7

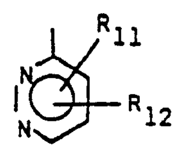,

Q'-8

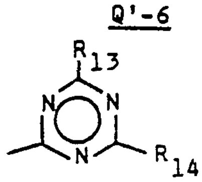

Q'-9

ou

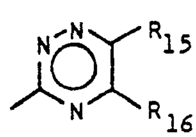;

Q'-10

W est O ou S;
W' est O ou S;
R est H, F, Cl, $CH_3$ ou $OCH_3$;
$R_1$ est H, F, Cl, Br, $CH_3$, $CF_3$ ou $OCH_3$;
$R_2$, $R_3$, $R_5$, $R_9$, $R_{10}$, $R_{11}$, $R_{12}$ et $R_{17}$ sont indépendamment H ou $CH_3$;
$R_4$, $R_6$, $R_7$ et $R_8$ sont indépendamment H, $CH_3$ ou $OCH_3$;
$R_{13}$, $R_{14}$, $R_{15}$ et $R_{16}$ sont indépendamment $CH_3$ ou $OCH_3$;

$R_{18}$ est H ou $CH_3$;
$R_{19}$ est H ou $CH_3$;
$R_{20}$ est H ou $CH_3$;
$R_{21}$ est H ou $CH_3$;
$R_{22}$ est H ou $CH_3$;
$R_{23}$ est H ou $CH_3$;
$R_{24}$ est H, $CH_3$, $C_2H_5$, Cl, Br, $OCH_3$ ou $OC_2H_5$;
$R_{25}$ est H, $CH_3$, $C_2H_5$, Cl, Br, $OCH_3$ ou $OC_2H_5$;
$R_{26}$ est H, $CH_3$, $C_2H_5$, Cl, Br, $OCH_3$, $OC_2H_5$ ou $CO_2CH_3$;
$R_{27}$ est H ou $CH_3$;
$R_{28}$ est H ou $CH_3$;
A est

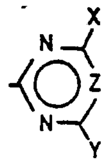 , 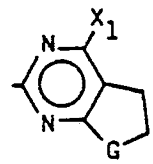 , 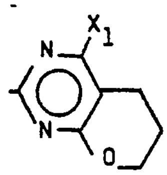 , 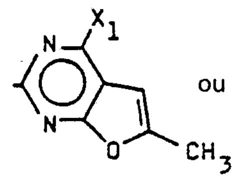 ou 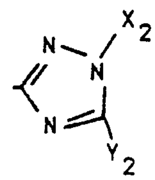 ;

X est $CH_3$, $OCH_3$ ou Cl;
Y est $CH_3$, $C_2H_5$, $CH_2OCH_3$, $OCH_3$, $OC_2H_5$, $CH(OCH_3)_2$, $SCH_3$, $CF_3$, $NH_2$, $NHCH_3$, $N(CH_3)_2$, $OCH_2CH_2OCH_3$, $OCH_2CF_3$ ou

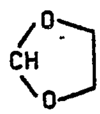 ;

$Y_1$ est $CH_3$, $C_2H_5$, $CH_2OCH_3$, $OCH_3$, $OC_2H_5$, $CH(OCH_3)_2$, $NH_2$, $NHCH_3$ ou $N(CH_3)_2$;
Z est CH ou N;
$X_1$ est $CH_3$ $OCH_3$ ou Cl;
G est O ou $CH_2$;
$X_2$ est $C_1$—$C_3$ alkyle ou $CH_2CF_3$;
$Y_2$ est $CH_3O$, $C_2H_5O$, $CH_3S$ ou $C_2H_5S$;
et leurs sels convenant pour l'agriculture;
avec les conditions que:
1) quand W est S, alors $R_{17}$ est H,

A est

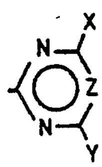 ,

Y est $CH_3$, $OCH_3$, $C_2H_5$, $CH_2OCH_3$, $CH(OCH_3)_2$ ou

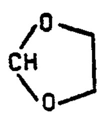 ;

2) quand Q est

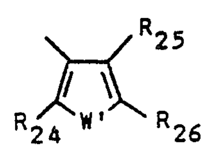 ,

alors $R_{24}$ est H, $CH_3$ ou $C_2H_5$; $R_{25}$ est H, $CH_3$ ou $C_2H_5$; et $R_{26}$ est H, $CH_3$, $C_2H_5$ ou —$CO_2CH_3$;
3) quand l'un de $R_{24}$, $R_{25}$, $R_{26}$ est autre que H, alors les deux autres substituants sont H ou $CH_3$;
4) le nombre total d'atomes de carbone dans $R_{18}$, $R_{19}$, $R_{20}$; $R_{21}$; $R_{22}$; $R_{23}$; $R_{27}$ et $R_{28}$ est inférieur ou égal à 4;
5) quand X est Cl, alors Z est CH et Y ou $Y_1$ est $OCH_3$, $NH_2$, $OC_2H_5$, $NHCH_3$ ou $N(CH_3)_2$; et

6) quand R est $CH_3$, alors R est dans la position 3, 4 ou 5 du noyau benzénique, par rapport au groupe sulfonamide dans la position 1.

2. Les composés selon la revendication 1, formule I, dans lesquels W est O.

3. Les composés selon la revendication 2, dans lesquels $R_1$ et $R_{17}$ sont H.

4. Les composés selon la revendication 3, dans lesquels $R_{24}$ est H, $CH_3$ ou $C_2H_5$; $R_{25}$ est H, $CH_3$ ou $C_2H_5$; et $R_{26}$ est H, $CH_3$, $C_2H_5$ ou $—CO_2CH_3$.

5. Les composés selon la revendication 4, dans lesquels Y est $CH_3$, $CH_2OCH_3$, $OCH_3$, $OC_2H_5$, $CH(OCH_3)_2$ ou

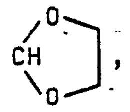

et A est

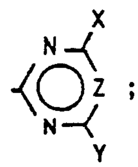

6. Les composés selon la revendication 1, formula Ia, dans lesquels R et $R_{17}$ sont H.

7. Les composés selon la revendication 6, dans lesquels $Y_1$ est $CH_3$, $OCH_3$, $CH_2OCH_3$ ou $N(CH_3)_2$.

8. Les composés selon la revendication 4, dans lesquels W' est O.

9. Les composés selon la revendication 4, dans lesquels W' est S.

10. Les composés selon la revendication 7, dans lesquels Q' est une pyridine substituée.

11. Les composés selon la revendication 7, dans lesquels Q' est une pyrimidine substituée.

12. Les composés selon la revendication 7, dans lesquels Q' est un pyrazine substituée.

13. Les composés selon la revendication 7, dans lesquels Q' est une triazine substituée.

14. Les composé selon la revendication 1, choisi parmi le N - [(4,6 - diméthylpyrimidin - 2 - yl)-aminocarbonyl] - 2 - (tétrahydrofuran - 2 - yl)benzènesulfonamide; le N - [(4 - méthoxy - 6 - méthyle-pyrimidin - 2 - yl)aminocarbonyl] - 2 - (tétrahydrofuran - 2 - yl)benzènesulfonamide; le N - [(4,6 - diméthoxypyrimidin - 2 - yl)aminocarbonyl] - 2 - (tétrahydrofuran - 2 - yl)benzènesulfonamide; le N - [(4 - chloro - 6 - méthoxypyrimidin - 2 - yl)aminocarbonyl] - 2 - (tétrahydrofuran - 2 - yl)-benzènesulfonamide; le N - [(4,6 - diméthoxy - 1,3,5 - triazin - 2 - yl)aminocarbonyl] - 2 - (tétrahydrofuran - 2 - yl)benzènesulfonamide; le N - [(4 - méthoxy - 6 - méthyl - 1,3,5 - triazin - 2 - yl)aminocarbonyl] - 2 - tétrahydrofuran - 2 - yl)benzènesulfonamide; ou un de leurs sels convenant pour l'agriculture.

15. Un composé selon la revendication 1, choisi parmi le N - [(4 - méthoxy - 6 - méthylpyrimidin - 2 - yl)aminocarbonyl] - 2 - (2 - pyridinyl)benzènesulfonamide; le N - [(4,6-diméthylpyrimidin - 2 - yl)aminocarbonyl] - 2 - (2 - pyridinyl)benzènesulfonamide; le N - [(4,6-diméthoxy - pyrimidin - 2 - yl)-aminocarbonyl] - 2 - (2 - pyridinyl)benzènesulfonamide; ou un de leurs sels convenant pour l'agriculture.

16. Un composé selon la revendication 1, qui est le N - [(4 - méthoxy - 6 - méthylpyrimidin - 2 - yl)aminocarbonyl] - 2 - (2 - pyrimidinyl)benzènesulfonamide; ou un de ses sels convenant pour l'agriculture.

17. Une composition utilisable pour lutter contre le croissance de végétation indésirable comprenant une quantité efficace d'un composé à activité herbicide et au moins un des ingrédients suivants; agent tensio-actif, diluant solide ou liquide, caractérisée en ce que le composé à activité herbicide comprend un composé selon l'une quelconque des revendications 1 à 16.

18. Un procédé de lutte contre la croissance de végétation indésirable en appliquant au lieu à protéger une quantité efficace d'une composé à activité herbicide, caractérisé en ce que le composé à activité herbicide comprend un composé selon l'une quelconque des revendications 1 à 16.

19. Un procédé pour régler la croissance de plantes en appliquant au lieu de ces plantes une quantité efficace, mais substantiellement non-phytotoxique, d'un agent de réglage de la croissance de plantes, caractérisé en ce que l'agent de réglage de la croissance des plantes comprend un composé selon l'une quelconque des revendications 1 à 16.

20. Un procédé de préparation d'un composé de la revendication 1, qui comprend

(a) la réaction d'un benzènesulfonamide de formule

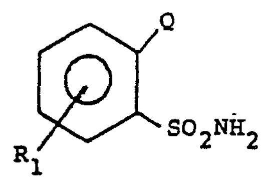

(II)

avec un méthyl hétéroaryl carbamate de formule

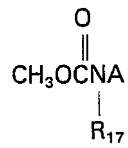

(III)

en présence d'une quantité équimolaire de triméthylaluminium pour obtenir un composé de formule (I) où $R_1$, $R_{17}$, Q et A sont tels que définis dans la revendication 1 et W est O; ou

(b) la réaction d'un isocyanate ou isothiocyanate de benzènesulfonyle de formule

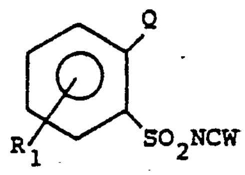

(IV)

avec un aminohétérocycle de formule

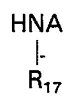

(V)

où $R_1$, $R_{17}$, W et A sont tels que définis dans la revendication 1 et Q est choisi parmi Q—1, Q—2 ou Q—6 à Q—11; ou

(c) la réaction du sulfonamide de formule (II) avec un isothiocyanate hétérocyclique de formule

SCN—A (Va)

où A est tel que défini dans la revendication 1; ou

(d) la réaction d'un sulfonamide de formule

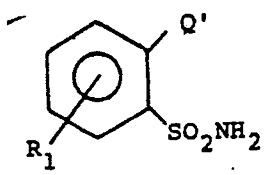

avec un méthyl pyrimidinyl carbamate ou un méthyl triazinyl carbamate de formule

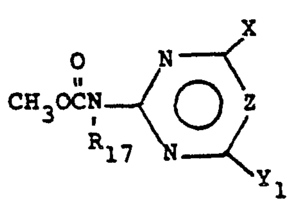

en présence d'une quantité équimolaire de triméthylaluminium, où $R_1$, $R_{17}$, Q', X, Y et Z sont tels que définis dans la revendication 1, pour obtenir un produit de formule (Ia).

**Revendications pour l'Etat contractant: AT**

1. Un procédé pour la préparation d'un composé de la formule

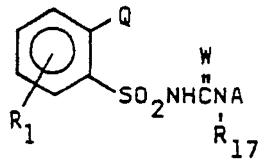

I

ou

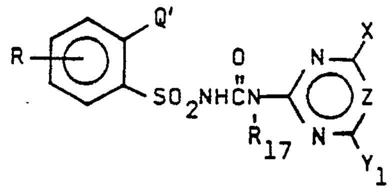

Ia

où

Q est

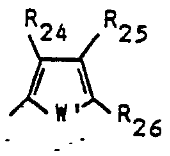

Q-1

,

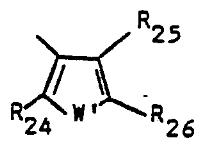

Q-2

,

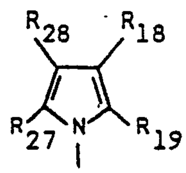

Q-3

,

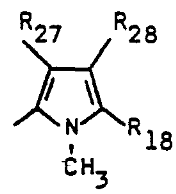

Q-4

,

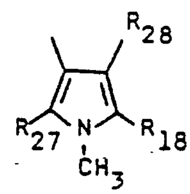

Q-5

,

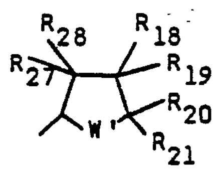

Q-6

,

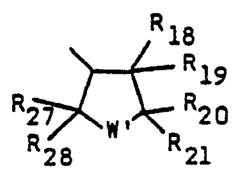

Q-7

,

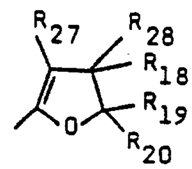

Q-8

,

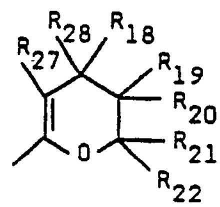

Q-9

,

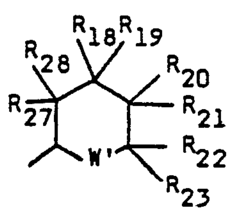

Q-10

ou

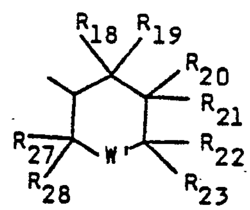

Q-11

;

ou Q est

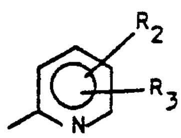

Q'-1

,

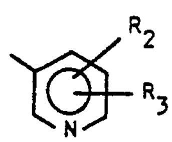

Q'-2

,

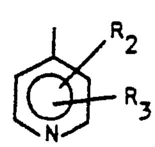

Q'-3

,

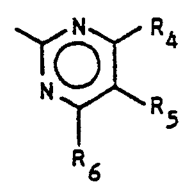

Q'-4

,

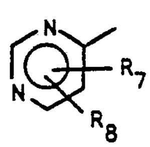

Q'-5

,

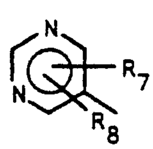

Q'-6

,

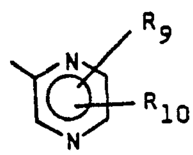

Q'-7

,

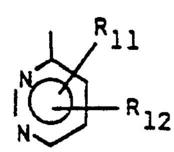

Q'-8

,

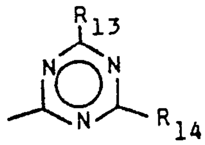 ou 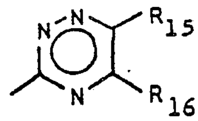 ;

Q'-9 Q'-10

W est O ou S;
W' est O ou S;
R est H, F, Cl, $CH_3$ ou $OCH_3$;
$R_1$ est H, F, Cl, Br, $CH_3$, $CF_3$ ou $OCH_3$;
$R_2$, $R_3$, $R_5$, $R_9$, $R_{10}$, $R_{11}$, $R_{12}$ et $R_{17}$ sont indépendamment H ou $CH_3$;
$R_4$, $R_6$, $R_7$ et $R_8$ sont indépendamment H, $CH_3$ ou $OCH_3$;
$R_{13}$, $R_{14}$, $R_{15}$ et $R_{16}$ sont indépendamment $CH_3$ ou $OCH_3$;
$R_{18}$ est H ou $CH_3$;
$R_{19}$ est H ou $CH_3$;
$R_{20}$ est H ou $CH_3$;
$R_{21}$ est H ou $CH_3$;
$R_{22}$ est H ou $CH_3$;
$R_{23}$ est H ou $CH_3$;
$R_{24}$ est H, $CH_3$, $C_2H_5$, Cl, Br, $OCH_3$ ou $OC_2H_5$;
$R_{25}$ est H, $CH_3$, $C_2H_5$, Cl, Br, $OCH_3$ ou $OC_2H_5$;
$R_{26}$ est H, $CH_3$, $C_2H_5$, Cl, Br, $OCH_3$, $OC_2H_5$ ou $CO_2CH_3$;
$R_{27}$ est H ou $CH_3$;
$R_{28}$ est H ou $CH_3$;
A est

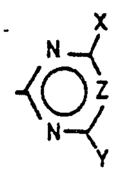 , 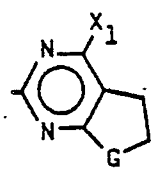 , 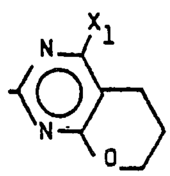 , 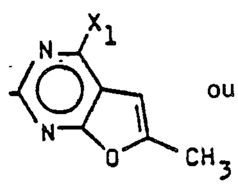 ou 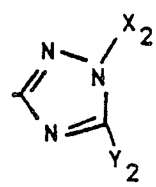 ;

X est $CH_3$, $OCH_3$ ou Cl;
Y est $CH_3$, $C_2H_5$, $CH_2OCH_3$, $OCH_3$, $OC_2H_5$, $CH(OCH_3)_2$, $SCH_3$, $CF_3$, $NH_2$, $NHCH_3$, $N(CH_3)_2$, $OCH_2CH_2OCH_3$, $OCH_2CF_3$ ou

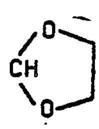 ;

$Y_1$ est $CH_3$, $C_2H_5$, $CH_2OCH_3$, $OCH_3$, $OC_2H_5$, $CH(OCH_3)_2$, $NH_2$, $NHCH_3$ ou $N(CH_3)_2$;
Z est CH ou N;
$X_1$ est $CH_3$ $OCH_3$ ou Cl;
G est O ou $CH_2$;
$X_2$ est $C_1$—$C_3$ alkyle ou $CH_2CF_3$;
$Y_2$ est $CH_3O$, $C_2H_5O$, $CH_3S$ ou $C_2H_5S$;
et leurs sels convenant pour l'agriculture;
acec les conditions que:
1) quand W est S, alors $R_{17}$ est H,

A est 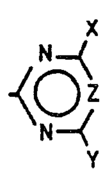 ,

Y est $CH_3$, $OCH_3$, $C_2H_5$, $CH_2OCH_3$, $CH(OCH_3)_2$ ou

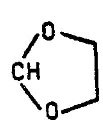 ;

2) quand Q est

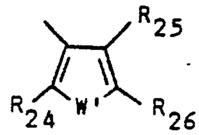 ,

alors $R_{24}$ est H, $CH_3$ ou $C_2H_5$; $R_{25}$ est H, $CH_3$ ou $C_2H_5$; et $R_{26}$ est H, $CH_3$, $C_2H_5$ ou $—CO_2CH_3$;

3) quand l'un de $R_{24}$, $R_{25}$, $R_{26}$ est autre que H, alors les deux autres substituants sont H ou $CH_3$;

4) le nombre total d'atomes de carbone dans $R_{18}$, $R_{19}$, $R_{20}$; $R_{21}$; $R_{22}$; $R_{23}$; $R_{27}$ et $R_{28}$ est inférieur ou égal à 4;

5) quand X est Cl, alors Z est CH et Y ou $Y_1$ est $OCH_3$, $NH_2$, $OC_2H_5$, $NHCH_3$ ou $N(CH_3)_2$; et

6) quand R est $CH_3$, alors R est dans la position 3, 4 ou 5 du noyau benzénique, par rapport au groupe sulfonamide dans la position 1;

qui comprend:

(a) la réaction d'un benzènesulfonamide de formule

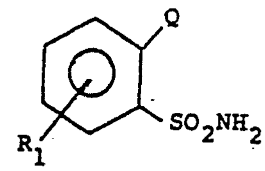 (II)

avec un méthyl hétéroaryl carbamate de formule

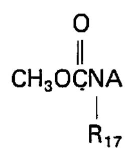 (III)

en présence d'une quantité équimolaire de triméthylaluminium pour obtenir un composé de formule (I) où $R_1$, $P_{17}$, Q et A sont tels que définis ci dessus et W est O; ou

(b) la réaction d'un isocyanate ou isothiocyanate de benzènesulfonyle de formule

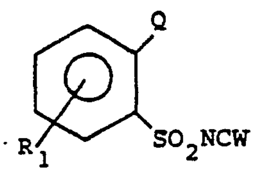 (IV)

avec un aminohétérocycle de formule

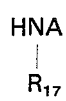 (V)

où $R_1$, $R_{17}$, W et A sont tels que définis ci-dessus et Q est choisi parmi Q—1, Q—2 ou Q—6 à Q—11; ou

(c) la réaction du sulfonamide de formule (II) avec un isothiocyanate hétérocyclique de formule

SCN—A (Va)

où A est tel que défini ci dessus; ou

(d) la réaction d'un sulfonamide de formule

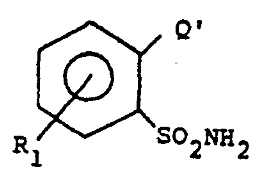

avec un méthyl pyrimidinyl carbamate ou un méthyl triazinyl carbamate de formule

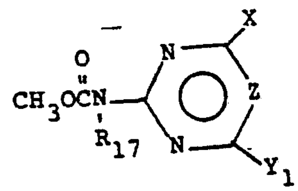

en présence d'une quantité équimolaire de triméthylaluminium, où $R_1$, $R_{17}$, Q', X, Y et Z sont tels que définis ci-dessus, pour obtenir un produit de formule (Ia).

2. Un procédé selon la revendication 1, dans lequel le produit est de formule I, où W est O.

3. Un procédé selon la revendication 2, dans lequel $R_1$ et $R_7$ sont H.

4. Un procédé selon la revendication 3, dans lequel $R_{24}$ est H, $CH_3$ ou $C_2H_5$; $R_{25}$ est H, $CH_3$ ou $C_2H_5$; et $R_{26}$ est H, $CH_3$, $C_2H_5$ ou $-CO_2CH_3$.

5. Un procédé selon la revendication 4, dans lequel Y est $CH_3$, $CH_2OCH_3$, $OCH_3$, $OC_2H_5$, $CH(OCH_3)_2$ ou

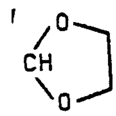,

et A est

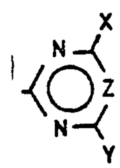;

6. Un procédé selon la revendication 1, dans lequel le produit est de formule Ia, où R et $R_{17}$ sont H.

7. Un procédé selon la revendication 6, dans lequel Y est $CH_3$, $OCH_3$, $CH_2OCH_3$ ou $N(CH_3)_2$.

8. Un procédé selon la revendication 4, dans lequel W' = O.

9. Un procédé selon la revendication 4, dans lequel W' = S.

10. Un procédé selon la revendication 7, dans lequel Q' est une pyridine substituée.

11. Un procédé selon la revendication 7, dans lequel Q' est une pyrimidine substituée.

12. Un procédé selon la revendication 7, dans lequel Q' est une pyrazine substituée.

13. Un procédé selon la revendication 7, dans lequel Q' est une triazine substituée.

14. Un procédé selon la revendication 1, dans lequel le produit est un composé choisi parmi le N - [(4,6 - diméthylpyrimidin - 2 - yl)aminocarbonyl] - 2 - (tétrahydrofuran - 2 - yl)benzènesulfonamide; le N - [(4 - méthoxy - 6 - méthylpyrimidin - 2 - yl)aminocarbonyl] - 2 - (tétrahydrofuran - 2 - yl)-benzènesulfonamide; le N - [(4,6 - diméthoxypyrimidin - 2 - yl)aminocarbonyl] - 2 - (tétrahydrofuran - 2 - yl)benzènesulfonamide; le N - [(4 - chloro - 6 - méthoxypyrimidin - 2 - yl)aminocarbonyl] - 2 - (tétrahydrofuran - 2 - yl)benzènesulfonamide; le N - [(4,6 - diméthoxy - 1,3,5 - triazin - 2 - yl)amino-carbonyl] - 2 - (tétrahydrofuran - 2 - yl)benzènesulfonamide; le N - [(4 - méthoxy - 6 - méthyl - 1,3,5 - triazin - 2 - yl)aminocarbonyl] - 2 - tétrahydrofuran - 2 - yl)benzènesulfonamide; ou un de leurs sels convenant pour l'agriculture.

15. Un procédé selon la revendication 1, dans lequel le produit est un composé choisi parmi le N - [(4 - méthoxy - 6 - méthylpyrimidin - 2 - yl)aminocarbonyl] - 2 - (2 - pyridinyl)benzènesulfonamide; le N - [(4,6-diméthylpyrimidin - 2 - yl)aminocarbonyl] - 2 - (2 - pyridinyl)benzènesulfonamide; le N - [(4,6-diméthoxy - pyrimidin - 2 - yl)aminocarbonyl] - 2 - (2 - pyridinyl)benzènesulfonamide; ou un de leurs sels convenant pour l'agriculture.

16. Un procédé selon la revendication 1, dans lequel le produit est le N - [(4 - méthoxy - 6 - méthylpyrimidin - 2 - yl)aminocarbonyl] - 2 - (2 - pyrimidinyl)benzènesulfonamide; ou un de ses sels convenant pour l'agriculture.

17. Une composition utilisable pour lutter contre le croissance de végétation indésirable comprenant une quantité efficace d'un composé à activité herbicide et au moins un des ingrédients suivants: agent tensio-actif, diluant solide ou liquide, caractérisée en ce que le composé à activité herbicide comprend un composé de formule (I) ou (Ia) tel que défini dans l'une quelconque des revendications 1 à 16.

18. Un procédé de lutte contre la croissance de végétation indésirable en appliquant au lieu à protéger une quantité efficace d'une composé à activité herbicide, caractérisé en ce que le composé à activité herbicide comprend un composé de formule (I) ou (Ia) tel que défini dans l'une quelconque des revendications 1 à 16.

19. Un procédé pour régler la croissance de plantes en appliquant au lieu de ces plantes une quantité efficace, mais substantiellement non-phytotoxique, d'un agent de réglage de la croissance de plantes, caractérisé en ce que l'agent de réglage de la croissance de plantes comprend un composé de formule (I) ou (Ia) tel que défini dans l'une quelconque des revendications 1 à 16.